# EUROPEAN PATENT SPECIFICATION

(11) **EP 2 916 876 B1**
(45) Date of publication and mention of the grant of the patent: **20.10.2021**
(21) Application number: 13853763.4
(22) Date of filing: 05.11.2013
(51) Int. Cl.: A61L 15/22, A61F 13/02, A61L 15/42, A61L 15/44, A61L 15/46

(54) **METHODS AND COMPOSITIONS FOR WOUND HEALING**
VERFAHREN UND ZUSAMMENSETZUNGEN ZUR WUNDHEILUNG
PROCÉDÉS ET COMPOSITIONS POUR LA CICATRISATION

(30) Priority: 06.11.2012 US 201261723111 P
(43) Date of publication of application: 16.09.2015
(73) Proprietor: Imbed Biosciences, Inc., Madison, WI 53711 (US)
(72) Inventor: AGARWAL, Ankit, Madison, Wisconsin 53705 (US); ABBOTT, Nicholas L., Madison, Wisconsin 53711 (US)
(74) Representative: Witte, Weller & Partner Patentanwälte mbB
(86) International application number: PCT/US2013/068463
(87) International publication number: WO 2014/074503

(56) References cited:
- WO-A1-2005/058199
- US-A1- 2004 214 326
- US-A1- 2005 249 791
- US-A1- 2009 263 468
- US-A1- 2011 189 287
- US-A1- 2011 189 287
- AGARWAL ET AL.: 'Surfaces modified with nanometer-thick silver-impregnated polymeric films that kill bacteria but support growth of mammalian cells' BIOMATERIALS vol. 31, no. 4, February 2010, pages 680 - 690, XP026762077
- None

### SEQUENCE LISTING

<110> Imbed Biosciences, Inc.
   Agarwal, Ankit
   Abbott, Nicholas L.
<120> Methods and Compositions for Wound Healing
<130> IMBED-33575/WO-1/ORD
<150> US 61/723,111
   <151> 2012-11-06
<160> 100
<170> PatentIn version 3.5
<210> 1
   <211> 64
   <212> PRT
   <213> Bos taurus
<400> 1
   Met Arg Leu His His Leu Leu Leu Ala Leu Leu Phe Leu Val Leu Ser
   1               5                   10                  15
   Ala Gly Ser Gly Phe Thr Gln Gly Val Arg Asn Ser Gln Ser Cys Arg
               20                  25                  30
   Arg Asn Lys Gly Ile Cys Val Pro Ile Arg Cys Pro Gly Ser Met Arg
           35                  40                  45
   Gln Ile Gly Thr Cys Leu Gly Ala Gln Val Lys Cys Cys Arg Arg Lys
       50                  55                  60
<210> 2
   <211> 24
   <212> PRT
   <213> Xenopus laevis
<400> 2 Ala Leu Asn Ala Val Leu Lys Gln
   20
<210> 3
   <211> 81
   <212> PRT
   <213> Xenopus laevis
<400> 3
   Met Tyr Lys Gly Ile Phe Leu Cys Val Leu Leu Ala Val Ile Cys Ala
   1               5                   10                  15
   Asn Ser Leu Ala Thr Pro Ser Ser Asp Ala Asp Glu Asp Asn Asp Glu
               20                  25                  30
   Val Glu Arg Tyr Val Arg Gly Trp Ala Ser Lys Ile Gly Gln Thr Leu
           35                  40                  45
   Gly Lys Ile Ala Lys Val Gly Leu Lys Glu Leu Ile Gln Pro Lys Arg
       50                  55                  60
   Glu Ala Met Leu Arg Ser Ala Glu Ala Gln Gly Lys Arg Pro Trp Ile
   65                  70                  75                  80
   Leu
<210> 4
   <211> 303
   <212> PRT
   <213> Xenopus laevis
<400> 4
   Met Phe Lys Gly Leu Phe Ile Cys Ser Leu Ile Ala Val Ile Cys Ala
   1               5                   10                  15
   Asn Ala Leu Pro Gln Pro Glu Ala Ser Ala Asp Glu Asp Met Asp Glu
               20                  25                  30
   Arg Glu Val Arg Gly Ile Gly Lys Phe Leu His Ser Ala Gly Lys Phe
           35                  40                  45
   Gly Lys Ala Phe Val Gly Glu Ile Met Lys Ser Lys Arg Asp Ala Glu
       50                  55                  60
   Ala Val Gly Pro Glu Ala Phe Ala Asp Glu Asp Leu Asp Glu Arg Glu
   65                  70                  75                  80
   Val Arg Gly Ile Gly Lys Phe Leu His Ser Ala Lys Lys Phe Gly Lys
                   85                  90                  95
   Ala Phe Val Gly Glu Ile Met Asn Ser Lys Arg Asp Ala Glu Ala Val
               100                 105                 110
   Gly Pro Glu Ala Phe Ala Asp Glu Asp Leu Asp Glu Arg Glu Val Arg
           115                 120                 125
   Gly Ile Gly Lys Phe Leu His Ser Ala Lys Lys Phe Gly Lys Ala Phe
       130                 135                 140
   Val Gly Glu Ile Met Asn Ser Lys Arg Asp Ala Glu Ala Val Gly Pro
   145                 150                 155                 160
   Glu Ala Phe Ala Asp Glu Asp Leu Asp Glu Arg Glu Val Arg Gly Ile
                   165                 170                 175
   Gly Lys Phe Leu His Ser Ala Lys Lys Phe Gly Lys Ala Phe Val Gly
               180                 185                 190
   Glu Ile Met Asn Ser Lys Arg Asp Ala Glu Ala Val Gly Pro Glu Ala
           195                 200                 205
   Phe Ala Asp Glu Asp Phe Asp Glu Arg Glu Val Arg Gly Ile Gly Lys
       210                 215                 220
   Phe Leu His Ser Ala Lys Lys Phe Gly Lys Ala Phe Val Gly Glu Ile
   225                 230                 235                 240
   Met Asn Ser Lys Arg Asp Ala Glu Ala Val Gly Pro Glu Ala Phe Ala
                   245                 250                 255
   Asp Glu Asp Leu Asp Glu Arg Glu Val Arg Gly Ile Gly Lys Phe Leu
               260                 265                 270
   His Ser Ala Lys Lys Phe Gly Lys Ala Phe Val Gly Glu Ile Met Asn
           275                 280                 285
   Ser Lys Arg Asp Ala Glu Ala Val Asp Asp Arg Arg Trp Val Glu
       290                 295                 300
<210> 5
   <211> 17
   <212> PRT
   <213> Tachypleus gigas
<400> 5
   Lys Trp Cys Phe Arg Val Cys Tyr Arg Gly Ile Cys Tyr Arg Arg Cys
   1               5                   10                  15
   Arg
<210> 6
   <211> 17
   <212> PRT
   <213> Tachypleus gigas
<400> 6
   Arg Trp Cys Phe Arg Val Cys Tyr Arg Gly Ile Cys Tyr Arg Lys Cys
   1               5                   10                  15
   Arg
<210> 7
   <211> 129
   <212> PRT
   <213> Bufo bufo gagarizans
<400> 7
   Met Ser Gly Arg Gly Lys Gln Gly Gly Lys Val Arg Ala Lys Ala Lys
   1               5                   10                  15
   Thr Arg Ser Ser Arg Ala Gly Leu Gln Phe Pro Val Gly Arg Val His
               20                  25                  30
   Arg Leu Leu Arg Lys Gly Asn Tyr Ala Gln Arg Val Gly Ala Gly Ala
           35                  40                  45
   Pro Val Tyr Leu Ala Ala Val Leu Glu Tyr Leu Thr Ala Glu Ile Leu
       50                  55                  60
   Glu Leu Ala Gly Asn Ala Ala Arg Asp Asn Lys Lys Thr Arg Ile Ile
   65                  70                  75                  80
   Pro Arg His Leu Gln Leu Ala Val Arg Asn Asp Glu Glu Leu Asn Lys
                   85                  90                  95
   Leu Leu Gly Gly Val Thr Ile Ala Gln Gly Gly Val Leu Pro Asn Ile
               100                 105                 110
   Gln Ala Val Leu Leu Pro Lys Thr Glu Ser Ser Lys Pro Ala Lys Ser
           115                 120                 125
   Lys
<210> 8
   <211> 21
   <212> PRT
   <213> Bufo bufo gagarizans
<400> 8
   Thr Arg Ser Ser Arg Ala Gly Leu Gln Phe Pro Val Gly Arg Val His
   1               5                   10                  15
   Arg Leu Leu Arg Lys
   20
<210> 9
   <211> 63
   <212> PRT
   <213> Bombyx mori
<400> 9
   Met Asn Phe Val Arg Ile Leu Ser Phe Val Phe Ala Leu Val Leu Ala
   1               5                   10                  15
   Leu Gly Ala Val Ser Ala Ala Pro Glu Pro Arg Trp Lys Leu Phe Lys
               20                  25                  30
   Lys Ile Glu Lys Val Gly Arg Asn Val Arg Asp Gly Leu Ile Lys Ala
           35                  40                  45
   Gly Pro Ala Ile Ala Val Ile Gly Gln Ala Lys Ser Leu Gly Lys
       50                  55                  60
<210> 10
   <211> 63
   <212> PRT
   <213> Bombyx mori
<400> 10
   Met Asn Phe Ala Lys Ile Leu Ser Phe Val Phe Ala Leu Val Leu Ala
   1               5                   10                  15
   Leu Ser Met Thr Ser Ala Ala Pro Glu Pro Arg Trp Lys Ile Phe Lys
               20                  25                  30
   Lys Ile Glu Lys Met Gly Arg Asn Ile Arg Asp Gly Ile Val Lys Ala
           35                  40                  45
   Gly Pro Ala Ile Glu Val Leu Gly Ser Ala Lys Ala Ile Gly Lys
       50                  55                  60
<210> 11
   <211> 63
   <212> PRT
   <213> Drosophila melanogaster
<400> 11
   Met Asn Phe Tyr Lys Ile Phe Val Phe Val Ala Leu Ile Leu Ala Ile
   1               5                   10                  15
   Ser Ile Gly Gln Ser Glu Ala Gly Trp Leu Lys Lys Leu Gly Lys Arg
               20                  25                  30
   Ile Glu Arg Ile Gly Gln His Thr Arg Asp Ala Thr Ile Gln Gly Leu
           35                  40                  45
   Gly Ile Ala Gln Gln Ala Ala Asn Val Ala Ala Thr Ala Arg Gly
       50                  55                  60
<210> 12
   <211> 31
   <212> PRT
   <213> Sus scrofa
<400> 12
   Ser Trp Leu Ser Lys Thr Ala Lys Lys Leu Glu Asn Ser Ala Lys Lys
   1               5                   10                  15
   Arg Ile Ser Glu Gly Ile Ala Ile Ala Ile Gln Gly Gly Pro Arg
               20                  25                  30
<210> 13
   <211> 13
   <212> PRT
   <213> Bos taurus
<400> 13
   Ile Leu Pro Trp Lys Trp Pro Trp Trp Pro Trp Arg Arg
   1               5                   10
<210> 14
   <211> 34
   <212> PRT
   <213> Lactococcus lactis
<400> 14 Ser Lys
<210> 15
   <211> 20
   <212> PRT
   <213> Rana catesbeiana
<400> 15
   Phe Leu Gly Gly Leu Ile Lys Ile Val Pro Ala Met Ile Cys Ala Val
   1               5                   10                  15
   Thr Lys Lys Cys
               20
<210> 16
   <211> 25
   <212> PRT
   <213> Bos taurus
<400> 16
   Phe Lys Cys Arg Arg Trp Gln Trp Arg Met Lys Lys Leu Gly Ala Pro
   1               5                   10                  15
   Ser Ile Thr Cys Val Arg Arg Ala Phe
               20                  25
<210> 17
   <211> 19
   <212> PRT
   <213> Sus scrofa
<220>
   <221> misc_feature
   <222> (19)..(19)
   <223> Xaa can be any naturally occurring amino acid
<400> 17 Gly Arg Xaa
<210> 18
   <211> 16
   <212> PRT
   <213> Sus scrofa
<400> 18
   Gly Gly Arg Leu Cys Tyr Cys Arg Arg Arg Phe Cys Ile Cys Val Gly
   1               5                   10                  15
<210> 19
   <211> 51
   <212> PRT
   <213> Homo sapiens
<400> 19
   Met Lys Phe Phe Val Phe Ala Leu Ile Leu Ala Leu Met Leu Ser Met
   1               5                   10                  15
   Thr Gly Ala Asp Ser His Ala Lys Arg His His Gly Tyr Lys Arg Lys
               20                  25                  30
   Phe His Glu Lys His His Ser His Arg Gly Tyr Arg Ser Asn Tyr Leu
           35                  40                  45
   Tyr Asp Asn
       50
<210> 20
   <211> 38
   <212> PRT
   <213> Macaca fascicularis
<400> 20
   Asp Ser His Glu Glu Arg His His Gly Arg His Gly His His Lys Tyr
   1               5                   10                  15
   Gly Arg Lys Phe His Glu Lys His His Ser His Arg Gly Tyr Arg Ser
               20                  25                  30
   Asn Tyr Leu Tyr Asp Asn
           35
<210> 21
   <211> 33
   <212> **PRT**
   <213> Phyllomedusa sauvagei
<400> 21 Gln
<210> 22
   <211> 34
   <212> PRT
   <213> Phyllomedusa sauvagei
<400> 22 Thr Gln
<210> 23
   <211> 30
   <212> **PRT**
   <213> Phyllomedusa sauvagei
<400> 23
   Ala Leu Trp Lys Asn Met Leu Lys Gly Ile Gly Lys Leu Ala Gly Lys
   1               5                   10                  15
   Ala Ala Leu Gly Ala Val Lys Lys Leu Val Gly Ala Glu Ser
               20                  25                  30
<210> 24
   <211> 21
   <212> **PRT**
   <213> Misgurnus anguillicaudatus
<400> **24**
   Arg Gln Arg Val Glu Glu Leu Ser Lys Phe Ser Lys Lys Gly Ala Ala
   1               5                   10                  15
   Ala Arg Arg Arg Lys
               20
<210> 25
   <211> 27
   <212> **PRT**
   <213> Apis mellifera
<400> 25
   Gly Ile Gly Ala Val Leu Lys Val Leu Thr Thr Gly Leu Pro Ala Leu
   1               5                   10                  15
   Ile Ser Trp Ile Ser Arg Lys Lys Arg Gln Gln
               20                  25
<210> 26
   <211> 33
   <212> PRT
   <213> Pardachirus pavoninus
<400> 26 Glu
<210> 27
   <211> 33
   <212> PRT
   <213> Pardachirus pavoninus
<400> 27 Glu
<210> 28
   <211> 176
   <212> PRT
   <213> Bos taurus
<400> 28
   Met Glu Thr Gln Arg Ala Ser Leu Ser Leu Gly Arg Cys Ser Leu Trp
   1               5                   10                  15
   Leu Leu Leu Leu Gly Leu Val Leu Pro Ser Ala Ser Ala Gln Ala Leu
               20                  25                  30
   Ser Tyr Arg Glu Ala Val Leu Arg Ala Val Asp Gln Phe Asn Glu Arg
           35                  40                  45
   Ser Ser Glu Ala Asn Leu Tyr Arg Leu Leu Glu Leu Asp Pro Thr Pro
       50                  55                  60
   Asn Asp Asp Leu Asp Pro Gly Thr Arg Lys Pro Val Ser Phe Arg Val
   65                  70                  75                  80
   Lys Glu Thr Asp Cys Pro Arg Thr Ser Gln Gln Pro Leu Glu Gln Cys
                   85                  90                  95
   Asp Phe Lys Glu Asn Gly Leu Val Lys Gln Cys Val Gly Thr Val Thr
               100                 105                 110
   Leu Asp Pro Ser Asn Asp Gln Phe Asp Ile Asn Cys Asn Glu Leu Gln
           115                 120                 125
   Ser Val Arg Phe Arg Pro Pro Ile Arg Arg Pro Pro Ile Arg Pro Pro
       130                 135                 140
   Phe Tyr Pro Pro Phe Arg Pro Pro Ile Arg Pro Pro Ile Phe Pro Pro
   145                 150                 155                 160
   Ile Arg Pro Pro Phe Arg Pro Pro Leu Gly Pro Phe Pro Gly Arg Arg
                   165                 170                 175
<210> 29
   <211> 155
   <212> PRT
   <213> Bos taurus
<400> 29
   Met Glu Thr Pro Arg Ala Ser Leu Ser Leu Gly Arg Trp Ser Leu Trp
   1               5                   10                  15
   Leu Leu Leu Leu Gly Leu Ala Leu Pro Ser Ala Ser Ala Gln Ala Leu
               20                  25                  30
   Ser Tyr Arg Glu Ala Val Leu Arg Ala Val Asp Gln Leu Asn Glu Gln
           35                  40                  45
   Ser Ser Glu Pro Asn Ile Tyr Arg Leu Leu Glu Leu Asp Gln Pro Pro
       50                  55                  60
   Gln Asp Asp Glu Asp Pro Asp Ser Pro Lys Arg Val Ser Phe Arg Val
   65                  70                  75                  80
   Lys Glu Thr Val Cys Ser Arg Thr Thr Gln Gln Pro Pro Glu Gln Cys
                   85                  90                  95
   Asp Phe Lys Glu Asn Gly Leu Leu Lys Arg Cys Glu Gly Thr Val Thr
               100                 105                 110
   Leu Asp Gln Val Arg Gly Asn Phe Asp Ile Thr Cys Asn Asn His Gln
           115                 120                 125
   Ser Ile Arg Ile Thr Lys Gln Pro Trp Ala Pro Pro Gln Ala Ala Arg
       130                 135                 140
   Leu Cys Arg Ile Val Val Ile Arg Val Cys Arg
   145                 150                 155
<210> 30
   <211> 29
   <212> PRT
   <213> Ceratitis capitata
<400> 30
   Ser Ile Gly Ser Ala Leu Lys Lys Ala Leu Pro Val Ala Lys Lys Ile
   1               5                   10                  15
   Gly Lys Ile Ala Leu Pro Ile Ala Lys Ala Ala Leu Pro
               20                  25
<210> 31
   <211> 29
   <212> PRT
   <213> Ceratitis capitata
<400> 31
   Ser Ile Gly Ser Ala Phe Lys Lys Ala Leu Pro Val Ala Lys Lys Ile
   1               5                   10                  15
   Gly Lys Ala Ala Leu Pro Ile Ala Lys Ala Ala Leu Pro
               20                  25
<210> 32
   <211> 170
   <212> PRT
   <213> Homo sapiens
<400> 32
   Met Lys Thr Gln Arg Asn Gly His Ser Leu Gly Arg Trp Ser Leu Val
   1               5                   10                  15
   Leu Leu Leu Leu Gly Leu Val Met Pro Leu Ala Ile Ile Ala Gln Val
               20                  25                  30
   Leu Ser Tyr Lys Glu Ala Val Leu Arg Ala Ile Asp Gly Ile Asn Gln
           35                  40                  45
   Arg Ser Ser Asp Ala Asn Leu Tyr Arg Leu Leu Asp Leu Asp Pro Arg
       50                  55                  60
   Pro Thr Met Asp Gly Asp Pro Asp Thr Pro Lys Pro Val Ser Phe Thr
   65                  70                  75                  80
   Val Lys Glu Thr Val Cys Pro Arg Thr Thr Gln Gln Ser Pro Glu Asp
                   85                  90                  95
   Cys Asp Phe Lys Lys Asp Gly Leu Val Lys Arg Cys Met Gly Thr Val
               100                 105                 110
   Thr Leu Asn Gln Ala Arg Gly Ser Phe Asp Ile Ser Cys Asp Lys Asp
           115                 120                 125
   Asn Lys Arg Phe Ala Leu Leu Gly Asp Phe Phe Arg Lys Ser Lys Glu
       130                 135                 140
   Lys Ile Gly Lys Glu Phe Lys Arg Ile Val Gln Arg Ile Lys Asp Phe
   145                 150                 155                 160
   Leu Arg Asn Leu Val Pro Arg Thr Glu Ser
                   165                 170
<210> 33
   <211> 170
   <212> **PRT**
   <213> Equus caballus
<400> 33
   Met Glu Thr Gln Arg Asn Thr Arg Cys Leu Gly Arg Trp Ser Pro Leu
   1 5 10 15
   Leu Leu Leu Leu Gly Leu Val Ile Pro Pro Ala Thr Thr Gln Ala Leu
   20 25 30
   Ser Tyr Lys Glu Ala Val Leu Arg Ala Val Asp Gly Leu Asn Gln Arg
   35 40 45
   Ser Ser Asp Glu Asn Leu Tyr Arg Leu Leu Glu Leu Asp Pro Leu Pro
   50 55 60
   Lys Gly Asp Lys Asp Ser Asp Thr Pro Lys Pro Val Ser Phe Met Val
   65 70 75 80
   Lys Glu Thr Val Cys Pro Arg Ile Met Lys Gln Thr Pro Glu Gln Cys
   85 90 95
   Asp Phe Lys Glu Asn Gly Leu Val Lys Gln Cys Val Gly Thr Val Ile
   100 105 110
   Leu Asp Pro Val Lys Asp Tyr Phe Asp Ala Ser Cys Asp Glu Pro Gln
   115 120 125
   Arg Val Lys Arg Phe His Ser Val Gly Ser Leu Ile Gln Arg His Gln
   130 135 140
   Gln Met Ile Arg Asp Lys Ser Glu Ala Thr Arg His Gly Ile Arg Ile
   145 150 155 160
   Ile Thr Arg Pro Lys Leu Leu Leu Ala Ser
   165 170
<210> 34
   <211> 159
   <212> PRT
   <213> Bos taurus
<400> 34
   Met Glu Thr Gln Arg Ala Ser Leu Ser Leu Gly Arg Trp Ser Leu Trp
   1               5                   10                  15
   Leu Leu Leu Leu Gly Leu Ala Leu Pro Ser Ala Ser Ala Gln Ala Leu
               20                  25                  30
   Ser Tyr Arg Glu Ala Val Leu Arg Ala Val Asp Gln Leu Asn Glu Lys
           35                  40                  45
   Ser Ser Glu Ala Asn Leu Tyr Arg Leu Leu Glu Leu Asp Pro Pro Pro
       50                  55                  60
   Lys Glu Asp Asp Glu Asn Pro Asn Ile Pro Lys Pro Val Ser Phe Arg
   65                  70                  75                  80
   Val Lys Glu Thr Val Cys Pro Arg Thr Ser Gln Gln Ser Pro Glu Gln
                   85                  90                  95
   Cys Asp Phe Lys Glu Asn Gly Leu Leu Lys Glu Cys Val Gly Thr Val
               100                 105                 110
   Thr Leu Asp Gln Val Gly Ser Asn Phe Asp Ile Thr Cys Ala Val Pro
           115                 120                 125
   Gln Ser Val Gly Gly Leu Arg Ser Leu Gly Arg Lys Ile Leu Arg Ala
       130                 135                 140
   Trp Lys Lys Tyr Gly Pro Ile Ile Val Pro Ile Ile Arg Ile Gly
   145                 150                 155
<210> 35
   <211> 156
   <212> **PRT**
   <213> Equus caballus
<400> 35
   Met Glu Thr Gln Arg Asn Thr Arg Cys Leu Gly Arg Trp Ser Pro Leu
   1               5                   10                  15
   Leu Leu Leu Leu Gly Leu Val Ile Pro Pro Ala Thr Thr Gln Ala Leu
               20                  25                  30
   Ser Tyr Lys Glu Ala Val Leu Arg Ala Val Asp Gly Leu Asn Gln Arg
           35                  40                  45
   Ser Ser Asp Glu Asn Leu Tyr Arg Leu Leu Glu Leu Asp Pro Leu Pro
       50                  55                  60
   Lys Gly Asp Lys Asp Ser Asp Thr Pro Lys Pro Val Ser Phe Met Val
   65                  70                  75                  80
   Lys Glu Thr Val Cys Pro Arg Ile Met Lys Gln Thr Pro Glu Gln Cys
                   85                  90                  95
   Asp Phe Lys Glu Asn Gly Leu Val Lys Gln Cys Val Gly Thr Val Ile
               100                 105                 110
   Leu Gly Pro Val Lys Asp His Phe Asp Val Ser Cys Gly Glu Pro Gln
           115                 120                 125
   Arg Val Lys Arg Phe Gly Arg Leu Ala Lys Ser Phe Leu Arg Met Arg
       130                 135                 140
   Ile Leu Leu Pro Arg Arg Lys Ile Leu Leu Ala Ser
   145                 150                 155
<210> 36
   <211> 160
   <212> PRT
   <213> Ovis aries
<400> 36
   Met Glu Thr Gln Arg Ala Ser Leu Ser Leu Gly Arg Cys Ser Leu Trp
   1               5                   10                  15
   Leu Leu Leu Leu Gly Leu Ala Leu Pro Ser Ala Ser Ala Gln Val Leu
               20                  25                  30
   Ser Tyr Arg Glu Ala Val Leu Arg Ala Ala Asp Gln Leu Asn Glu Lys
           35                  40                  45
   Ser Ser Glu Ala Asn Leu Tyr Arg Leu Leu Glu Leu Asp Pro Pro Pro
       50                  55                  60
   Lys Gln Asp Asp Glu Asn Ser Asn Ile Pro Lys Pro Val Ser Phe Arg
   65                  70                  75                  80
   Val Lys Glu Thr Val Cys Pro Arg Thr Ser Gln Gln Pro Ala Glu Gln
                   85                  90                  95
   Cys Asp Phe Lys Glu Asn Gly Leu Leu Lys Glu Cys Val Gly Thr Val
               100                 105                 110
   Thr Leu Asp Gln Val Arg Asn Asn Phe Asp Ile Thr Cys Ala Glu Pro
           115                 120                 125
   Gln Ser Val Arg Gly Leu Arg Arg Leu Gly Arg Lys Ile Ala His Gly
       130                 135                 140
   Val Lys Lys Tyr Gly Pro Thr Val Leu Arg Ile Ile Arg Ile Ala Gly
   145                 150                 155                 160
<210> 37
   <211> 12
   <212> PRT
   <213> Bos taurus
<400> 37
   Arg Leu Cys Arg Ile Val Val Ile Arg Val Cys Arg
   1               5                   10
<210> 38
   <211> 30
   <212> **PRT**
   <213> Homo sapiens
<400> 38
   Ala Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 39
   <211> 29
   <212> PRT
   <213> Homo sapiens
<400> 39
   Cys Tyr Cys Arg Ile Pro Ala Cys Ile Ala Gly Glu Arg Arg Tyr Gly
   1               5                   10                  15
   Thr Cys Ile Tyr Gln Gly Arg Leu Trp Ala Phe Cys Cys
               20                  25
<210> 40
   <211> 30
   <212> **PRT**
   <213> Homo sapiens
<400> **40**
   Asp Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 41
   <211> 33
   <212> **PRT**
   <213> Homo sapiens
<400> **41** Val
<210> 42
   <211> 33
   <212> **PRT**
   <213> Oryctolagus cuniculus
<400> **42** Arg
<210> 43
   <211> 33
   <212> **PRT**
   <213> Oryctolagus cuniculus
<400> 43 Arg
<210> **44**
   <211> 34
   <212> **PRT**
   <213> Oryctolagus cuniculus
<400> **44** Arg Arg
<210> 45
   <211> 34
   <212> **PRT**
   <213> Oryctolagus cuniculus
<400> **45** **Pro Arg**
<210> 46
   <211> 34
   <212> **PRT**
   <213> Oryctolagus cuniculus
<400> **46**
   Val Ser Cys Thr Cys Arg Arg Phe Ser Cys Gly Phe Gly Glu Arg Ala
   1               5                   10                  15
   Ser Gly Ser Cys Thr Val Asn Gly Gly Val Arg His Thr Leu Cys Cys
               20                  25                  30
   Arg Arg
<210> 47
   <211> 33
   <212> **PRT**
   <213> Oryctolagus cuniculus
<400> **47** Arg
<210> 48
   <211> 32
   <212> PRT
   <213> Rattus norvegicus
<400> 48
   Val Thr Cys Tyr Cys Arg Arg Thr Arg Cys Gly Phe Arg Glu Arg Leu
   1 5 10 15
   Ser Gly Ala Cys Gly Tyr Arg Gly Arg Ile Tyr Arg Leu Cys Cys Arg
   20 25 30
<210> 49
   <211> 30
   <212> **PRT**
   <213> Rattus norvegicus
<400> **49**
   Cys Ser Cys Arg Tyr Ser Ser Cys Arg Phe Gly Glu Arg Leu Leu Ser
   1 5 10 15
   Gly Ala Cys Arg Leu Asn Gly Arg Ile Tyr Arg Leu Cys Cys
   20 25 30
<210> 50
   <211> 31
   <212> PRT
   <213> Rattus norvegicus
<400> 50
   Ala Cys Thr Cys Arg Ile Gly Ala Cys Val Ser Gly Glu Arg Leu Thr
   1 5 10 15
   Gly Ala Cys Gly Leu Asn Gly Arg Ile Tyr Arg Leu Cys Cys Arg
   20 25 30
<210> 51
   <211> 31
   <212> PRT
   <213> Guinea pig
<400> 51
   Arg Arg Cys Ile Cys Thr Thr Arg Thr Cys Arg Phe Pro Tyr Arg Arg
   1               5                   10                  15
   Leu Gly Thr Cys Ile Phe Gln Asn Arg Val Tyr Thr Phe Cys Cys
               20                  25                  30
<210> 52
   <211> 67
   <212> PRT
   <213> Homo sapiens
<400> 52
   Met Arg Ile His Tyr Leu Leu Phe Ala Leu Leu Phe Leu Phe Leu Val
   1               5                   10                  15
   Pro Val Pro Gly His Gly Gly Ile Ile Asn Thr Leu Gln Lys Tyr Tyr
               20                  25                  30
   Cys Arg Val Arg Gly Gly Arg Cys Ala Val Leu Ser Cys Leu Pro Lys
           35                  40                  45
   Glu Glu Gln Ile Gly Lys Cys Ser Thr Arg Gly Arg Lys Cys Cys Arg
       50                  55                  60
   Arg Lys Lys
   65
<210> 53
   <211> 18
   <212> PRT
   <213> Macaca mulatta
<400> 53
   Arg Cys Ile Cys Thr Arg Gly Phe Cys Arg Cys Leu Cys Arg Arg Gly
   1               5                   10                  15
   Val Cys
<210> 54
   <211> 78
   <212> **PRT**
   <213> Helianthus annuus
<400> **54**
   Met Lys Ser Ser Met Lys Met Phe Ala Ala Leu Leu Leu Val Val Met
   1 5 10 15
   Cys Leu Leu Ala Asn Glu Met Gly Gly Pro Leu Val Val Glu Ala Arg
   20 25 30
   Thr Cys Glu Ser Gln Ser His Lys Phe Lys Gly Thr Cys Leu Ser Asp
   35 40 45
   Thr Asn Cys Ala Asn Val Cys His Ser Glu Arg Phe Ser Gly Gly Lys
   50 55 60
   Cys Arg Gly Phe Arg Arg Arg Cys Phe Cys Thr Thr His Cys
   65 70 75
<210> 55
   <211> 78
   <212> PRT
   <213> Helianthus annuus
<400> 55
   Met Lys Ser Ser Met Lys Met Phe Ala Ala Leu Leu Leu Val Val Met
   1 5 10 15
   Cys Leu Leu Ala Asn Glu Met Gly Gly Pro Leu Val Val Glu Ala Arg
   20 25 30
   Thr Cys Glu Ser Gln Ser His Lys Phe Lys Gly Thr Cys Leu Ser Asp
   35 40 45
   Thr Asn Cys Ala Asn Val Cys His Ser Glu Arg Phe Ser Gly Gly Lys
   50 55 60
   Cys Arg Gly Phe Arg Arg Arg Cys Phe Cys Thr Thr His Cys
   65 70 75
<210> 56
   <211> 30
   <212> **PRT**
   <213> Macaca mulatta
<400> 56
   Ala Cys Tyr Cys Arg Ile Pro Ala Cys Leu Ala Gly Glu Arg Arg Tyr
   1 5 10 15
   Gly Thr Cys Phe Tyr Met Gly Arg Val Trp Ala Phe Cys Cys
   20 25 30
<210> 57
   <211> 37
   <212> PRT
   <213> Androctonus australis hector
<400> 57
   Gly Phe Gly Cys Pro Phe Asn Gln Gly Ala Cys His Arg His Cys Arg
   1               5                   10                  15
   Ser Ile Arg Arg Arg Gly Gly Tyr Cys Ala Gly Leu Phe Lys Gln Thr
               20                  25                  30
   Cys Thr Cys Tyr Arg
           35
<210> 58
   <211> 38
   <212> PRT
   <213> Mytilus galloprovincialis
<220>
   <221> misc_feature
   <222> (28)..(28)
   <223> Xaa can be any naturally occurring amino acid
<400> 58
   Gly Phe Gly Cys Pro Asn Asn Tyr Gln Cys His Arg His Cys Lys Ser
   1               5                   10                  15
   Ile Pro Gly Arg Cys Gly Gly Tyr Cys Gly Gly Xaa His Arg Leu Arg
               20                  25                  30
   Cys Thr Cys Tyr Arg Cys
           35
<210> 59
   <211> 54
   <212> **PRT**
   <213> Heuchera sanguinea
<400> 59
   Asp Gly Val Lys Leu Cys Asp Val Pro Ser Gly Thr Trp Ser Gly His
   1               5                   10                  15
   Cys Gly Ser Ser Ser Lys Cys Ser Gln Gln Cys Lys Asp Arg Glu His
               20                  25                  30
   Phe Ala Tyr Gly Gly Ala Cys His Tyr Gln Phe Pro Ser Val Lys Cys
           35                  40                  45
   Phe Cys Lys Arg Gln Cys
       50
<210> 60
   <211> 49
   <212> PRT
   <213> Clitoria ternatea
<400> 60 Cys
<210> 61
   <211> 91
   <212> PRT
   <213> Mus musculus
<400> 61
   Met Lys Lys Leu Val Leu Leu Phe Ala Leu Val Leu Leu Ala Phe Gln
   1               5                   10                  15
   Val Gln Ala Asp Ser Ile Gln Asn Thr Asp Glu Glu Thr Lys Thr Glu
               20                  25                  30
   Glu Gln Pro Gly Glu Lys Asp Gln Ala Val Ser Val Ser Phe Gly Asp
           35                  40                  45
   Pro Gln Gly Ser Ala Leu Gln Asp Ala Ala Leu Gly Trp Gly Arg Arg
       50                  55                  60
   Cys Pro Gln Cys Pro Arg Cys Pro Ser Cys Pro Ser Cys Pro Arg Cys
   65                  70                  75                  80
   Pro Arg Cys Pro Arg Cys Lys Cys Asn Pro Lys
                   85                  90
<210> 62
   <211> 40
   <212> PRT
   <213> Bos taurus
<400> 62
   Gln Gly Val Arg Asn Phe Val Thr Cys Arg Ile Asn Arg Gly Phe Cys
   1               5                   10                  15
   Val Pro Ile Arg Cys Pro Gly His Arg Arg Gln Ile Gly Thr Cys Leu
               20                  25                  30
   Gly Pro Gln Ile Lys Cys Cys Arg
           35                  40
<210> 63
   <211> 40
   <212> PRT
   <213> Bos taurus
<400> 63
   Gln Gly Val Arg Asn Phe Val Thr Cys Arg Ile Asn Arg Gly Phe Cys
   1               5                   10                  15
   Val Pro Ile Arg Cys Pro Gly His Arg Arg Gln Ile Gly Thr Cys Leu
               20                  25                  30
   Gly Pro Arg Ile Lys Cys Cys Arg
           35                  40
<210> 64
   <211> 42
   <212> PRT
   <213> Bos taurus
<400> 64
   Gln Gly Val Arg Asn His Val Thr Cys Arg Ile Tyr Gly Gly Phe Cys
   1               5                   10                  15
   Val Pro Ile Arg Cys Pro Gly Arg Thr Arg Gln Ile Gly Thr Cys Phe
               20                  25                  30
   Gly Arg Pro Val Lys Cys Cys Arg Arg Trp
           35                  40
<210> 65
   <211> 40
   <212> PRT
   <213> Bos taurus
<400> 65
   Gln Val Val Arg Asn Pro Gln Ser Cys Arg Trp Asn Met Gly Val Cys
   1               5                   10                  15
   Ile Pro Ile Ser Cys Pro Gly Asn Met Arg Gln Ile Gly Thr Cys Phe
               20                  25                  30
   Gly Pro Arg Val Pro Cys Cys Arg
           35                  40
<210> 66
   <211> 41
   <212> PRT
   <213> Bos taurus
<400> 66
   Gln Arg Val Arg Asn Pro Gln Ser Cys Arg Trp Asn Met Gly Val Cys
   1               5                   10                  15
   Ile Pro Phe Leu Cys Arg Val Gly Met Arg Gln Ile Gly Thr Cys Phe
               20                  25                  30
   Gly Pro Arg Val Pro Cys Cys Arg Arg
           35                  40
<210> 67
   <211> 42
   <212> PRT
   <213> Bos taurus
<400> 67
   Gln Gly Val Arg Asn His Val Thr Cys Arg Ile Asn Arg Gly Phe Cys
   1               5                   10                  15
   Val Pro Ile Arg Cys Pro Gly Arg Thr Arg Gln Ile Gly Thr Cys Phe
               20                  25                  30
   Gly Pro Arg Ile Lys Cys Cys Arg Ser Trp
           35                  40
<210> 68
   <211> 40
   <212> PRT
   <213> Bos taurus
<400> 68
   Gln Gly Val Arg Ser Tyr Leu Ser Cys Trp Gly Asn Arg Gly Ile Cys
   1 5 10 15
   Leu Leu Asn Arg Cys Pro Gly Arg Met Arg Gln Ile Gly Thr Cys Leu
   20 25 30
   Ala Pro Arg Val Lys Cys Cys Arg
   35 40
<210> 69
   <211> 42
   <212> PRT
   <213> Bos taurus
<400> 69
   Ser Gly Ile Ser Gly Pro Leu Ser Cys Gly Arg Asn Gly Gly Val Cys
   1 5 10 15
   Ile Pro Ile Arg Cys Pro Val Pro Met Arg Gln Ile Gly Thr Cys Phe
   20 25 30
   Gly Arg Pro Val Lys Cys Cys Arg Ser Trp
   35 40
<210> 70
   <211> 38
   <212> PRT
   <213> Bos taurus
<400> 70
   Asp Phe Ala Ser Cys His Thr Asn Gly Gly Ile Cys Leu Pro Asn Arg
   1 5 10 15
   Cys Pro Gly His Met Ile Gln Ile Gly Ile Cys Phe Arg Pro Arg Val
   20 25 30
   Lys Cys Cys Arg Ser Trp
   35
<210> 71
   <211> 74
   <212> **PRT**
   <213> Zophobas atratus
<400> 71
   Ser Leu Gln Gly Gly Ala Pro Asn Phe Pro Gln Pro Ser Gln Gln Asn
   1               5                   10                  15
   Gly Gly Trp Gln Val Ser Pro Asp Leu Gly Arg Asp Asp Lys Gly Asn
               20                  25                  30
   Thr Arg Gly Gln Ile Glu Ile Gln Asn Lys Gly Lys Asp His Asp Phe
           35                  40                  45
   Asn Ala Gly Trp Gly Lys Val Ile Arg Gly Pro Asn Lys Ala Lys Pro
       50                  55                  60
   Thr Trp His Val Gly Gly Thr Tyr Arg Arg
   65                  70
<210> 72
   <211> 67
   <212> PRT
   <213> Homo sapiens
<400> 72
   Met Arg Ile His Tyr Leu Leu Phe Ala Leu Leu Phe Leu Phe Leu Val
   1               5                   10                  15
   Pro Val Pro Gly His Gly Gly Ile Ile Asn Thr Leu Gln Lys Tyr Tyr
               20                  25                  30
   Cys Arg Val Arg Gly Gly Arg Cys Ala Val Leu Ser Cys Leu Pro Lys
           35                  40                  45
   Glu Glu Gln Ile Gly Lys Cys Ser Thr Arg Gly Arg Lys Cys Cys Arg
       50                  55                  60
   Arg Lys Lys
   65
<210> 73
   <211> 40
   <212> **PRT**
   <213> Aedes aegypti
<400> 73
   Ala Thr Cys Asp Leu Leu Ser Gly Phe Gly Val Gly Asp Ser Ala Cys
   1               5                   10                  15
   Ala Ala His Cys Ile Ala Arg Gly Asn Arg Gly Gly Tyr Cys Asn Ser
               20                  25                  30
   Lys Lys Val Cys Val Cys Arg Asn
           35                  40
<210> 74
   <211> 35
   <212> **PRT**
   <213> Mytilus edulis
<220>
   <221> misc_feature
   <222> (28)..(28)
   <223> Xaa can be any naturally occurring amino acid
<400> 74
   Gly Phe Gly Cys Pro Asn Asp Tyr Pro Cys His Arg His Cys Lys Ser
   1               5                   10                  15
   Ile Pro Gly Arg Tyr Gly Gly Tyr Cys Gly Gly Xaa His Arg Leu Arg
               20                  25                  30
   Cys Thr Cys
           35
<210> 75
   <211> 40
   <212> PRT
   <213> Sarcophaga peregrina
<400> 75
   Ala Thr Cys Asp Leu Leu Ser Gly Ile Gly Val Gln His Ser Ala Cys
   1               5                   10                  15
   Ala Leu His Cys Val Phe Arg Gly Asn Arg Gly Gly Tyr Cys Thr Gly
               20                  25                  30
   Lys Gly Ile Cys Val Cys Arg Asn
           35                  40
<210> 76
   <211> 95
   <212> PRT
   <213> Oryctolagus cuniculus
<400> 76
   Met Arg Thr Leu Ala Leu Leu Ala Ala Ile Leu Leu Val Ala Leu Gln
   1               5                   10                  15
   Ala Gln Ala Glu His Val Ser Val Ser Ile Asp Glu Val Val Asp Gln
               20                  25                  30
   Gln Pro Pro Gln Ala Glu Asp Gln Asp Val Ala Ile Tyr Val Lys Glu
           35                  40                  45
   His Glu Ser Ser Ala Leu Glu Ala Leu Gly Val Lys Ala Gly Val Val
       50                  55                  60
   Cys Ala Cys Arg Arg Ala Leu Cys Leu Pro Arg Glu Arg Arg Ala Gly
   65                  70                  75                  80
   Phe Cys Arg Ile Arg Gly Arg Ile His Pro Leu Cys Cys Arg Arg
              85                  90                  95
<210> 77
   <211> 92
   <212> PRT
   <213> Mus musculus
<400> 77
   Met Lys Pro Leu Val Leu Leu Ser Ala Leu Val Leu Leu Ser Phe Gln
   1               5                   10                  15
   Val Gln Ala Asp Pro Ile Gln Asn Thr Asp Glu Glu Thr Lys Thr Glu
               20                  25                  30
   Glu Gln Ser Gly Glu Glu Asp Gln Ala Val Ser Val Ser Phe Gly Asp
           35                  40                  45
   Arg Glu Gly Ala Ser Leu Gln Glu Glu Ser Leu Arg Asp Leu Val Cys
       50                  55                  60
   Tyr Cys Arg Thr Arg Gly Cys Lys Arg Arg Glu Arg Met Asn Gly Thr
   65                  70                  75                  80
   Cys Arg Lys Gly His Leu Met Tyr Thr Leu Cys Cys
                   85                  90
<210> 78
   <211> 93
   <212> PRT
   <213> Mus musculus
<400> 78
   Met Lys Thr Phe Val Leu Leu Ser Ala Leu Val Leu Leu Ala Phe Gln
   1               5                   10                  15
   Val Gln Ala Asp Pro Ile His Lys Thr Asp Glu Glu Thr Asn Thr Glu
               20                  25                  30
   Glu Gln Pro Gly Glu Glu Asp Gln Ala Val Ser Ile Ser Phe Gly Gly
           35                  40                  45
   Gln Glu Gly Ser Ala Leu His Glu Glu Leu Ser Lys Lys Leu Ile Cys
       50                  55                  60
   Tyr Cys Arg Ile Arg Gly Cys Lys Arg Arg Glu Arg Val Phe Gly Thr
   65                  70                  75                  80
   Cys Arg Asn Leu Phe Leu Thr Phe Val Phe Cys Cys Ser
                   85                  90
<210> 79
   <211> 35
   <212> PRT
   <213> Mus musculus
<400> 79
   Leu Arg Asp Leu Val Cys Tyr Cys Arg Ala Arg Gly Cys Lys Gly Arg
   1               5                   10                  15
   Glu Arg Met Asn Gly Thr Cys Arg Lys Gly His Leu Leu Tyr Met Leu
               20                  25                  30
   Cys Cys Arg
           35
<210> 80
   <211> 43
   <212> PRT
   <213> Pyrrhocoris apterus
<400> 80
   Ala Thr Cys Asp Ile Leu Ser Phe Gln Ser Gln Trp Val Thr Pro Asn
   1               5                   10                  15
   His Ala Gly Cys Ala Leu His Cys Val Ile Lys Gly Tyr Lys Gly Gly
               20                  25                  30
   Gln Cys Lys Ile Thr Val Cys His Cys Arg Arg
           35                  40
<210> 81
   <211> 32
   <212> PRT
   <213> Rattus norvegicus
<400> 81
   Val Thr Cys Tyr Cys Arg Ser Thr Arg Cys Gly Phe Arg Glu Arg Leu
   1               5                   10                  15
   Ser Gly Ala Cys Gly Tyr Arg Gly Arg Ile Tyr Arg Leu Cys Cys Arg
               20                  25                  30
<210> 82
   <211> 31
   <212> PRT
   <213> Rattus norvegicus
<400> 82
   Val Thr Cys Ser Cys Arg Thr Ser Ser Cys Arg Phe Gly Glu Arg Leu
     1               5                   10                  15
   Ser Gly Ala Cys Arg Leu Asn Gly Arg Ile Tyr Arg Leu Cys Cys
               20                  25                  30
<210> 83
   <211> 34
   <212> **PRT**
   <213> Oryctolagus cuniculus
<400> 83 Arg Arg
<210> 84
   <211> 64
   <212> **PRT**
   <213> Pan troglodytes
<400> **84**
   Met Arg Val Leu Tyr Leu Leu Phe Ser Phe Leu Phe Ile Phe Leu Met
   1               5                   10                  15
   Pro Leu Pro Gly Val Phe Gly Gly Ile Ser Asp Pro Val Thr Cys Leu
               20                  25                  30
   Lys Ser Gly Ala Ile Cys His Pro Val Phe Cys Pro Arg Arg Tyr Lys
           35                  40                  45
   Gln Ile Gly Thr Cys Gly Leu Pro Gly Thr Lys Cys Cys Lys Lys Pro
       50                  55                  60
<210> 85
   <211> 64
   <212> PRT
   <213> Homo sapiens
<400> 85
   Met Arg Val Leu Tyr Leu Leu Phe Ser Phe Leu Phe Ile Phe Leu Met
   1               5                   10                  15
   Pro Leu Pro Gly Val Phe Gly Gly Ile Gly Asp Pro Val Thr Cys Leu
               20                  25                  30
   Lys Ser Gly Ala Ile Cys His Pro Val Phe Cys Pro Arg Arg Tyr Lys
           35                  40                  45
   Gln Ile Gly Thr Cys Gly Leu Pro Gly Thr Lys Cys Cys Lys Lys Pro
       50                  55                  60
<210> 86
   <211> 68
   <212> PRT
   <213> Homo sapiens
<400> 86
   Met Arg Thr Ser Tyr Leu Leu Leu Phe Thr Leu Cys Leu Leu Leu Ser
   1 5 10 15
   Glu Met Ala Ser Gly Gly Asn Phe Leu Thr Gly Leu Gly His Arg Ser
   20 25 30
   Asp His Tyr Asn Cys Val Ser Ser Gly Gly Gln Cys Leu Tyr Ser Ala
   35 40 45
   Cys Pro Ile Phe Thr Lys Ile Gln Gly Thr Cys Tyr Arg Gly Lys Ala
   50 55 60
   Lys Cys Cys Lys
   65
<210> 87
   <211> 64
   <212> PRT
   <213> Capra hircus
<400> 87
   Met Arg Leu His His Leu Leu Leu Val Leu Phe Phe Leu Val Leu Ser
   1 5 10 15
   Ala Gly Ser Gly Phe Thr Gln Gly Ile Arg Ser Arg Arg Ser Cys His
   20 25 30
   Arg Asn Lys Gly Val Cys Ala Leu Thr Arg Cys Pro Arg Asn Met Arg
   35 40 45
   Gln Ile Gly Thr Cys Phe Gly Pro Pro Val Lys Cys Cys Arg Lys Lys
   50 55 60
<210> 88
   <211> 64
   <212> **PRT**
   <213> Capra hircus
<400> 88
   Met Arg Leu His His Leu Leu Leu Ala Leu Phe Phe Leu Val Leu Ser
   1               5                   10                  15
   Ala Gly Ser Gly Phe Thr Gln Gly Ile Ile Asn His Arg Ser Cys Tyr
               20                  25                  30
   Arg Asn Lys Gly Val Cys Ala Pro Ala Arg Cys Pro Arg Asn Met Arg
           35                  40                  45
   Gln Ile Gly Thr Cys His Gly Pro Pro Val Lys Cys Cys Arg Lys Lys
       50                  55                  60
<210> 89
   <211> 96
   <212> PRT
   <213> Macaca mulatta
<400> 89
   Met Arg Thr Leu Val Ile Leu Ala Ala Ile Leu Leu Val Ala Leu Gln
   1               5                   10                  15
   Ala Gln Ala Glu Pro Leu Gln Ala Arg Thr Asp Glu Ala Thr Ala Ala
               20                  25                  30
   Gln Glu Gln Ile Pro Thr Asp Asn Pro Glu Val Val Val Ser Leu Ala
           35                  40                  45
   Trp Asp Glu Ser Leu Ala Pro Lys Asp Ser Val Pro Gly Leu Arg Lys
       50                  55                  60
   Asn Met Ala Cys Tyr Cys Arg Ile Pro Ala Cys Leu Ala Gly Glu Arg
   65                  70                  75                  80
   Arg Tyr Gly Thr Cys Phe Tyr Arg Arg Arg Val Trp Ala Phe Cys Cys
                   85                  90                  95
<210> 90
   <211> 96
   <212> PRT
   <213> Macaca mulatta
<400> 90
   Met Arg Thr Leu Val Ile Leu Ala Ala Ile Leu Leu Val Ala Leu Gln
   1               5                   10                  15
   Ala Gln Ala Glu Pro Leu Gln Ala Arg Thr Asp Glu Ala Thr Ala Ala
               20                  25                  30
   Gln Glu Gln Ile Pro Thr Asp Asn Pro Glu Val Val Val Ser Leu Ala
           35                  40                  45
   Trp Asp Glu Ser Leu Ala Pro Lys Asp Ser Val Pro Gly Leu Arg Lys
       50                  55                  60
   Asn Met Ala Cys Tyr Cys Arg Ile Pro Ala Cys Leu Ala Gly Glu Arg
   65                  70                  75                  80
   Arg Tyr Gly Thr Cys Phe Tyr Leu Gly Arg Val Trp Ala Phe Cys Cys
                   85                  90                  95
<210> 91
   <211> 33
   <212> **PRT**
   <213> Mesocricetus auratus
<400> 91 Arg
<210> 92
   <211> 31
   <212> PRT
   <213> Mesocricetus auratus
<400> 92
   Cys Phe Cys Lys Arg Pro Val Cys Asp Ser Gly Glu Thr Gln Ile Gly
   1               5                   10                  15
   Tyr Cys Arg Leu Gly Asn Thr Phe Tyr Arg Leu Cys Cys Arg Gln
               20                  25                  30
<210> 93
   <211> 39
   <212> **PRT**
   <213> Gallus gallus
<400> 93
   Gly Arg Lys Ser Asp Cys Phe Arg Lys Asn Gly Phe Cys Ala Phe Leu
   1               5                   10                  15
   Lys Cys Pro Tyr Leu Thr Leu Ile Ser Gly Lys Cys Ser Arg Phe His
   20 25 30
   Leu Cys Cys Lys Arg Ile Trp
   35
<210> 94
   <211> 43
   <212> **PRT**
   <213> Allomyrina dichotoma
<400> 94
   Val Thr Cys Asp Leu Leu Ser Phe Glu Ala Lys Gly Phe Ala Ala Asn
   1 5 10 15
   His Ser Leu Cys Ala Ala His Cys Leu Ala Ile Gly Arg Arg Gly Gly
   20 25 30
   Ser Cys Glu Arg Gly Val Cys Ile Cys Arg Arg
   35 40
<210> 95
   <211> 31
   <212> PRT
   <213> Cavia porcellus
<400> 95
   Arg Arg Cys Ile Cys Thr Thr Arg Thr Cys Arg Phe Pro Tyr Arg Arg
   1 5 10 15
   Leu Gly Thr Cys Ile Phe Gln Asn Arg Val Tyr Thr Phe Cys Cys
   20 25 30
<210> 96
   <211> 7
   <212> PRT
   <213> Artificial Sequence
<220>
   <223> Synthetic
<400> 96
   Lys Leu Ala Lys Lys Leu Ala
   1 5
<210> 97
   <211> 7
   <212> PRT
   <213> Artificial Sequence
<220>
   <223> Synthetic
<400> 97
   Lys Leu Ala Lys Leu Ala Lys
   1               5
<210> 98
   <211> 21
   <212> PRT
   <213> Artificial Sequence
<220>
   <223> Synthetic
<400> 98
   Lys Ala Leu Lys Ala Leu Lys Lys Ala Leu Lys Ala Leu Lys Lys Ala
   1               5                   10                  15
   Leu Lys Ala Leu Lys
               20
<210> 99
   <211> 7
   <212> PRT
   <213> Artificial Sequence
<220>
   <223> Synthetic
<400> 99
   Lys Leu Gly Lys Lys Leu Gly
   1               5
<210> 100
   <211> 7
   <212> PRT
   <213> Artificial Sequence
<220>
   <223> Synthetic
<400> 100
   Lys Ala Ala Lys Lys Ala Ala
   1               5

## Description

### FIELD OF THE INVENTION

The present invention relates to a process for manufacturing a wound active nanoscale polymer matrix microsheet.

### BACKGROUND OF THE INVENTION

The primary goal in the treatment of wounds is to achieve wound closure. Open cutaneous wounds represent one major category of wounds and include burn wounds, wounds resulting from chemical (especially alkali) burns, wounds from physical trauma, neuropathic ulcers, pressure sores, venous stasis ulcers, and diabetic ulcers. Open cutaneous wounds routinely heal by a process which comprises six major components: i) inflammation, ii) fibroblast proliferation, iii) blood vessel proliferation, iv) connective tissue synthesis, v) epithelialization, and vi) wound contraction. Wound healing is impaired when these components, either individually or as a whole, do not function properly. Numerous factors can affect wound healing, including but not limited to malnutrition, systemic debility due to a variety of causes, wound infection, local lack of progenitor cells, local and/or systemic pharmacological agents (e.g., numerous chemotherapeutic agents, actinomycin and steroids), repeated local trauma, diabetes and other endocrine/metabolic diseases (e.g., Cushing's disease), and advanced age (Hunt and Goodson, 1988, Current Surgical Diagnosis & Treatment, Appleton & Lange, pp. 86-98). Additionally, wounds that are extensive in size, regardless of the initiating cause, present special challenges due to the large surface area that must be re-epithelialized to re-establish surface integrity.

Delayed wound healing causes substantial morbidity in subjects with diabetes. Diabetes mellitus is a chronic disorder of glucose metabolism and homeostasis that damages many organs. It is the eighth leading cause of death in the United States (Harris et al., 1987, Diabetes 36:523). In persons with diabetes, vascular disease, neuropathy, infections, and recurrent trauma predispose the extremities, especially the foot, to pathologic changes. These pathological changes can ultimately lead to chronic ulceration, which may necessitate amputation. Chronic wounds and wounds with pathological or dysregulated healing represent a major health burden and drain on health care resources. Chronic wounds have major impacts on the physical and mental health, productivity, morbidity, mortality and cost of care for affected individuals. The most common types of chronic wounds are caused by systemic diseases such as diabetes, vascular problems such as venous hypertension and by immobility-induced pressure sores; accounting for 70% of all chronic wounds. Statistics on the prevalence of chronic wounds varies, however studies report that 0.2% to 1% of the population suffer from venous ulcers, 0.5% from pressure ulcers, and 5% to 10% of people with diabetes experience neuropathic ulcers. The economic impact of chronic wounds for these conditions alone in the United States has been estimated to be well over $15 billion, annually. With the population growing older, cases of diabetes mellitus will increase as will the magnitude of the problem associated with chronic wounds in these patients.

Normal wound healing is an enormously complex process involving the coordinated interplay between fibroblasts, vascular cells, extracellular matrix and epithelial cells to result in a seamless progression through an inflammatory reaction, wound repair, contracture and coverage by an epithelial barrier. However, in many patients, due to either the local wound environment or systemic disease or other factors, the wound healing processes can become asynchronous (i.e., loss of connectivity with triggering mechanisms associated with prior cellular events) and are unable to progress to closure, resulting in a chronic ulcer.

Wounds that do not readily heal can cause the subject considerable physical, emotional, and social distress as well as great financial expense (Richey et al., 1989, Annals of Plastic Surgery 23:159). Indeed, wounds that fail to heal properly and become infected may require excision of the affected tissue. A number of treatment modalities have been developed as scientists' basic understanding of wounds and wound healing mechanisms has progressed.

The most commonly used conventional modality to assist in wound healing involves the use of wound dressings. In the 1960s, a major breakthrough in wound care occurred when it was discovered that wound healing with moist, occlusive dressings was, generally speaking, more effective than the use of dry, non-occlusive dressings (Winter, 1962, Nature 193:293). Today, numerous types of dressings are routinely used, including films (e.g., polyurethane films), hydrocolloids (hydrophilic colloidal particles bound to polyurethane foam), hydrogels (cross-linked polymers containing about at least 60% water), foams (hydrophilic or hydrophobic), calcium alginates (nonwoven composites of fibers from calcium alginate), and cellophane (cellulose with a plasticizer) (Kannon and Garrett, 1995, Dermatol. Surg. 21:583; Davies, 1983, Burns 10:94). Unfortunately, certain types of wounds (e.g., diabetic ulcers, pressure sores) and the wounds of certain subjects (e.g., recipients of exogenous corticosteroids) do not heal in a timely manner (or at all) with the use of such dressings.

Several pharmaceutical modalities have also been utilized in an attempt to improve wound healing. For example, some practitioners have utilized treatment regimens involving zinc sulfate. However, the efficacy of these regimens has been primarily attributed to their reversal of the effects of sub-normal serum zinc levels (e.g., decreased host resistance and altered intracellular bactericidal activity) (Riley, 1981, Am. Fam. Physician 24:107). While other vitamin and mineral deficiencies have also been associated with decreased wound healing (e.g., deficiencies of vitamins A, C and D; and calcium, magnesium, copper, and iron), there is no strong evidence that increasing the serum levels of these substances above their normal levels actually enhances wound healing. Thus, except in very limited circumstances, the promotion of wound healing with these agents has met with little success.

Current clinical approaches used to promote healing in dysregulated wounds include protection of the wound bed from mechanical trauma (e.g. splinting, bandaging), meticulous control of surface microbial burden (antibiotics, antimicrobial peptides, bacteriophages, antiseptics and other antimicrobial compounds that broadly inhibit wound pathogens (e.g., silver sulfadiazine) combined with topical application of soluble cytoactive factors (e.g. growth factors exemplified by but not limited to epidermal growth factor-EGF, exogenous extracellular matrix constituents such as fibronectin), surgical excision of the wound margin or entire bed and surgical placement of tissue flaps and/or autografts, allografts and xenografts. All of these approaches fall short of promoting optimal healing conditions in many of the most challenging wounds. It is likely that a major contributing factor to the failure of these traditional approaches is the fact that they do not alter the intrinsic chemistry/structure of the wound bed itself that has been shown in many cases to contribute significantly to their persistence. Additionally, the historical use of a single factor or set of factors to treat all wounds often falls short due to the great heterogeneity found in wound beds themselves and the complex environment of the wound itself containing a community of signaling molecules that frequently modulate the activity of individual molecules.

Of broad-spectrum bactericidal agents, silver is considered particularly favorable because the likelihood of developing bacterial resistance to silver is believed to be very low; therefore, it can be employed as a bactericidal agent continuously. However, currently available methods of applying silver as a bactericidal agent for wound treatment are inadequate. For example, 0.5% silver nitrate solution is a standard and popular agent for topical burn wound therapy, providing a beneficial effect in decreasing wound surface inflammation. However, while such formulations have a high concentration of silver, there is no residual activity, necessitating frequent applications (e.g., up to 12 times a day) which poses a severe logistical burden in clinical settings. Silver ions released through use of 0.5% silver nitrate solution become rapidly inactive through formation of chemical complexes by chloride within 2 hours. Frequent dressings also result in large excesses of silver being delivered to the wound, causing wound-discoloration and toxic effects (Dunn et al., 2004, Burns 30(supplement 1):S1). Additionally, nitrate is toxic to wounds and to mammalian cells. The reduction of nitrate to nitrite further causes oxidant-induced damage to cells, which is cited as the most likely reason for the impaired re-epithelialization with use of silver nitrate solution in partial thickness burns or donor sites.

Silver compounds such as silver sulfadiazine in cream formulations (e.g., Flammazine®, silvadene®) have also been used for wound treatment. However, such formulations also have limited residual activity and have to be applied twice a day. Bacterial resistance does develop to these formulations, and, impaired re-epithelialization has also been observed. Bone marrow toxicity has been observed with silver sulfadiazine, primarily due to its propylene glycol component.

Additionally, in some methods, silver itself is incorporated into the dressing instead of being applied as a separate formulation. Controlled and prolonged release of silver to the wound allows dressings to be changed less frequently. However, dressings have to be impregnated with large amount of silver, which results in cytotoxicity to mammalian cells. Silver released from a commercially available wound-dressing (Acticoat™) containing nanocrystalline silver (Dunn et al., 2004, Burns 30(supplement 1):S1) is toxic to *in vitro* monolayer cell cultures of keratinocytes and fibroblasts (Poon et al., 2004, Burns 30:140; Trop et al., 2006, J. Trauma 60:648).

The complex nature of pathologic wounds, and the lack of significant clinical progress based on current therapies, indicates the urgent need for new and unconventional approaches. What is needed are safe, effective, and interactive means for enhancing the healing of chronic and severe wounds. The methods should be adaptable without regard to the type of wound, or the nature of the patient population, to which the subject belongs.

US 2011/0189287 A1 discloses methods and compositions for wound healing utilizing cross-linker covalent modification molecules to attach and deliver wound active agents to a wound.

### SUMMARY OF THE INVENTION

The present invention relates to a process for manufacturing a wound active nanoscale polymer matrix micro sheet as claimed in the attached claims. The wound active agents disclosed for use in the process herein are highly bactericidal but that support growth and viability of mammalian cells (e.g., keratinocytes, neurons, vascular endothelial cells and fibroblasts cells). In preferred embodiments, the wound active agent is silver, including but not limited to silver nanoparticles. In particularly preferred embodiments, the silver loading of some embodiments of the present invention is between 0.35 and 0.4 µg/cm². Polyelectrolyte multilayer micro sheets produced according to the present invention comprising silver (e.g., silver nanoparticles) find use in the treatment of wounds and the prevention of infection, including but not limited to as coatings on devices that come partially, directly, indirectly, or completely in contact with the body of a subject (e.g., a human patient).

In some embodiments, the at least one wound active agent includes, but is not limited to, trophic factors (including polypetide growth factors, neuropeptides, neurotrophins, , extracellular matrices and their individual native constituents (exemplified by but not limited to laminin, fibronectin, vitronectin, collagens, also select amino acid sequences found in these proteins known to promote cell behaviors favorable to wound healing e.g., integrin binding sequences exemplified by but not limited to RGD, EILDV,VCAM-1 and their recombined or synthetic analogs, enzymes, enzyme inhibitors, polypeptides, antimicrobial peptides ( exemplified by but not limited to defensins, magaignins, cathelocidins, bactenicin) anti-infective agents including silver containing compounds (e.g., ionic silver, elemental silver, silver nanoparticles, and formulations thereof), buffering agents, vitamins and minerals, compounds that promote generation/stabilization of nitic oxide, energy sources for cells, analgesics, anticoagulants, coagulation factors, anti-inflammatory agents, vasoconstrictors, vasodilators, diuretics, and anti-cancer agents. In other embodiments the kits include small interfering RNAs (siRNAs-also referred to as micro RNAs) that are capable of promoting cellular behaviors conducive to the wound healing process. In other embodiments, the kits include compounds that promote/stabilize a favorable pH, osmotic environment, surface energy, surface charge, surface functionalities that enhance galvano and magneto positive effects on wound healing, or balance of MMP/other peptidase/protease activity.

The present invention provides processes for manufacture of a nanoscale polymer matrix microsheet comprising: a) forming a nanoscale polymer layer about 0.5 nm to 1000 nm thick on a substrate; b) introducing a bioactive agent into the nanoscale polymer layer to provide a wound active nanoscale polymer layer, wherein the bioactive agent is a wound active agent; c) forming a sacrificial polymer layer from 0,1µm thick to 100 µm thick on the wound active nanoscale polymer layer, wherein the sacrificial polymer layer is dissolvable.

In some embodiments, the nanoscale polymer matrix is a polymer multilayer. In some embodiments, the nanoscale polymer matrix is formed by alternating layers of at least one positively charged electrolyte and at least one negatively charged polyelectrolyte. In some embodiments, the at least one positively charged polyelectrolyte is selected form the group consisting of poly(allylamine hydrochloride) (PAH), polyl-lysine (PLL), poly(ethylene imine) (PEI), poly(histidine), poly(N,N-dimethyl aminoacrylate), poly(N,N,N-trimethylaminoacrylate chloride), poly(methyacrylamidopropyltrimethyl ammonium chloride), and natural or synthetic polysaccharides such as chitosan. In some embodiments, the at least one negatively charged polyelectrolyte is selected from the group consisting of poly(acrylic acid) (PAA), poly(styrenesulfonate) (PSS), alginate, hyaluronic acid, heparin, heparan sulfate, chondroitin sulfate, dextran sulfate, poly(methacrylic acid), oxidized cellulose, carboxymethyl cellulose, polyaspartic acid, and polyglutamic acid. In some embodiments, the polymer multilayer is formed by applying the at least one positively charged electrolyte and at least one negatively charged polyelectrolyte by a method selected from the group consisting of spraying polymer solutions on the substrate, dip coating the substrate in polymer solutions, or spin coating polymer solutions on the substrate. In some embodiments, the at least one positively charged electrolyte and the at least one negatively charged polyelectrolyte are synthetic polyelectrolytes.

In some embodiments, the wound active agent is incorporated into the nanoscale polymer layer so that the wound active agent is interspersed within the three dimensional structure of the nanoscale polymer layer. In some embodiments, the wound active agent is incorporated into the nanoscale polymer multilayer so that the wound active agent is interspersed within the layers the polymer multilayer. In some embodiments, the wound active agent is selected from the group consisting of an antimicrobial agent, an antibiofilm agent, a growth factor, a hemostatic agent, a wound active peptide, a wound active polypeptide, an analgesic, an anticoagulant, anti-inflammatory agent, and a drug molecule or a drug compound. In some embodiments, the antimicrobial agent is selected from the groups consisting of charged small molecule antimicrobial agents, antimicrobial polypeptides, metallic particles, and metal ion antimicrobial agents. In some embodiments, the metal ion antimicrobial agent is a metal ion, metal ion salt, or metal ion nanoparticle. In some embodiments, the metal ion nanoparticle is a silver nanoparticle. In some embodiments, the charged small molecule antimicrobial agent is selected from the group consisting of chlorhexidine, antibiotics, polyhexamethylene biguanide (PHMB), iodine, cadexomer iodine, povidone iodine (PVI), hydrogen peroxide, and vinegar (acetic acid). In some embodiments, the metal ion antibiofilm agent is a metal ion, a metal ion salt, or a metal ion nanoparticle. In some embodiments, the metal ion antibiofilm agent is a gallium salt, gallium nanoparticle, gallium alloy, or an alloy of gallium and silver. In some embodiments, the bioactive agent is introduced into the nanoscale polymer layer during the formation of the nanoscale polymer layer. In some embodiments, the bioactive agent is introduced into the nanoscale polymer layer after formation of the nanoscale polymer layer. In some embodiments, introducing a bioactive agent into the nanoscale polymer layer to provide a bioactive nanoscale polymer multilayer comprises introducing silver ions into the nanoscale polymer multilayer and reducing the silver ions in situ to provide silver nanoparticles. In some embodiments, introducing a bioactive agent into the nanoscale polymer layer to provide a bioactive nanoscale polymer multilayer comprises introducing a charged small molecule antimicrobial agent in between polyelectrolyte layers having a different charge. In some embodiments, the process comprises 1 to 20 repititions of the introducing step, e.g., to control and/or increase the amound of bioactive agent introduced into the nanoscale polymer layer. In some embodiments, the process comprises controlling the amount of the bioactive agent in the nanoscale polymer matrix microsheet by controlling the number of nanoscale polymer layers, by controlling the pH of forming the nanoscale polymer layer, and/or by controlling the number of introducing cycles.

The sacrificial polymer layer is from about 0.1 µm thick to about 100 µm thick. The sacrificial polymer layer comprises a water soluble polymer. In some embodiments, the water soluble sacrificial polymer layer is made of and/or comprises polyvinyl alcohol (PVA). In some embodiments, the soluble polymer has a molecular weight of greater than 22 kDa. In some embodiments, the water soluble polymer is removable by renal filtration.

In some embodiments, the water soluble sacrificial polymer layer is made of polyacrylic acid (PAA). In some embodiments, the sacrificial polymer layer comprising a water soluble polymer dissolves when exposed to moisture on a surface so that the bioactive nanoscale polymer multilayer is deposited on the surface. In some embodiments, the processes further comprise introducing a bioactive agent into the sacrificial polymer layer. In some embodiments, the processes further comprise introducing microparticles or nanoparticles into the sacrificial polymer layer. In some embodiments, the microparticles or nanoparticles in the sacrificial polymer layer are loaded with bioactive agents. In some embodiments, the processes further comprise introducing magnetic microparticles or nanoparticles into the sacrificial polymer layer. In some embodiments, the bioactive agent is selected from the group consisting of an antimicrobial agent, a growth factor, a hemostatic agent, a bioactive peptide, a bioactive polypeptide, an analgesic, an anticoagulant, anti-inflammatory agent, and a drug molecule or a drug compound. In some embodiments, the antimicrobial agent is selected from the goups consisting of charged small molecule antimicrobial agents, antimicrobial polypeptides, metallic particles, and metal ion antimicrobial agents. In some embodiments, the metal ion antimicrobial reagent is a metal ion, metal ion salt, or metal ion nanoparticle. In some embodiments, the metal ion nanoparticle is a silver nanoparticle. In some embodiments, the small molecule antimicrobial agent is selected from the group consisting of chlorhexidine, antibiotics, polyhexamethylene biguanide (PHMB), iodine, cadexomer iodine, povidone iodine (PVI), hydrogen peroxide, vinegar (acetic acid). In some embodiments, the substrate is selected from the group consisting of a polydimethylsiloxane (PDMS) substrate, a glass substrate, a plastic substrate, a metal substrate, and a Teflon substrate. In some embodiments, the substrate is functionalized to provide a low-energy surface. In some embodiments, the substrate is functionalized with octadecyltricrhlorosilane.

In some embodiments, the processes further comprise the step of removing the nanoscale polymer layer matrix microsheet comprising the wound active nanoscale polymer layer and associated sacrificial polymer layer from the substrate. In some embodiments, greater than 90%, greater than 95%, greater than 97%, greater than 98%, or greater than 99% of the nanoscale polymer matrix microsheet comprising the wound active nanoscale polymer layer and associated sacrificial polymer layer is removed from said substrate. In some embodiments, the processes further comprise incorporating nanoscale or microscale particles or beads into the nanoscale polymer layer. In some embodiments, the processes further comprise incorporating magnetic nanoparticles or microparticles into the nanoscale polymer layer.

In some embodiments, the nanoscale polymer layer comprises a polymer selected from the group consisting of polyelectrolytes, lipids, proteins, collagen, hyaluornic acid, chitosan, keratin, fibronectin, vitronectin, laminin, polysaccharides, polyanhydrides, poly (lactic-co-glycolic acid) (PLGA), poly(1-lactic acid) (PLLA), and combinations thereof or fragments thereof. In some embodiments, the sacrificial polymer layer is applied to the nanoscale polymer layer by spin coating, dip coating, or spray coating. In some embodiments, the nanoscale polymer matrix microsheet has a Young modulus of from about 0.1 GPa to about 10 GPa after removal from the substrate. In some embodiments, forming the nanoscale polymer layer comprises depositing a positively charged electrolyte and a negatively charged polyelectrolyte to form a bilayer. In some embodiments, the processes comprise 2 to 50 depositing steps. In some embodiments, forming the nanoscale polymer layer comprises a step of providing solution conditions comprising a pH from 1.5 to 2.5, a pH from 6.5 to 8.5, and/or a pH from 4.5 to 6.5. In some embodiments, the processes further comprise drying the nanoscale polymer matrix microsheet. In some embodiments, the process further comprises indroducing a second or more bioactive agent(s) into the nanoscale polymer layer.

With the process of the invention, a microsheet is manufactured comprising: a wound active nanoscale polymer layer about 0.5 nm to 1000 nm thick, the nanoscale wound active polymer layer having a wound active agent incorporated therein; and a sacrificial polymer layer adjacent to the wound active nanoscale polymer layer, the sacrificial polymer layer being from 0,1 µm thick to 100 µm thick on said wound active nanoscale polymer layer and being dissolvable when exposed to a moist surface. In some embodiments, the nanoscale polymer layer is a polymer multilayer. In some embodiments, the nanoscale polymer multilayer is formed by alternating layers of at least one positively charged electrolyte and at least one negatively charged polyelectrolyte. In some embodiments, the at least one positively charged polyelectrolyte is selected form the group consisting of poly(allylamine hydrochloride) (PAH), polyl-lysine (PLL), poly(ethylene imine) (PEI), poly(histidine), poly(N,N-dimethyl aminoacrylate), poly(N,N,N-trimethylaminoacrylate chloride), poly(methyacrylamidopropyltrimethyl ammonium chloride), and natural or synthetic polysaccharides such as chitosan. In some embodiments, the at least one negatively charged polyelectrolyte is selected from the group consisting of poly(acrylic acid) (PAA), poly(styrenesulfonate) (PSS), alginate, hyaluronic acid, heparin, heparan sulfate, chondroitin sulfate, dextran sulfate, poly(meth)acrylic acid, oxidized cellulose, carboxymethyl cellulose, polyaspartic acid, and polyglutamic acid. In some embodiments, the at least one positively charged electrolyte and the at least one negatively charged polyelectrolyte are synthetic polyelectrolytes. In some embodiments, the wound active agent is incorporated into the nanoscale polymer layer so that the wound active agent is interspersed within the three dimensional structure of the nanoscale polymer layer. In some embodiments, the wound active agent is incorporated into the nanoscale polymer multilayer so that the wound active agent is interspersed within the layers the nanoscale polymer multilayer.

In some embodiments, the wound active agent is selected from the group consisting of an antimicrobial agent, a growth factor, a hemostatic agent, a bioactive peptide, a bioactive polypeptide, an analgesic, an anticoagulant, anti-inflammatory agent, and a drug molecule or a drug compound. In some embodiments, the antimicrobial agent is selected from the goups consisting of small molecule antimicrobial agents, charged small molecule antimicrobial agents, antimicrobial polypeptides, metallic particles, and metal ion antimicrobial agents. In some embodiments, the metal ion antimicrobial reagent is a metal ion, metal ion salt, or metal ion nanoparticle. In some embodiments, the metal ion nanoparticle is a silver nanoparticle. In some embodiments, the charged small molecule antimicrobial agents or small molecule antimicrobial agent is selected from the group consisting of chlorhexidine, antibiotics, polyhexamethylene biguanide (PHMB), iodine, cadexomer iodine, povidone iodine (PVI), hydrogen peroxide, vinegar (acetic acid). The sacrificial polymer layer is from about 0.1 µm thick to about 100 µm thick. In some embodiments, the sacrificial polymer layer is from about 0.1 µm thick to about 50 µm thick. In some embodiments, wherein the sacrificial polymer layer is from about 1 µm thick to about 20 µm thick. In some embodiments, the sacrificial polymer layer is from about 1 µm thick to about 10 µm thick. In some embodiments, the sacrificial polymer layer comprises a water soluble polymer. In some embodiments, the sacrificial polymer layer comprises a wound active agent. In some embodiments, the wound active agent is selected from the group consisting of an antimicrobial agent, a growth factor, a hemostatic agent, a bioactive peptide, a bioactive polypeptide, an analgesic, an anticoagulant, anti-inflammatory agent, and a drug molecule, or a drug compound. In some embodiments, the antimicrobial agent is selected from the groups consisting of small molecule antimicrobial agents, antimicrobial polypeptides, metallic particles, and metal ion antimicrobial agents. In some embodiments, the metal ion antimicrobial reagent is a metal ion, metal ion salt, or metal ion nanoparticle. In some embodiments, the metal ion nanoparticle is a silver nanoparticle. In some embodiments, the small molecule antimicrobial agent is selected from the group consisting of chlorhexidine, antibiotics, polyhexamethylene biguanide (PHMB), iodine, cadexomer iodine, povidone iodine (PVI), hydrogen peroxide, and vinegar (acetic acid).

In some embodiments, the microsheets further comprise nanoscale or microscale particles incorporated into the nanoscale polymer layer.

In some embodiments, the nanoscale polymer layer comprises a polymer selected from the group consisting of polyelectrolytes, lipids, proteins, collagen, hyaluornic acid, chitosan, keratin, fibronectin, vitronectin, laminin, polysaccharides, polyanhydrides, poly (lactic-co-glycolic acid) (PLGA), poly(1-lactic acid) (PLLA), and combinations thereof or fragments thereof.

In some embodiments, the microsheet comprises the wound active agent at a concentration of approximately 0.01 to 100 µg/cm². In some embodiments, the bioactive agent is provided in an amount so that the wound active agent is released at a rate of about 0.01 and 100 µg/cm² per day. In some embodiments, the microsheet has an area of from about 0.2 to 600 cm².

In some embodiments, the sacrificial polymer layer has a uniform thickness with a variation of less than 500, 400, 300, 200, 100, 50, 20 or 10% of the average thickness when measured in cross section. In some embodiments, the microsheet has a Young modulus of from about 0.1 GPa to about 10 GPa, e.g., after removal from a substrate.

In some embodiments, when the article is applied to a surface, the sacrificial polymer layer dissolves when exposed to moisture to leave the nanoscale polymer layer on the surface. In some embodiments, the nanoscale polymer layer forms a barrier to exogenous pathogens when applied to a moist surface. In some embodiments, the microsheet further comprises a second or more wound active agent(s). In some embodiments, the microsheet is translucent and/or transparent.

The microsheet produced with the process of the invention can be used in a medical device comprising the microsheet produced as described above, wherein the medical device can be selected from the group consisting of a a wound dressing, an implantable medical device, a medical device that contacts skin, a catheter, a stent, a membrane, a contact lens, and a surgical mesh, wherein further the wound dressing can be a biologic wound dressing, an abiologic wound dressing, an absorbent material, a foam, a transparent thin film, a hydrogel, hydrofibers, hydrocolloids, alginate fibers, silica gel, sodium polyacrylate, potassium polyacrylamides, and combinations thereof.

### DESCRIPTION OF THE FIGURES

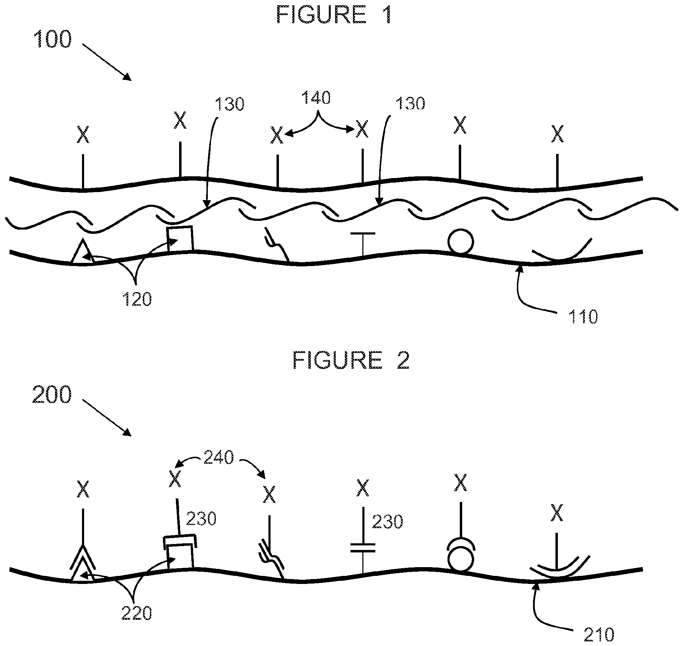

Figure 1 provides a schematic of a wound bed modified with a polyelectrolyte multilayer.
Figure 2 provides a schematic of a wound bed modified with covalent modifying agents.

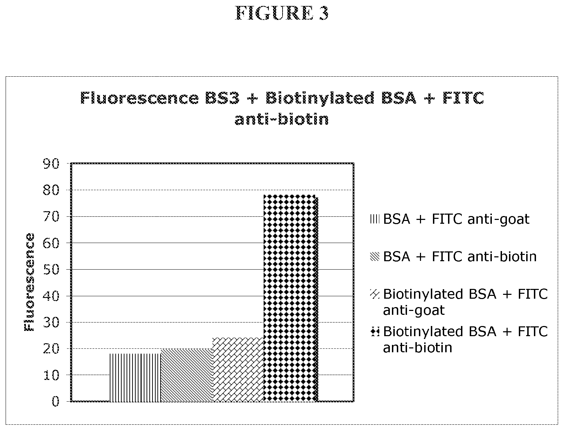

Figure 3 exemplifies the covalent immobilization of proteins to model amine-terminated treated glass surfaces.

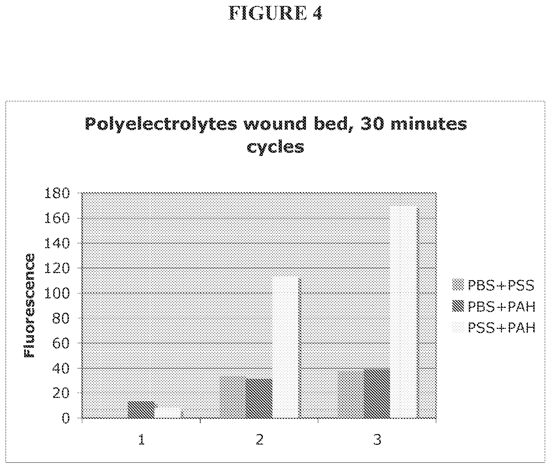

Figure 4 shows *ex vivo* results for multilayer deposition of polyelectrolytes polystyrene sulfonate (PSS) and FITC-labelled poly (allylamine hydrochloride) (FITC-PAH).

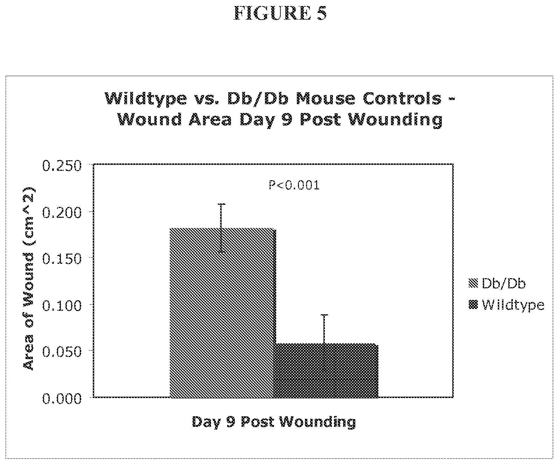

Figure 5 demonstrates the difference in healing of full thickness cutaneous wounds in diabetic (db/db) mice compared to control (wild type) mice.

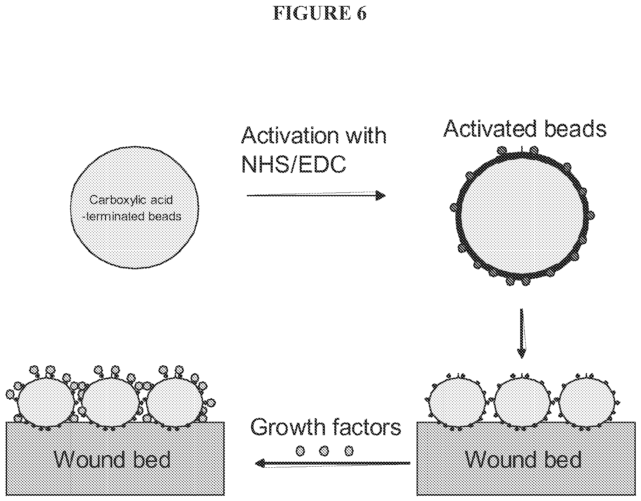

Figure 6 is exemplary of the use of beads for healing a wound bed. In this example, carboxylic acid-terminated beads are activated outside the wound bed by using NHS and EDC. The activated beads are introduced into the wound bed and are immobilized in the wound bed via reaction of the activated surfaces of the beads with amine groups present in the wound bed. Growth factors are introduced into the wound bed and immobilized via reaction with the exposed surfaces of the activated beads.

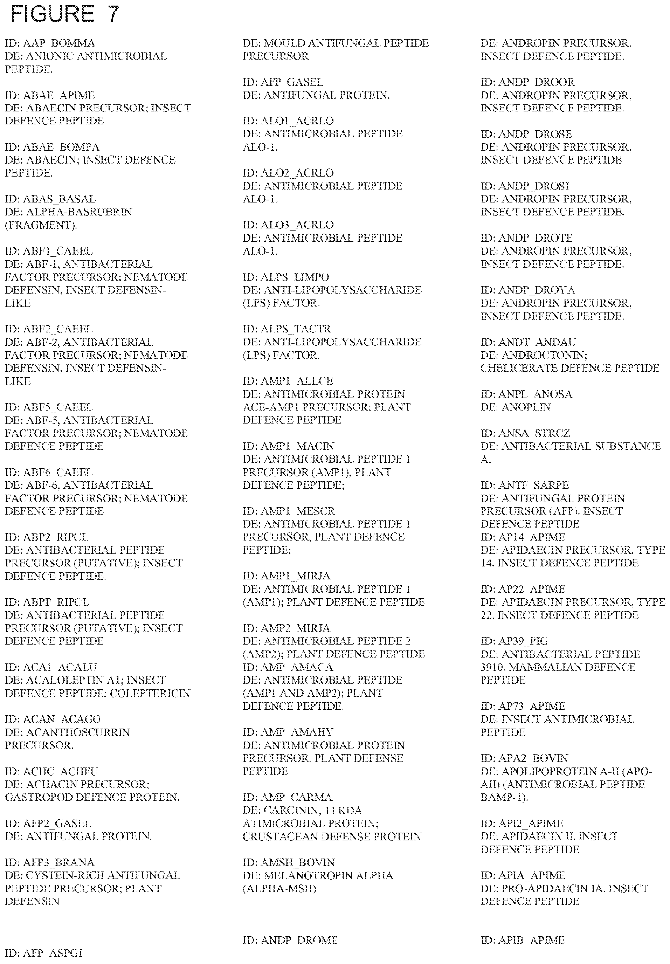

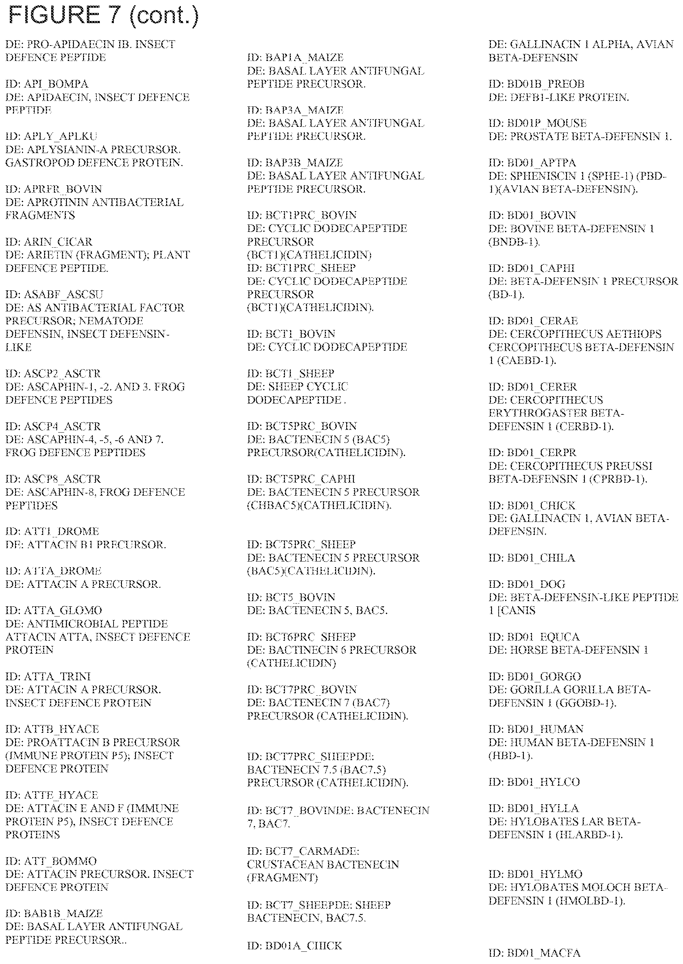

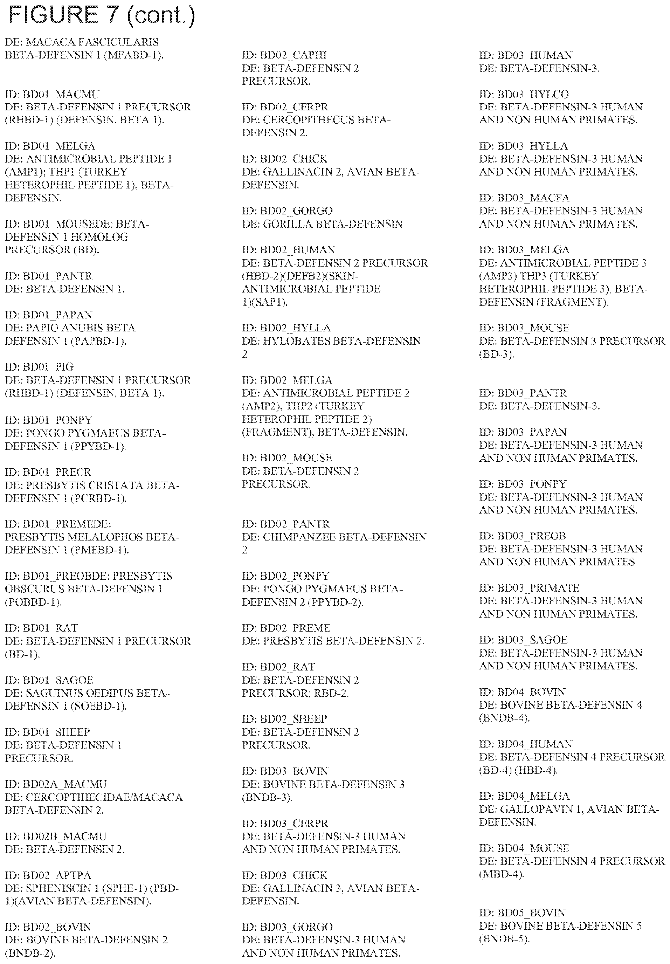

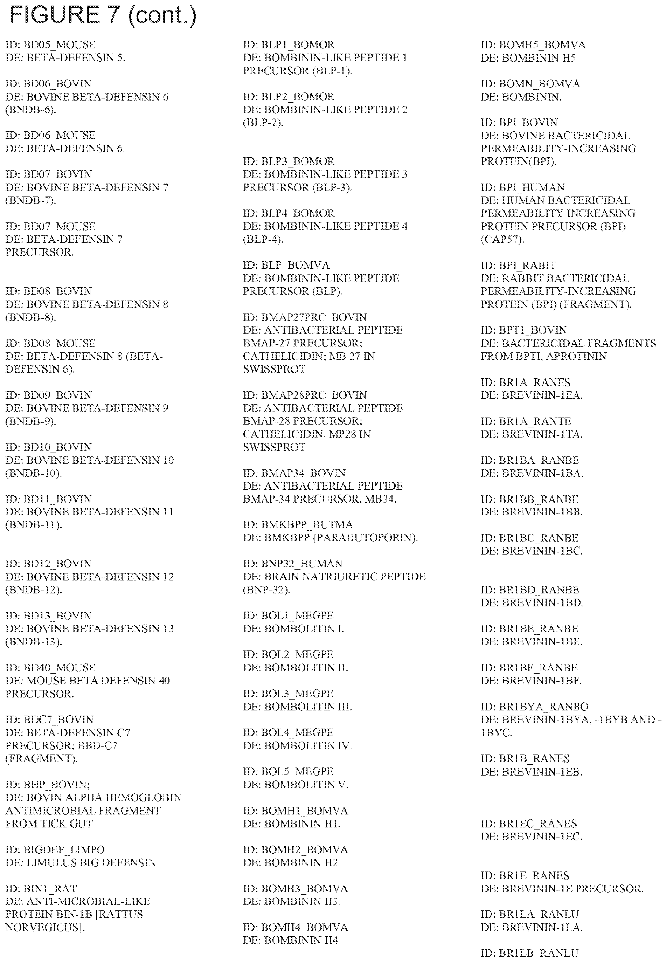

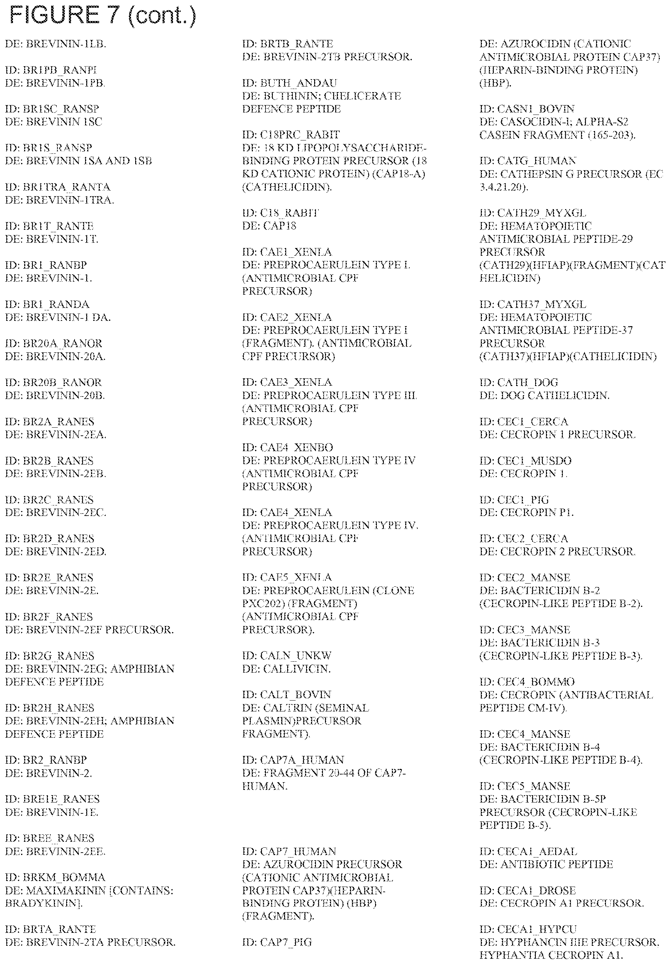

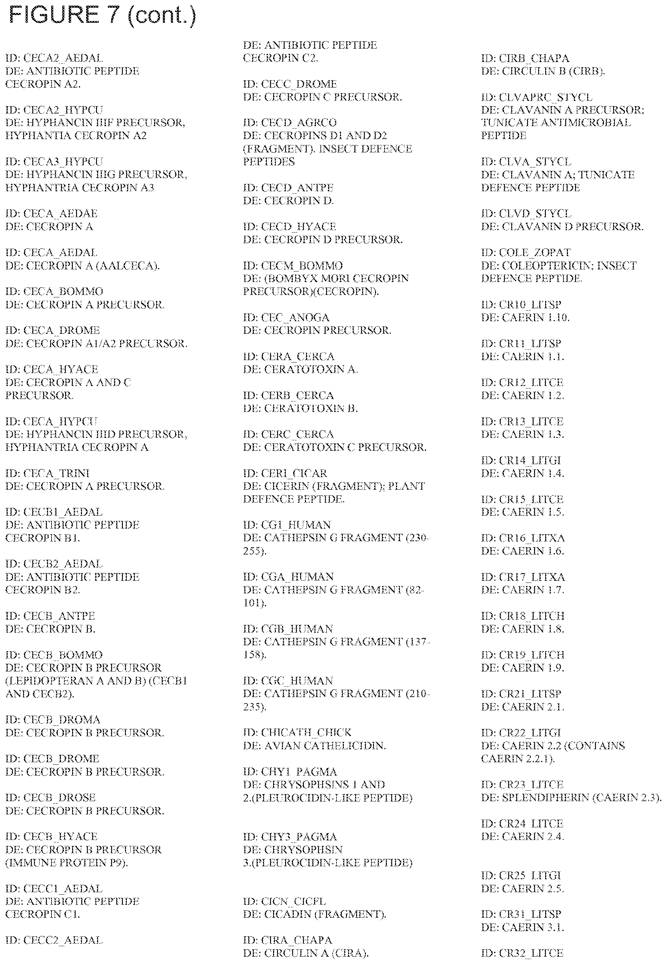

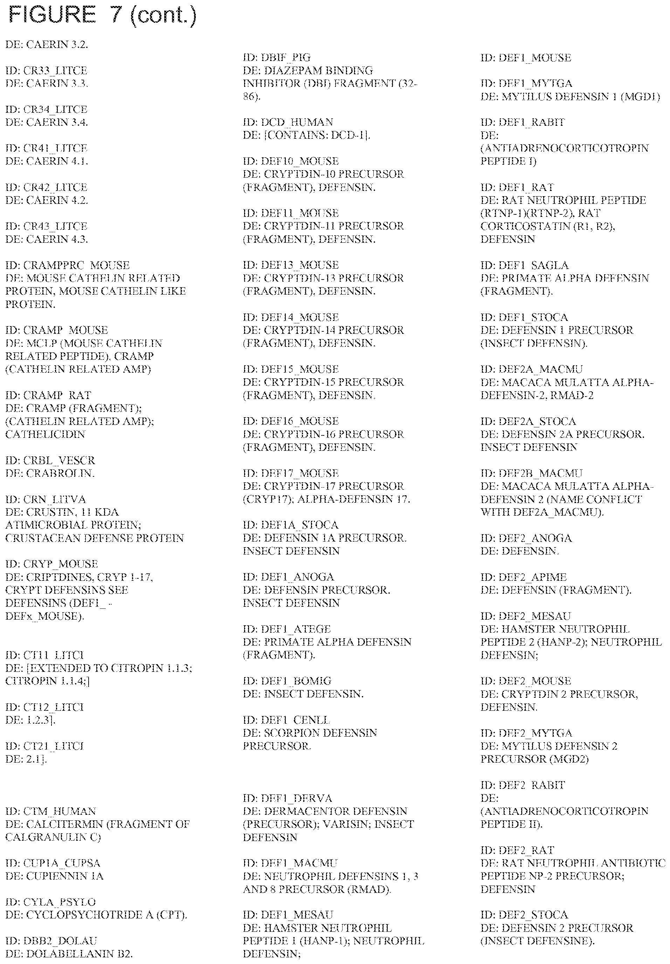

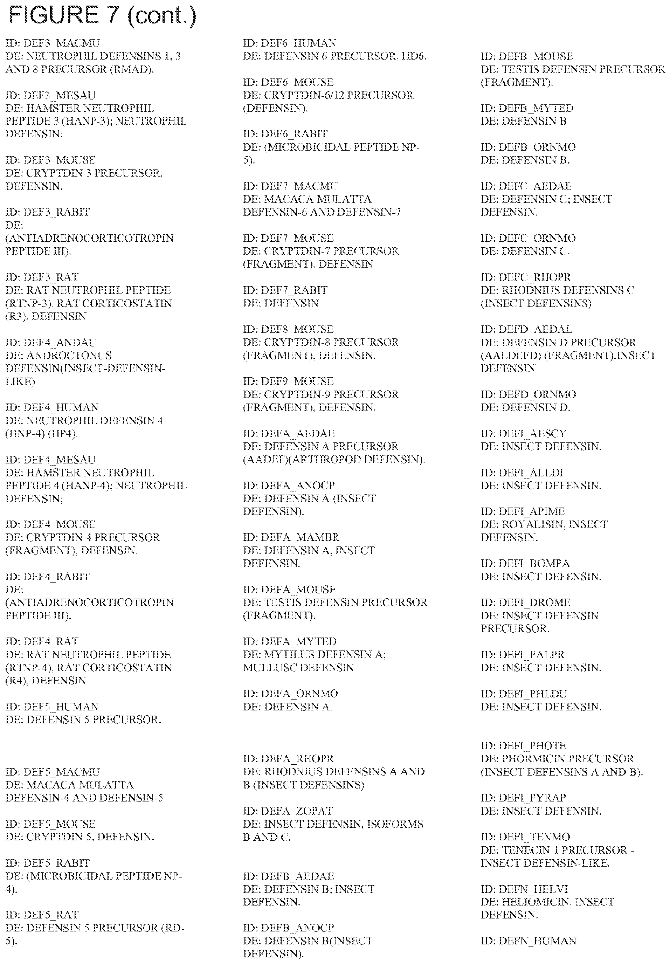

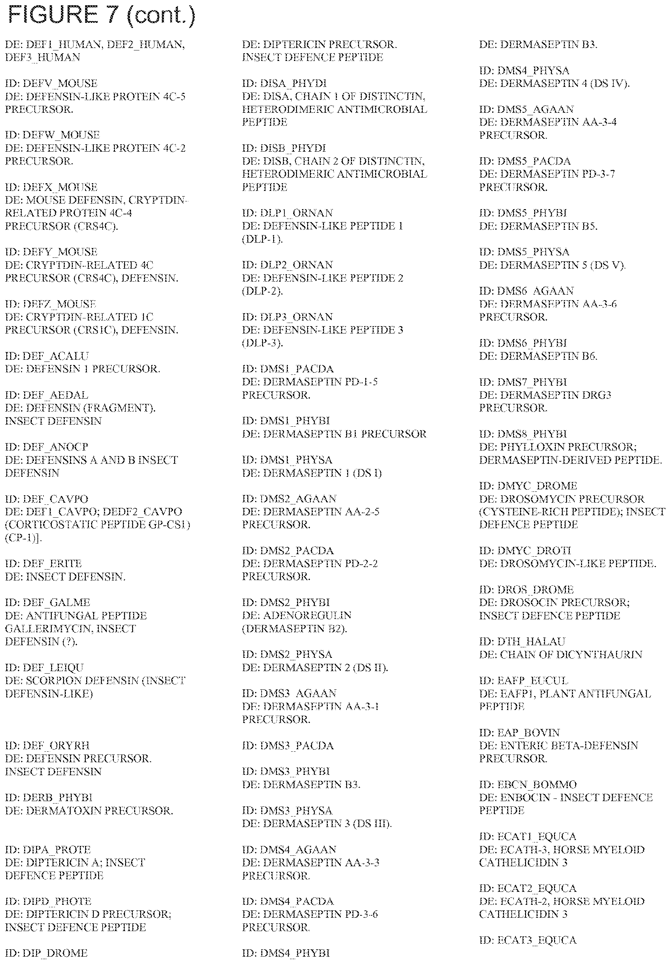

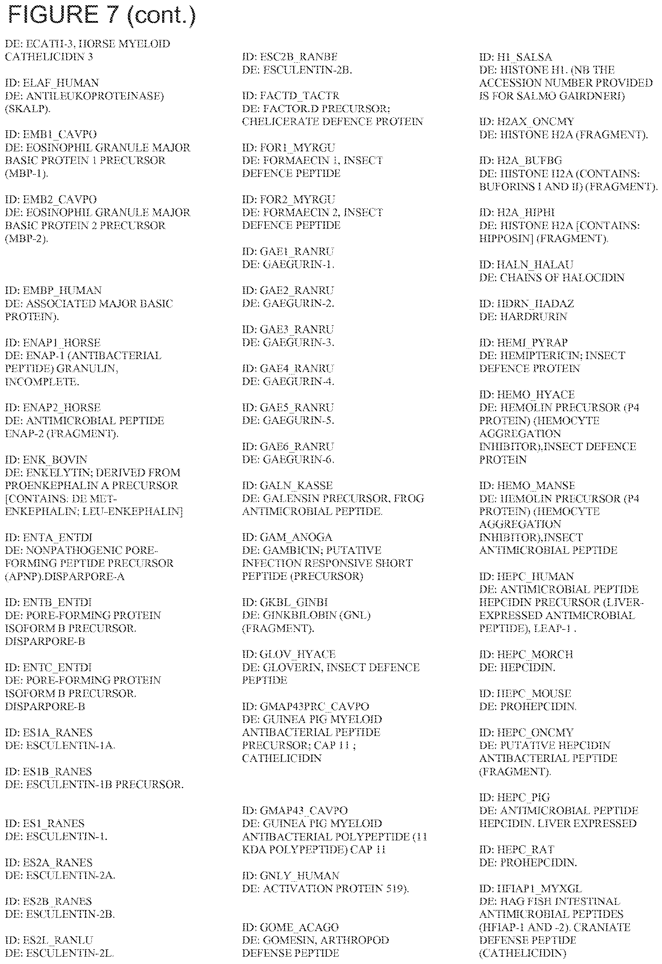

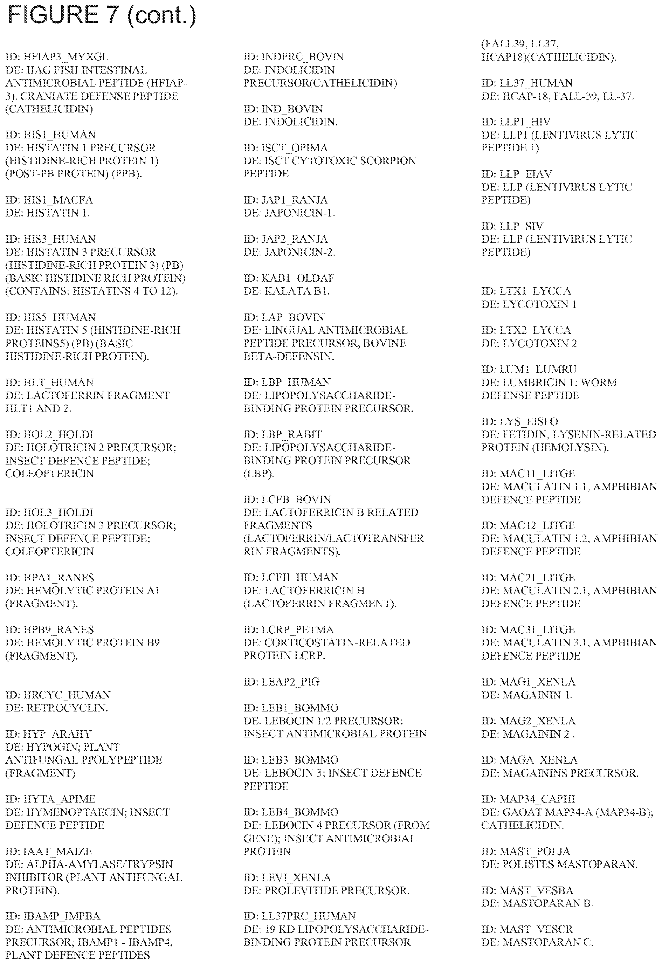

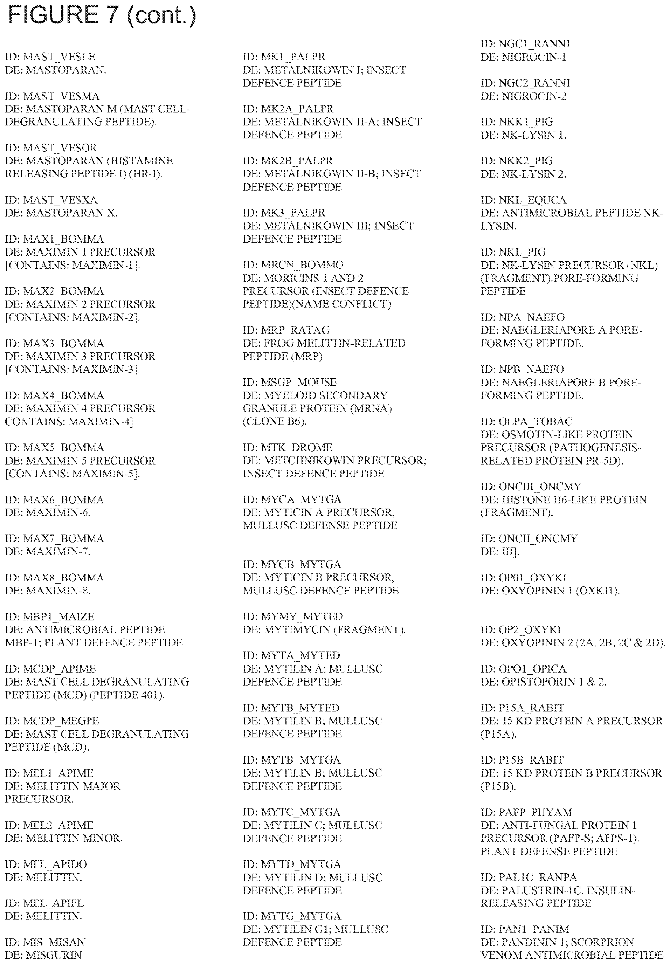

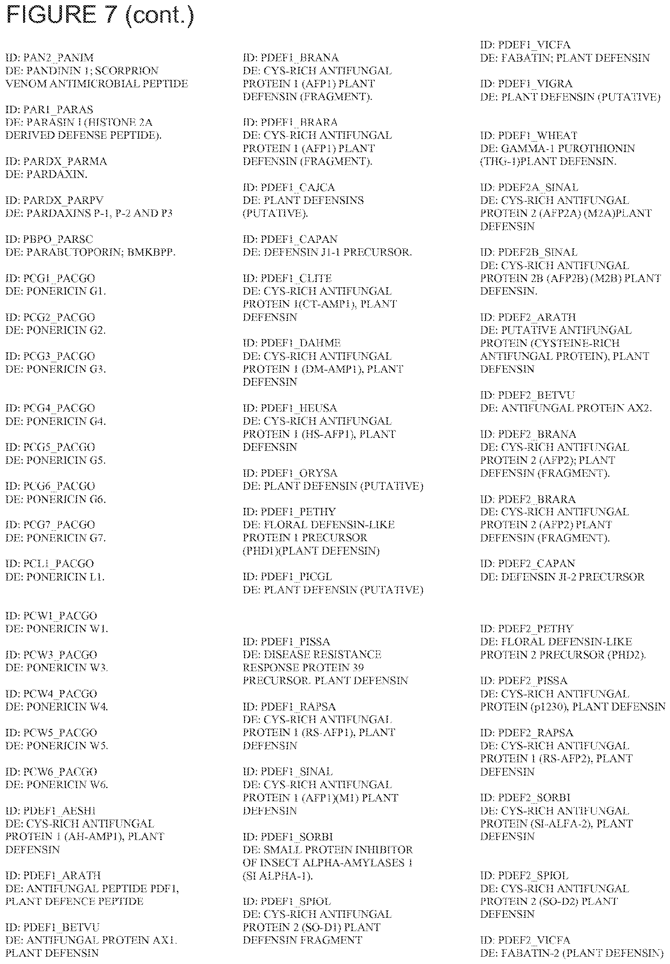

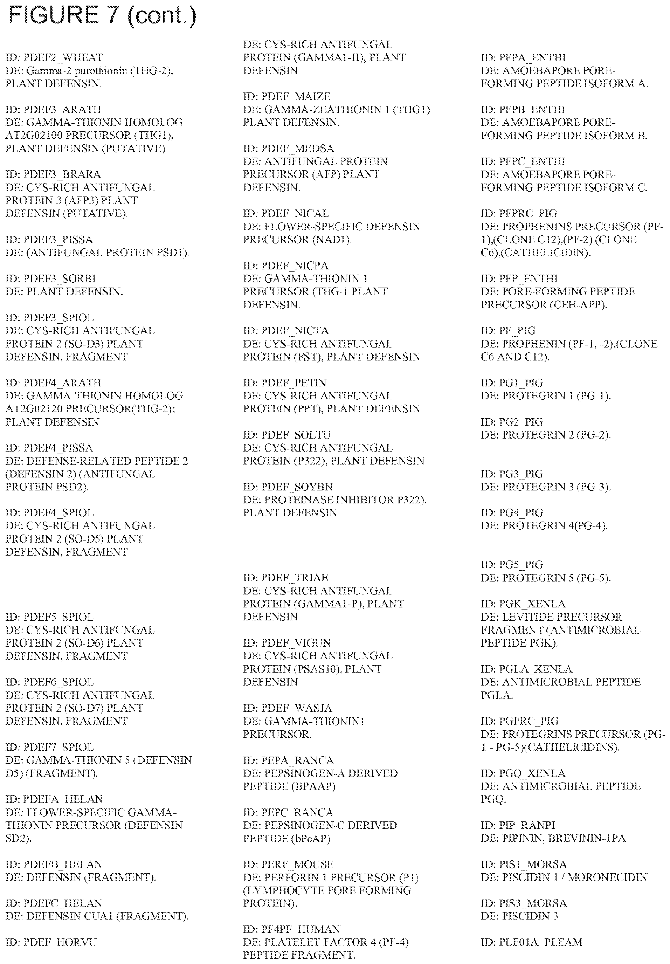

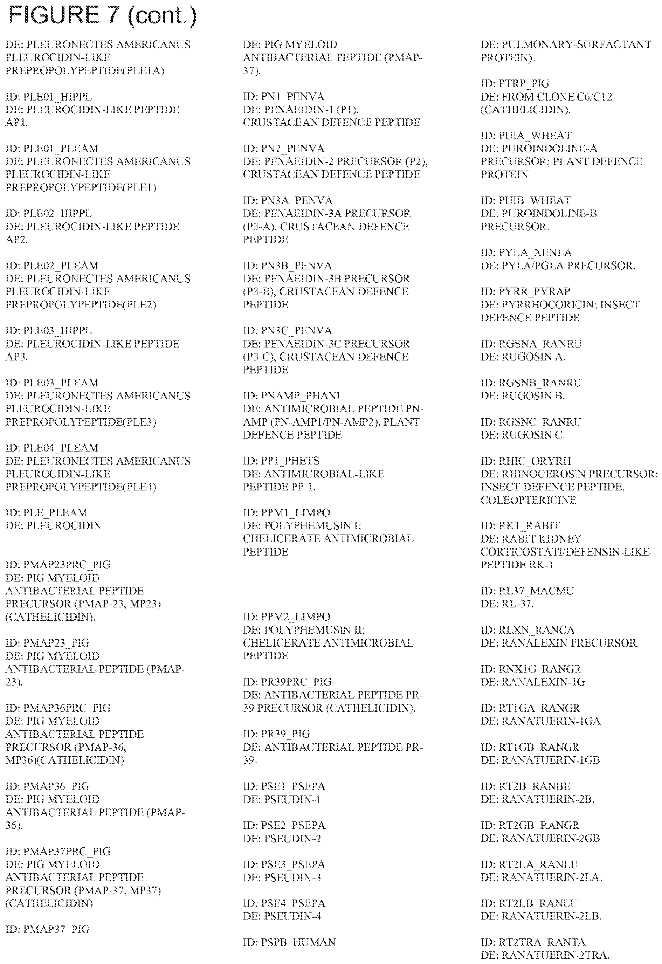

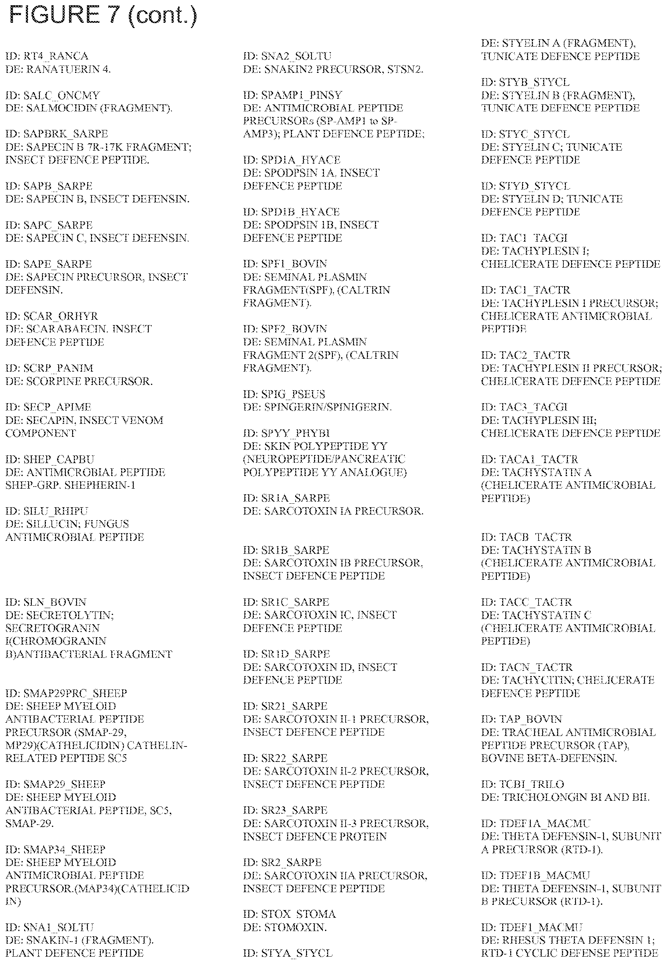

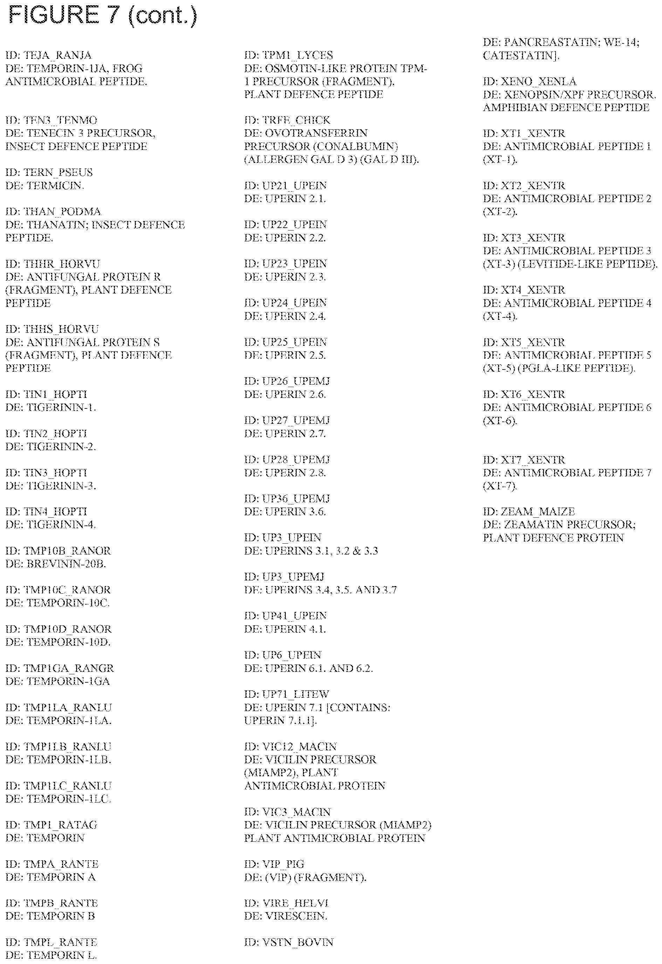

Figure 7 provides a list of antimicrobial polypeptides, identified by name and AMSDb database ID number.

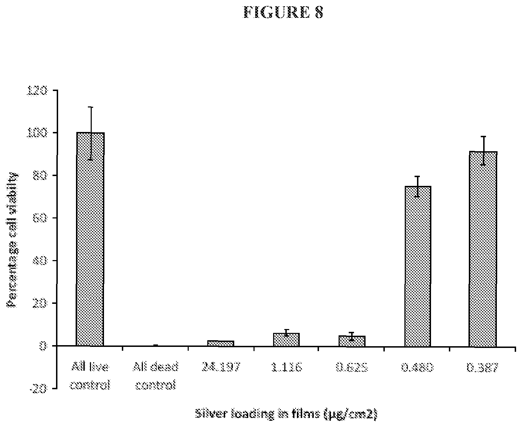

Figure 8 shows the viability of NIH-3T3 mouse fibroblasts after 24 hr incubation in growth media on silver-loaded polyelectrolyte multilayers (PEMs) (Example 13).

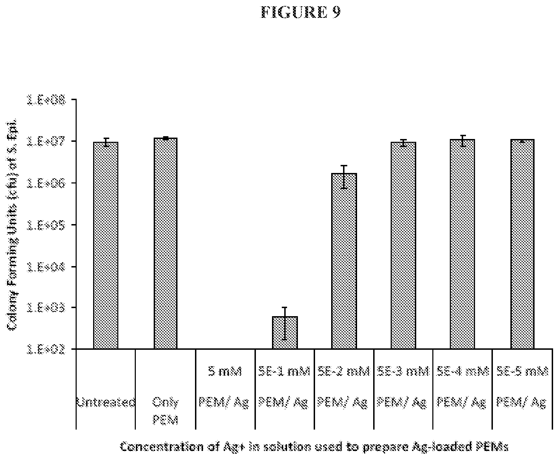

Figure 9 shows viable bacteria detected on silver-loaded PEMs 8 hr after incubation with 10⁸ cfu/ml of *S. epidermidis* in buffer. PEMs with varying silver loading were prepared by incubating them with serial decimal dilutions of bulk Ag⁺ solution. PEMs incubated with 5 mM Ag⁺ (silver nitrate) solution as indicated in this figure contained ~0.38 µg/cm² (where no cytotoxicity towards NIH-3T3 cells was observed in Figure 8), while silver loading in rest of the PEMs was below the detection limit of the elemental analysis spectrometer.

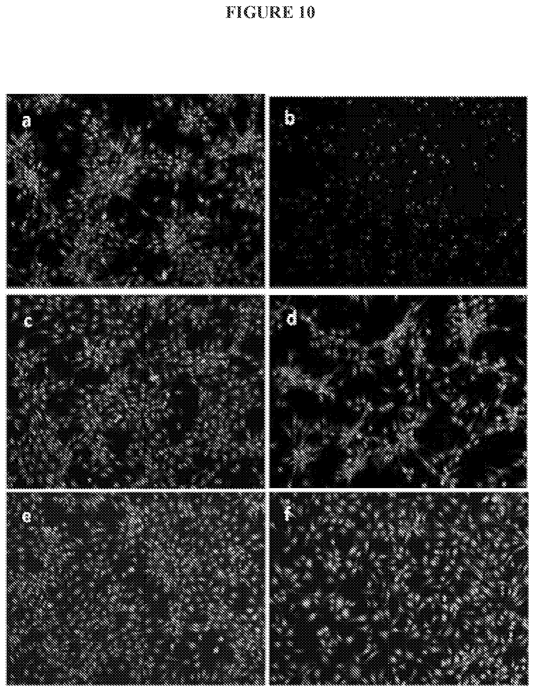

Figure 10 shows NIH 3T3 cells after 24 h incubation on glass surfaces coated with PEMs (10.5 bilayers of PAH_{7.5}/PAAₓ) without silver (a,c,e) or with silver nanoparticles at concentrations (b) 0.62, (d) 0.48, or (f) 0.39 µg/cm². PEMs were prepared with PAA solution of pH 5.5 (a, b), or 6.5 (c, d) or 7.5 (e, f).

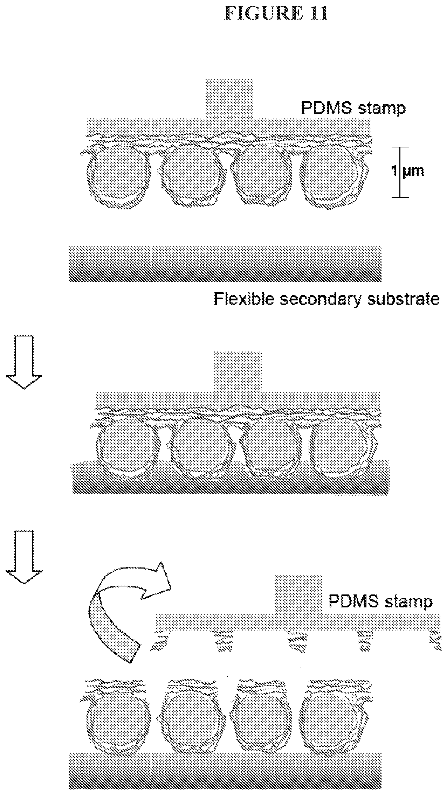

Figure 11 provides a schematic depiction of one embodiment of polymer multilayers with microscale beads.

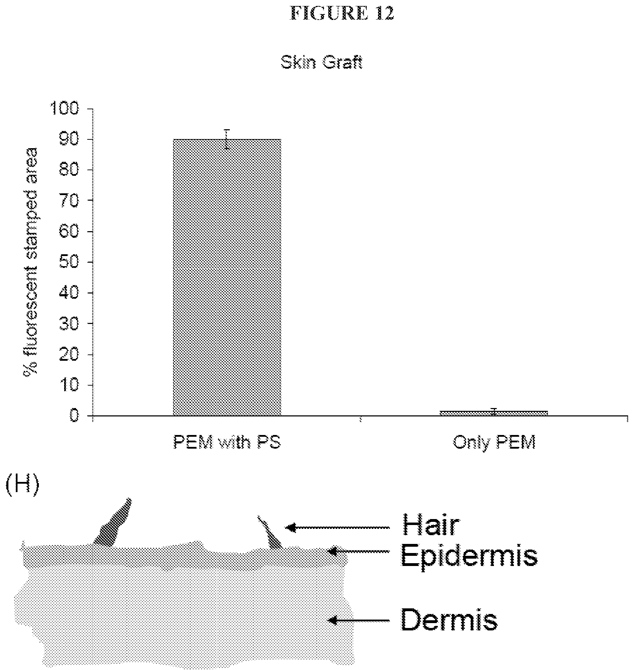

Figure 12 provides a graph depicting transfer of a polymer multilayer containing microscale beads to a soft surface as compared to transfer of a polymer multilayer without microscale beads.

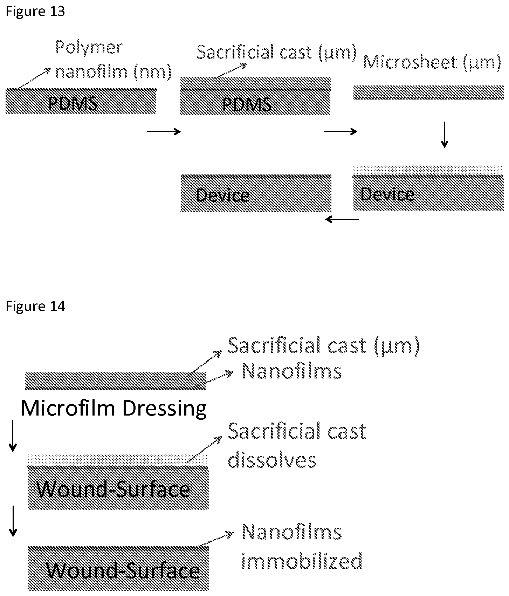

Figure 13 provides an illustration of the fabrication of microsheets comprised of a polymer nanofilm and a sacrificial cast.
Figure 14 provides an illustration of application of microsheets as a microfilm wound dressing. Sacrificial cast of the microfilm dressing dissolves quickly in moist wounds and immobilizes polymer nanofilm on wound surface.

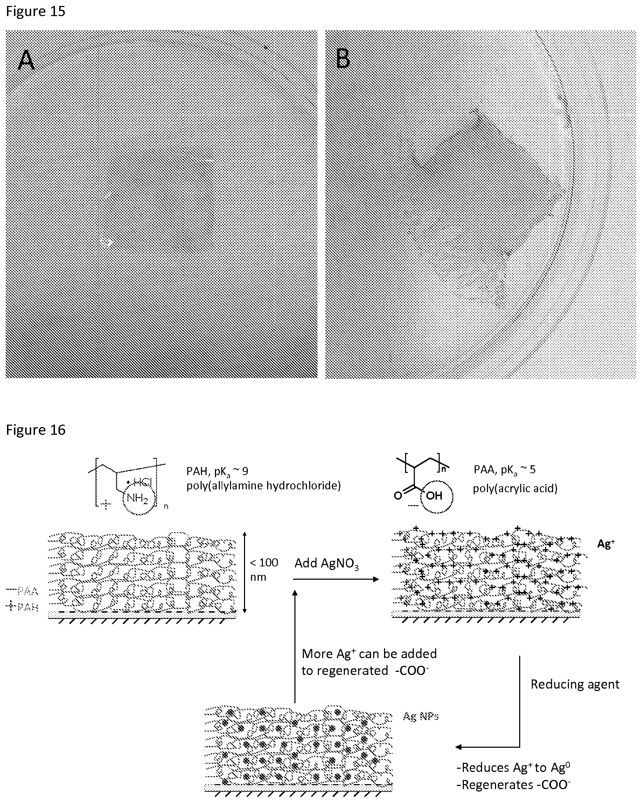

Figure 15 provides images of microsheets composed of a sacrificial water-soluble cast of PVA and a nanofilm made with PEMs containing silver-nanoparticles. (a) Microsheet (1"x1") placed on a glass dish. (b) Polymer nanofilm (1"x1") floating on a water surface after the dissolution of sacrificial cast of the microsheet in solution.
Figure 16 provides diagrams of showing assembly of polymer nanofilms deposited as PEMs of PAH and PAA on a substrate. PEMs are impregnated with silver ions (Ag+) and silver nanoparticles (AgNP).

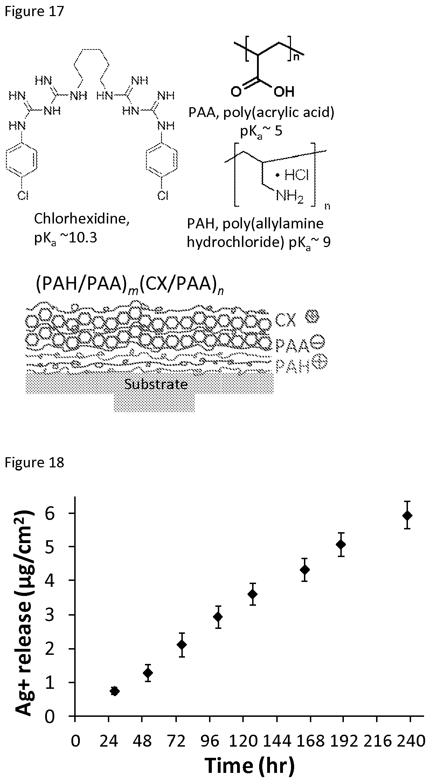

Figure 17 provides a diagram of assembly of polymer nanofilms deposited on a substrate as PEMs of PAA and PAH with chlorhexidine acetate.
Figure 18 provides a graph showing sustained release of silver-ions in PBS from polymer nanofilms assembled as PEMs of PAH and PAA containing 11±2.1 ug/cm2 of silver-nanoparticles.

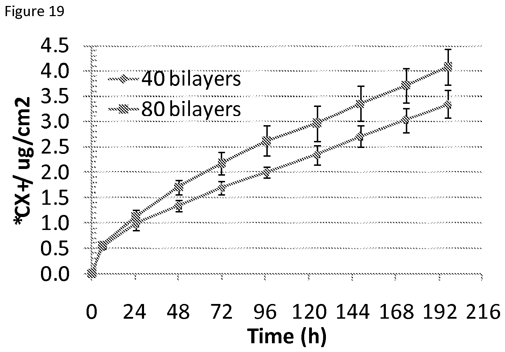

Figure 19 provides a graph showing sustained release of chlorhexidine (CX) in PBS from polymer nanofilms fabricated as PEMs containing chlorhexidine acetate molecules.

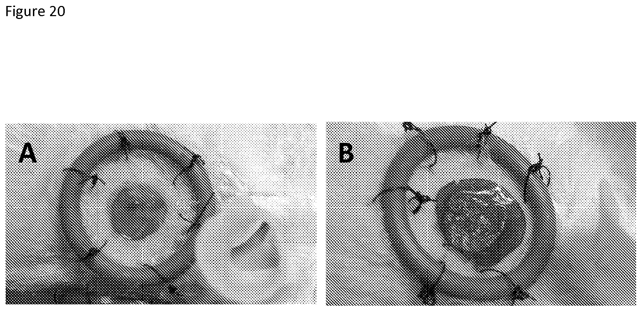

Figure 20A and B provides images showing application of a silver-microfilm wound dressing on excisional wounds in mice. (A) A 6 mm diameter full-thickness splinted wound on the flank of a mice. (b) Coverage of wound by a silver impregnated microfilm wound dressing. The sacrificial cast dissolved in moist wound to immobilize silver-nanofilm on the wound surface.

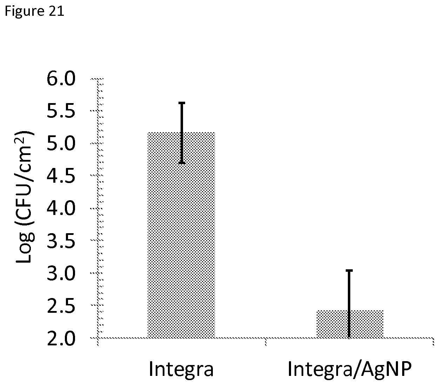

Figure 21 provides a graph showing reduction in the bacterial colonization of excisional wounds in mice on day 3 post-surgery by silver-microfilm wound dressing containing silver-nanoparticles. All wounds were inoculated with S. aureus on day 0 and covered with Integra wound dressing, or covered with silver-microfilm wound dressing along with Integra dressing.

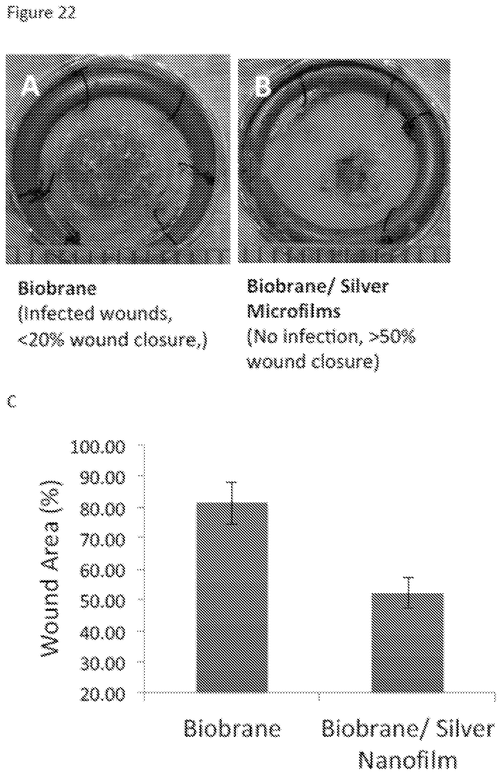

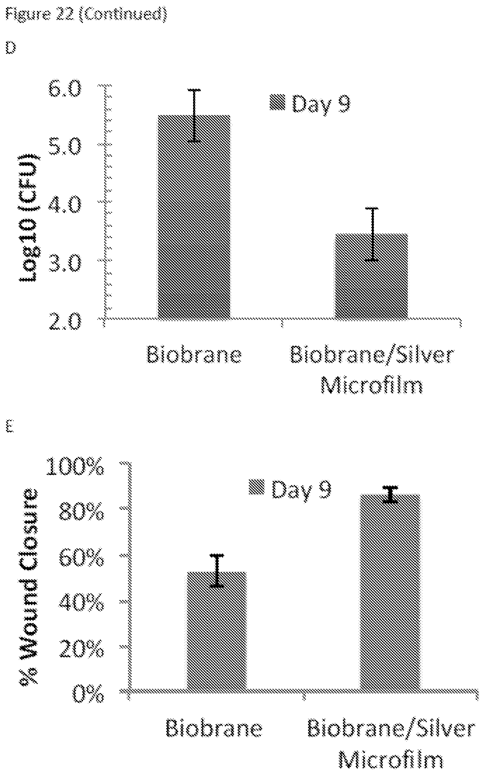

Figure 22 shows that silver-microfilm wound dressing prevents infection and expedites closure of contaminated wounds in mice. Two surgical wounds (6 mm diameter) created on the flanks of each balb/c mouse were inoculated with 2×10⁵ CFU of S. *aureus* and (A) covered with a biosynthetic dressing- Biobrane in one group or (B) covered with a silver-microfilm wound dressing before placement of Biobrane on the wounds. Figure shows representative images of wounds in group A and group B on day 9 post surgery. Figure 22C shows area (average ± SEM) of wounds (as percentage of original wound size) on day 9 post surgery in mice wounds that were treated either with only Biobrane dressing, or with Silver-microfilm wound dressing and Biobrane. (n=10 mice/group; p<0.05). Figure 22D shows microbial burden (average ± SEM) of S. *aureus* in wound biopsies harvested on day 9 post surgery from the two groups of mice. Microbial colonization of wounds was significantly less in the group treated with silver microfilm dressing (n=10 mice/group; p<0.05; Mann-Whitney U test). Figure 22E shows percentage wound closure (average ± SEM) (as percentage of original wound size) on day 9 post surgery. Wounds closure was significantly higher in the group treated with silver microfilm dressing (n=10 mice/group; p<0.05; Mann-Whitney U test).

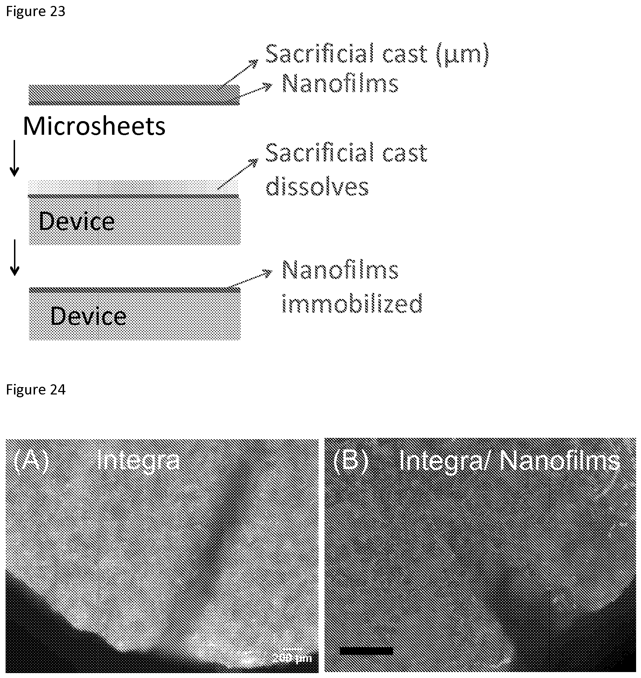

Figure 23 is a diagram of application of microsheets for surface modification of a medical device, such as surgical mesh, contact lens, or wound dressing. Sacrificial cast of the microsheet dissolves quickly on the moist surface and immobilizes polymer nanofilm on it.
Figure 24 provides images showing immobilization of polymer nanofilms by a microsheet on the surface of moist collagen matrix of Integra wound dressing. (A) Micrograph of the surface of collagen matrix of Integra wound dressing. (B) Micrograph of fluorescent polymer nanofilm immobilized on the collagen matrix of Integra wound dressing after the dissolution of the sacrificial cast of a microsheet. Scale bar= 200 um.

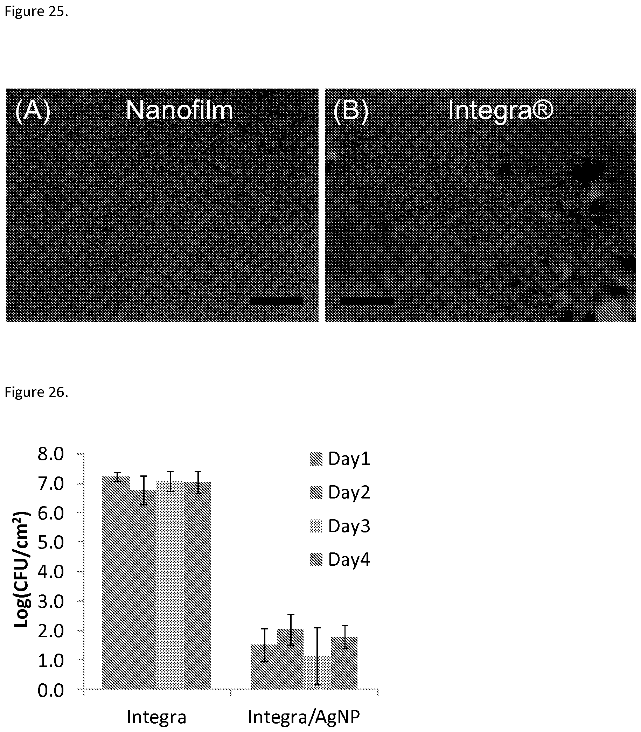

Figure 25 provides images showing immobilization of a polymer nanofilm containing microspheres by a microsheet on the moist collagen matrix of Integra wound dressing. (A) Micrograph of a microsheet with a polymer nanofilm containing 2 um size fluorescent microsphers, as assembled on an elastomeric sheet. (B) Micrograph of polymer nanofilm (with fluorescent microspheres) immobilized on the moist collagen matrix of Integra dressing after dissolution of the sacrificial cast of the microsheet. Scale bar= 20 um.
Figure 26 provides a graph showing reduction in bacterial colonization in solutions incubated on the surface of Integra wound dressing modified by silver-nanofilms (AgNP) using microsheets with water-soluble sacrificial casts of PVA. Solutions over test dressings were repeatedly inoculated with10⁷ CFU/ml of S. *aureus* every 24 h.

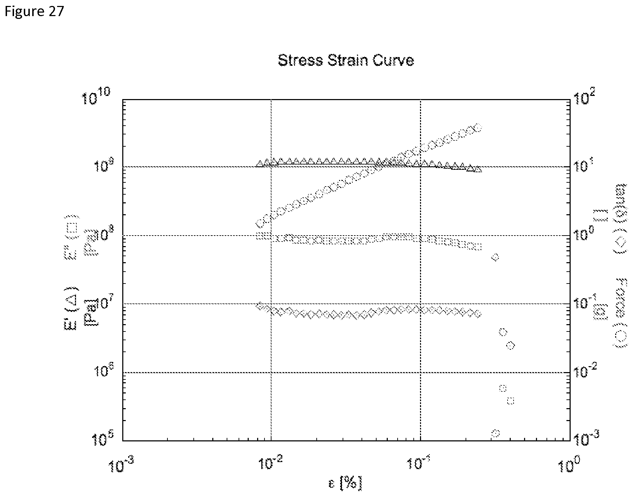

Figure 27 provides a stress strain curve showing mechanical strength of a silver-micro sheet.

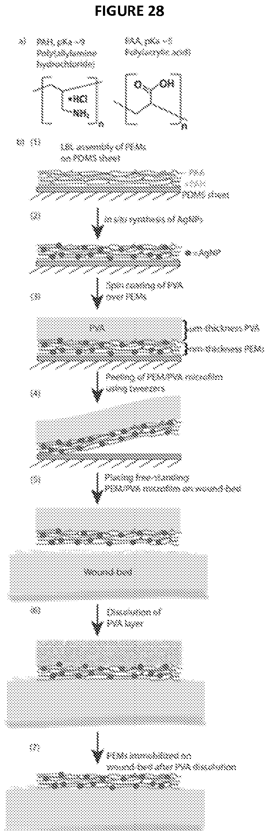

Figure 28 shows a schematic diagram according to an embodiment of the technology described herein. Figure 28a shows polyelectrolytes employed for layer-by-layer assembly of nanometer-thick PEMs. Figure 28b is a schematic illustration of the procedure used to fabricate PEM/PVA microfilms and apply them to wound-beds: (1) PEMs are assembled on PDMS sheets, (2) post-fabrication, PEMs are impregnated with silver ions that are subsequently reduced to silver-nanoparticles, (3) PVA solution is spin-coated over silver-loaded PEMs, (4) PEM/PVA microfilm is peeled from the PDMS sheet and (5) placed onto a moist wound-bed. 6) Dissolution of the PVA layer in the moist wound leads to (7) immobilization of the PEMs on the wound-bed.

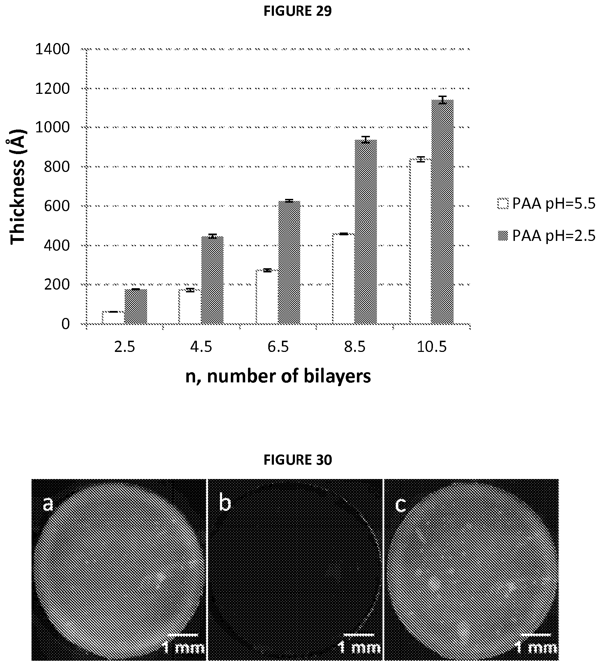

Figure 29 is a plot that shows the ellipsometric thickness of PEMs of (FITC-PAH/PAA) with increasing number of bilayers, n, assembled on silicon wafers. Data are presented as mean ± SEM with n = 12. As shown in the figure, PEMs assembled using PAA solution at pH = 2.5 are thicker than those fabricated using PAA solution at pH = 5.5. At a lower pH of PAA, there are less ionized carboxyl groups available for electrostatic binding with the charged polycations. This weaker electrostatic interaction between polymer segments, therefore, requires more polyanion deposition to compensate charge on polycations, and results in thicker multilayers. Polymer multilayers with PAA pH 2.5 show a linear growth trend (average thickness of ~1200 Å for 10.5 bilayers), while the one with PAA pH 5.5 shows an exponential growth trend (average thickness of ~800 Å for 10.5 bilayers).
Figure 30 is a series of fluorescence microscopy images showing that microfilms of PEMs/PVA are uniformly peeled from PDMS sheets on which they are fabricated and retain their silver loadings. Figure 30a, Figure 30b, and Figure 30b show representative fluorescent micrographs of a 6 mm diameter PEM/PVA microfilm with PEMs of (FITC-PAH/PAA5.5)_{10.5}. Figure 30a shows PEM on a PDMS sheet before peeling; Figure 30b shows PDMS after peeling; and Figure 30c shows PEM placed on a glass slide for imaging.

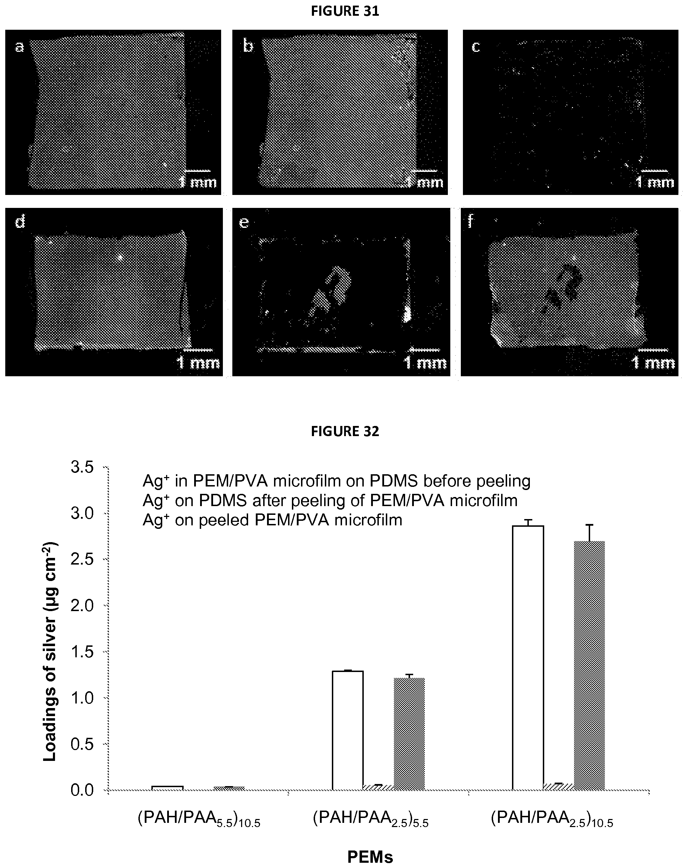

Figure 31 is a series of fluorescence micrographs showing that PEMs of AF-PAH/PAA_{5.5})_{10.5} (assembled using Alexa Fluor 560 labeled PAH) cannot be peeled as PEM/PVA microfilm from non-OTS functionalized silicon wafer (Figure 31a, Figure 31b, and Figure 31c), but can be peeled uniformly from OTS functionalized silicon wafers (Figure 31d, Figure 31e, and Figure 31f). Figure 31a and Figure 31d show AF-PEMs fabricated on Si wafer substrates not functionalized and functionalized with OTS, respectively; Figure 31b and Figure 31e show PEM left on Si wafer not functionalized and functionalized with OTS, respectively, after peeling off as a PEM/PVA microfilm; Figure 31c and Figure 31f shows a PEM/PVA microfilm peeled off the Si wafer substrates not functionalized and functionalized with OTS, respectively, and placed on a glass slide for imaging.
Figure 32 is a plot showing silver loading of PEMs/PVA microfilms before and after peeling the PEMs from the PDMS sheets, illustrated using PEMs with different silver loadings. Data are presented as mean ± SEM with n ≥ 4.

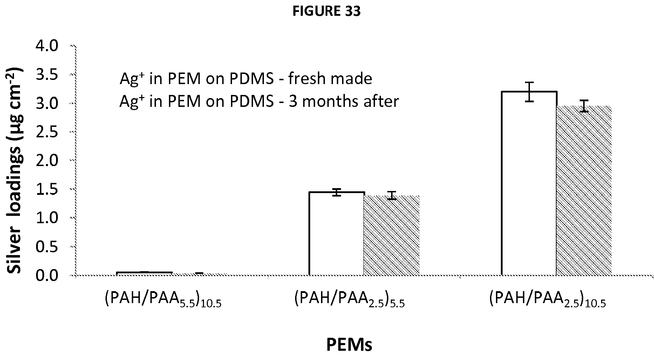

Figure 33 isa plot showing silver loading in PEMs stored for 3 months at ambient temperature is not significantly different from the freshly assembled PEMs. Data presented as mean ± SEM (n ≥ 3).

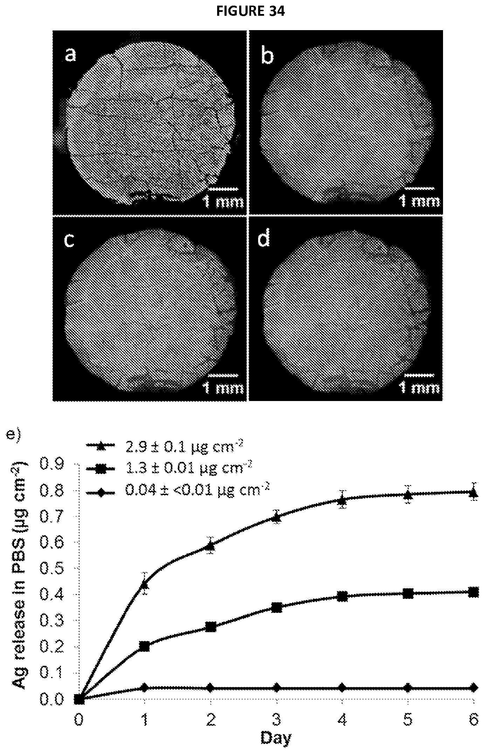

Figure 34 is a series of fluorescence microscopy images and a plot showing hcaracterization of PEMs immobilized from PEM/PVA microfilms onto human cadaver skin dermis (GammaGraft). Figure 34a shows a microfilm with PEMs of (FITC-PAH/PAA_{2.5})_{40.5} on a PDMS sheet; Figure34b shows a PEM/PVA microfilm placed onto skin dermis with the PEMs facing the surface of the tissue; Figure 34c shows PEMs immobilized on skin dermis after repeated rinsing with 1 mL PBS; and Figure 34d shows PEMs retained on human skin dermis after 3 days of continuous incubation in excess PBS on shaker plates. The plot in Figure 34e shows the sustained release of silver ions in PBS from the skin dermis modified with PEMs impregnated with a range of silver loadings. Data are presented as mean ± SEM with n ≥ 4.

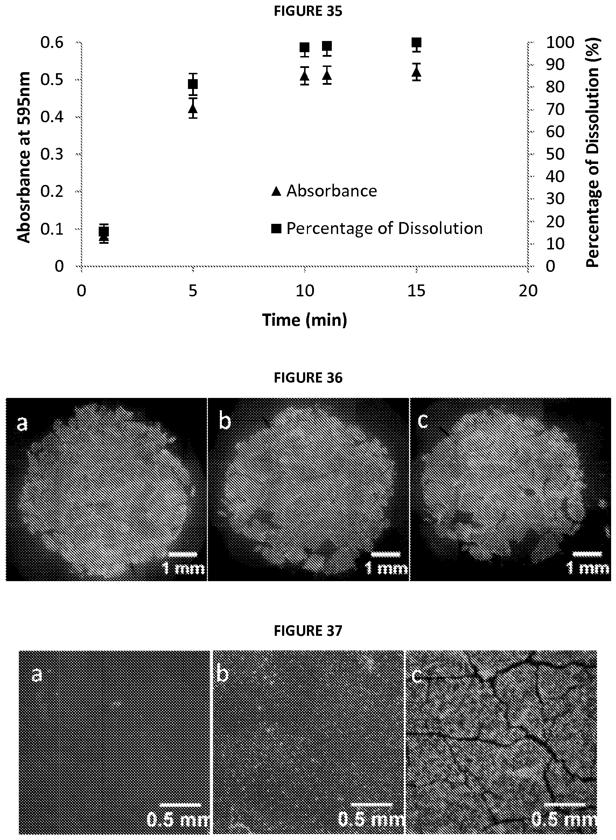

Figure 35 is a plot showing the PVA cast in the PEM/PVA microfilm dissolves completely in aqueous solutions within 10 minutes. The plot presents absorbance intensity of a Malachite Green-dye incorporated in PVA cast and released in 3 mL PBS buffer along with the dissolution of the PVA cast at different time points. Absorbance intensity of Malachite Green dye in PBS was measured on a UV-vis spectrophotometer at 595 nm wavelength. Data presented as mean ± SEM (n ≥ 5).
Figure 36 is a series of fluorescence micrographs showing the persistence of FITC-PEMs, e.g., (FITC-PAH/PAA_{2.5})_{40.5}, immobilized on skin dermis against mechanical pressure and abrasion. Figure 36a shows FITC-PEMs immobilized on skin dermis; Figure 36 shows intact FITC-PEMs on skin dermis after vertically applied mechanical pressure of ~8 kPa; Figure 36c shows FITC-PEMs retained on skin dermis after lateral abrasion with Telfa dressings (1 mm/s) following mechanical pressure treatment described in Figure 36b.
Figure 37 is a series of fluorescence micrographs showing formation of cracks in the PEMs of (PAH/PAA_{2.5}) with 40.5 bilayers. Figure 37a is a fluorescent micrograph of a PEM of (FITC-PAH/PAA_{2.5}) having 5.5 bilayers fabricated on an elastomeric PDMS sheet. Figure 37b is a fluorescent micrograph of a PEM of (FITC-PAH/PAA_{2.5}) having 10.5 bilayers fabricated on an elastomeric PDMS sheet. Figure 37c is a fluorescent micrograph of a PEM of (FITC-PAH/PAA_{2.5}) having 40.5 bilayers fabricated on an elastomeric PDMS sheet.

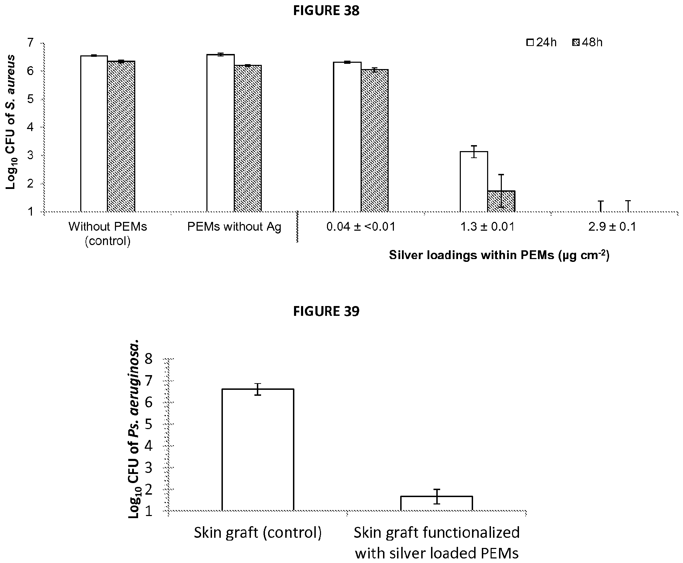

Figure 38 is a plot showing antibacterial activity in suspensions of S. *aureus* incubated for 24 h or 48 h over skin-dermis modified with PEM/PVA microfilms with a range of silver loadings. Modified skin-dermis was incubated in 96-well plates with 10⁷ CFU of S. *aureus* in 100 mL HBSS buffer on shaker plates (150 rpm) at 37°C. Data are presented as mean ± SEM with n ≥ 4.
Figure 39 is a plot showing antibacterial activity in suspensions *of Ps. aeruginosa* incubated for 24 hours over skin-dermis modified with silver/PEMs (with silver loadings of 2.9 ± 0.1 µg cm⁻²) using PEM/PVA microfilm. Modified skin dermis was placed in 96-well plates and incubated with 10⁷ CFU *of Ps. aeruginosa* in 100 mL PBS buffer for 24 hours with shaking (150 rpm) at 37°C. After incubation, bacteria were rinsed off and collected from the test wells and their serial dilutions were plated on blood agar plates. Data presented as mean ± SEM with n ≥ 4.

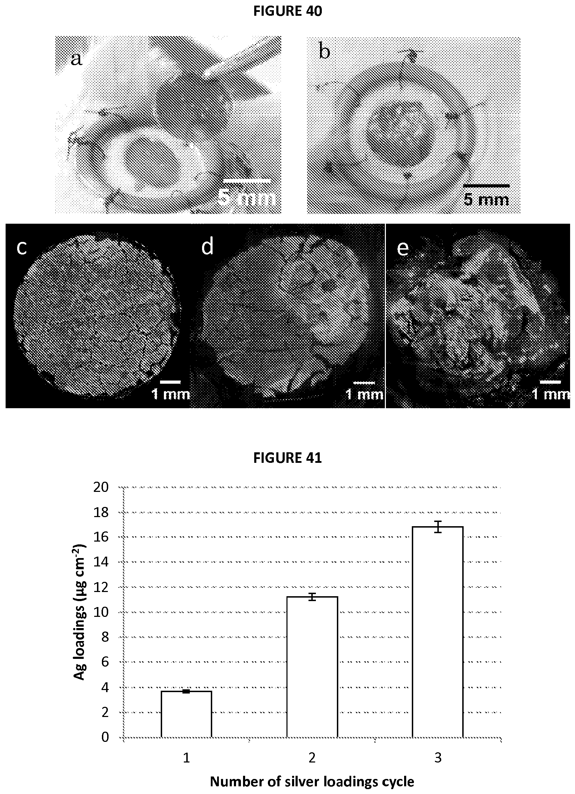

Figure 40 is a series of images showing modification of full-thickness splinted wound in mice with a silver/PEM using a microfilm. The photograph of Figure 40a shows splinted wounds in mice with PEM/PVA microfilm held by tweezers; Figure 40b shows PEMs adhered on mice wounds. Figure 40c, Figure 40d, and Figure 40e shows the persistence of fluorescence-tagged PEMs immobilized on the surface of excisional full-thickness. Figure 40c is a fluorescent micrographshowing a PEM/PVA microfilm comprising a PEM of (FITC-PAH/PAA_{2.5})_{40.5} on PDMS sheet before transfer; Figure 40d is a fluorescent micrographshowing the PEM/PVA microfilm comprising a PEM of (FITC-PAH/PAA_{2.5})_{40.5} on wound-bed harvested from mice after 1 h; and Figure 40e is a fluorescent micrographshowing the PEM/PVA microfilm comprising a PEM of (FITC-PAH/PAA_{2.5})_{40.5} on wound-bed harvested from mice after 3 days post-surgery.
Figure 41 is a plot showing silver loading in PEMs of (PAH/PAA_{2.5})_{10.5} tailored up to 16.8 ± 0.5 µg cm⁻² by repeating three cycles of silver ion exchange and their in situ reduction into silver nanoparticles. Data are presented as mean ± SEM (n ≥ 4).

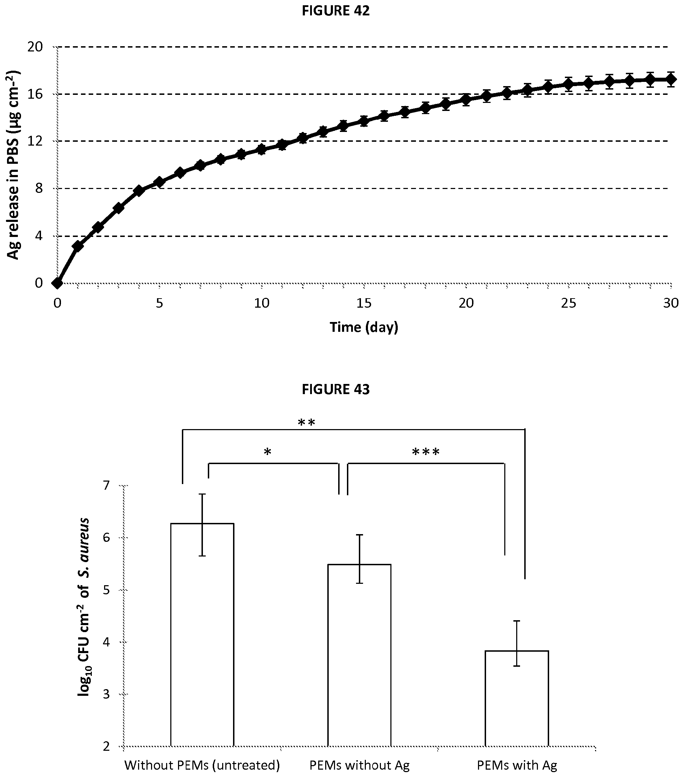

Figure 42 is a plot showing sustained release of silver ions into PBS from PEMs of (PAH/PAA_{2.5})_{10.5} with silver loadings of 16.8 ± 0.5 µg cm⁻². Silver loading in the PEMs was achieved by repeating three cycles of silver ion exchange and reduction. This construct of PEMs was used to treat contaminated wounds in mice. Data are presented as mean ± SEM with n = 3.
Figure 43 is a plot showing inhibition of microbial colonization in contaminated murine wounds treated with PEM/PVA microfilms (with silver loading of 16.8 ± 0.5 µg cm⁻²). Excisional 6 mm diameter splinted wounds topically inoculated with 3.0 × 10⁶ CFU/cm² of *S. aureus* were treated with the PEM/PVA microfilm and covered with a biosynthetic wound dressing Biobrane®. The plot shows bacterial counts recovered from wounds harvested and homogenized (along with Biobrane®) after 3 days post-surgery (^{∗}, P> 0.05, ^{∗∗}, P< 0.001, ^{∗∗∗}, P< 0.03). Data represent the mean ± SEM with n = 8 mice (=16 wounds) for each group.

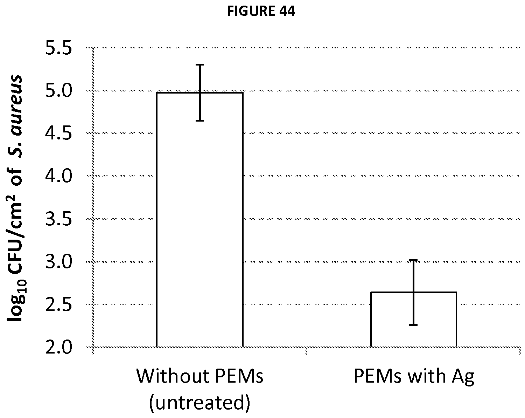

Figure 44 is a plot showing inhibition of microbial colonization in contaminated murine wounds treated with PEM/PVA microfilms (with silver loading of 17.63 ± 3.13 µg cm⁻²). Excisional 6 mm diameter splinted wounds topically inoculated with 2.71 × 10⁶ CFU/cm² of *S. aureus* were treated with the PEM/PVA microfilm and covered with a biosynthetic wound dressing Biobrane®. The plot shows bacterial counts recovered from wounds harvested and homogenized (along with Biobrane®) after 3 days post-surgery. Data represent the mean ± SEM with n=10 mice (=20 wounds) for each group (P < 0.001).

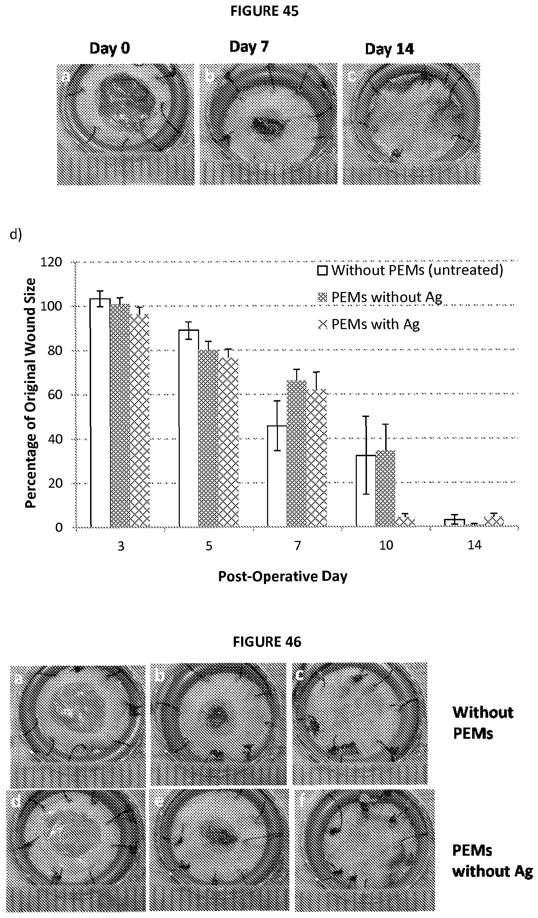

Figure 45 is a series of images and a plot showing PEM/PVA microfilms promote normal wound healing in excisional splinted wounds in mice. Figure 45a, Figure 45b, and Figure 45c show gross images of wounds on post-operative days 0, 7 and 14, respectively, that were modified post-surgery with silver/PEMs (with silver loading of 16.8 ± 0.5 µg cm⁻²). Each line on the scale represents 1 mm. Figure 45d shows the percentage of original wound size on post-operative days 3, 5, 7, 10, and 14 in wounds that did not receive PEMs and wounds that were modified with either PEMs containing no silver or PEMs containing 16.8 ± 0.5 µg cm⁻² silver. Each data point presents mean ± SEM of relative wound size. The sample sizes (n) for days 3, 5, and 7 were n = 8, 16 and 16, respectively, for each group. On day 7, some wounds were harvested for histopathology. The sample sizes for the three groups on days 10 and 14 were n = 4, 6 and 6, respectively.
Figure 46 is a series of images showing PEM/PVA microfilms promote normal wound healing in excisional splinted wounds in mice. The micrographs of Figure 46a, Figure 46b, and Figure 46c, respectively, show gross images of wounds without PEMs and the micrographs of Figure 46d, Figure 46e, and Figure 46f show gross images of wounds modified with PEMs containing no silver. Figure 46a and Figure 46d are images from post-operative day 0; Figure 46b and Figure 46e are images from post-operative 7; and Figure 46c and Figure 46f are images from post-operative 14. Each line on the scale represents 1 mm.

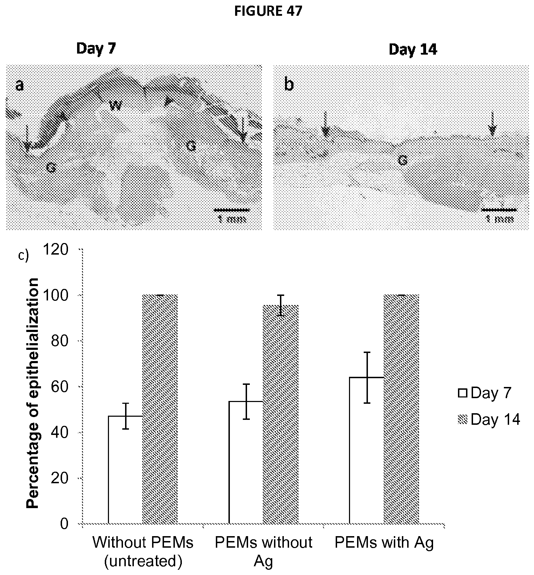

Figure 47 is a series of images and a plot showing PEM/PVA microfilm (with 16.8 ± 0.5 µg cm⁻² of silver) promotes epithelialization similar to the untreated wound in excisional splinted wounds in mice, as determined by histopathology. Figure 47a and Figure 47b show representative H&E-stained sections of wounds post-operative days 7 and 14, respectively, in wounds modified with silver/PEMs. Original wound edge (arrow), migrating epithelial tongue (arrow head), granulation tissue (G), and wound matrix (W) are marked. Figure 47c shows the percentage of original wound size on post-operative days 7 and 14 in wounds that did not receive PEMs, and wounds that were modified with PEMs containing no silver or PEMs containing silver. Each data point presents mean ± SEM of relative wound size. The sample sizes (n) for day 7 for the groups without PEMs, with PEM/PVA without Ag, and with PEM/PVA with Ag, were n = 4, 9, and 7, respectively. For day 14, n = 3 for each group.

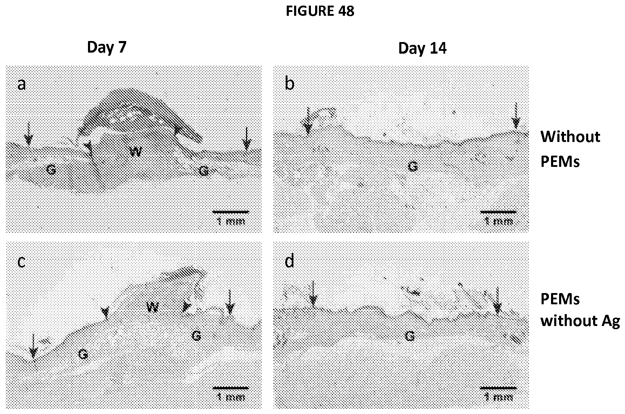

Figure 48 shows PEM/PVA microfilm promotes epithelialization similar to the untreated wound in excisional splinted wounds in mice, as determined by histopathology. Figure 48a and Figure 48b are micrographs showing H&E-stained sections from wounds without PEMs post-operative at days 7 and 14, respectively. Figure 48c and Figure 48d are micrographs showing H&E-stained sections from wounds immobilized with PEMs containing no silver post-operative at days 7 and 14, respectively. Original wound edge (arrow), migrating epithelial tongue (arrow head), granulation tissue (G), and wound matrix (W) are marked.

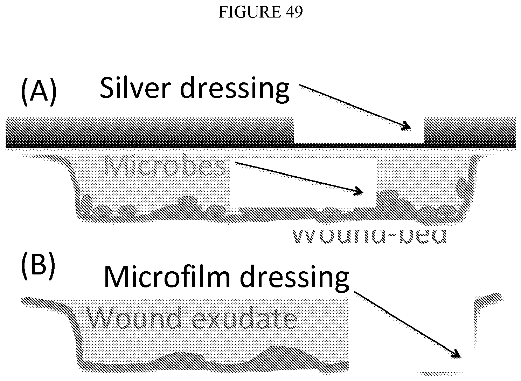

Figure 49 is a drawing comparing the coverage of a wound by conventional dressings and embodiments of the microfilm silver dressings provided herein. Figure 49A depicts a wound covered with a conventional dressing and Figure 49B depicts a wound covered with a microfilm dressing as discussed herein.

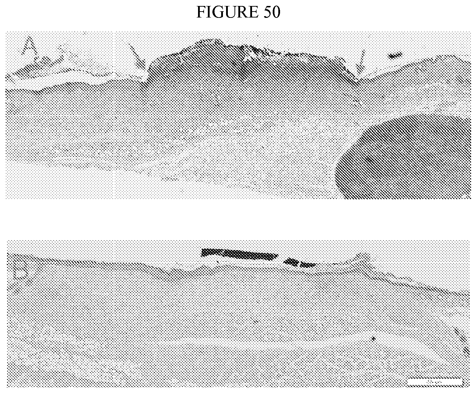

Figure 50 shows micrographs of haematoxylin and eosin (H&E) stained wounds on day 12 post-surgery that were created on the flanks of balb/c mice and inoculated with 2 × 10⁵ CFU *S. aureus.* Figure 50A shows a wound that was covered with a biosynthetic Biobrane dressing. Figure 50B shows a wound covered with a silver-microfilm wound dressing before placement of Biobrane on the wounds. In Figure 50, the scale = 0.5 mm.

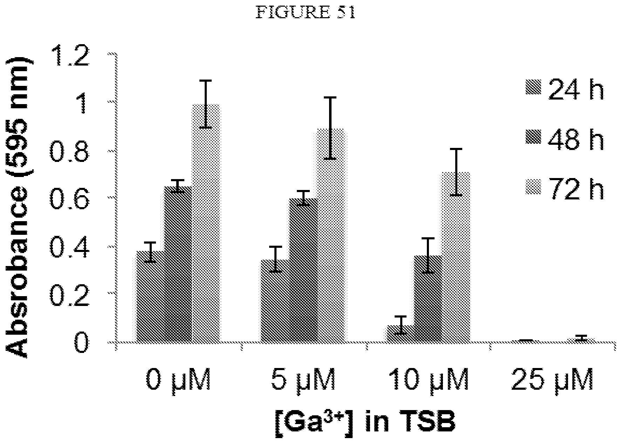

Figure 51 is a plot showing inhibition of *P. aeruginosa* biofilms at the bottom of 48-well plates in TSB media with Ga(NO₃)₃. The data are presented as the absorbance of crystal violet stained biofilms (n = 4, mean ± stdev).

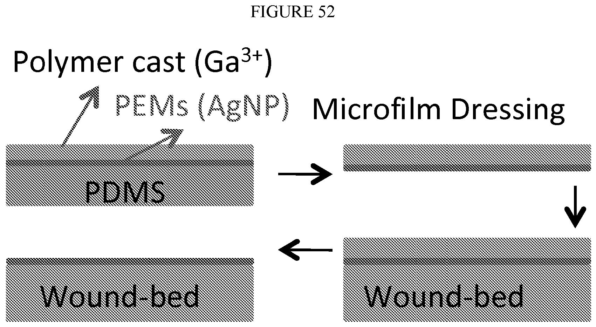

Figure 52 is a drawing showing a microfilm dressing comprising a water soluble polymeric cast (with antibiofilm Ga) that adheres to a polymer multilayer nanofilm (PEMs) containing silver nanoparticles on the wound-bed.

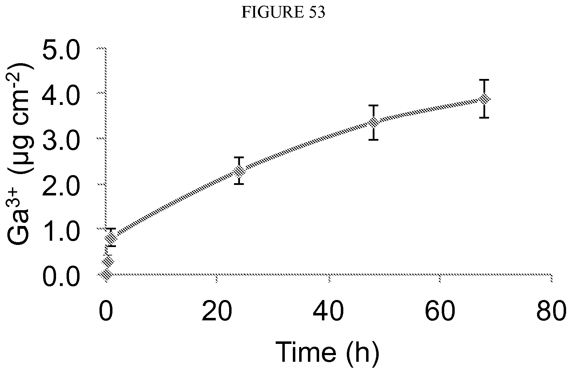

Figure 53 is a plot showing the sustained release of Ga³⁺ in PBS from PEMs of (PAA/PAH)₁₀ containing 16.1 ± 0.8 µg/cm² of gallium.

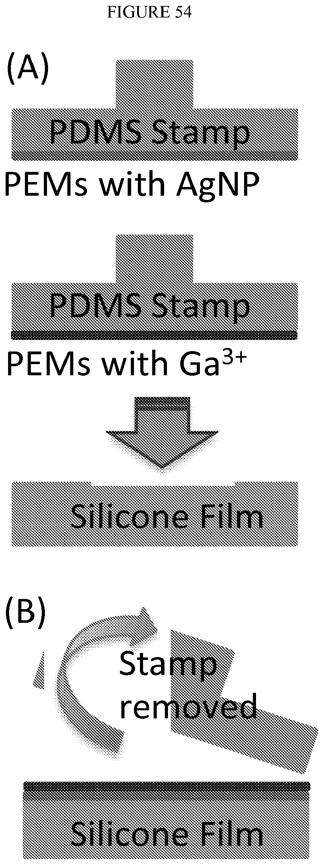

Figure 54 is a drawing showing the sequential transfer of nanocoatings of PEMs containing gallium (Ga) or silver nanoparticles (AgNP) by stamping onto a silicone film dressing. Figure 54A shows the sequential depostion of nanocoatings onto silicone film and Figure 54B shows a removal of a stamp to leave the nanocoating on the silicone film.

### DEFINITIONS

To facilitate an understanding of the invention set forth in the disclosure that follows, a number of terms are defined below.

The term "wound" refers broadly to injuries to the skin and subcutaneous tissue initiated in different ways (e.g., pressure sores from extended bed rest and wounds induced by trauma) and with varying characteristics. The methods and compositions described herein are useful for treatment of all types of wounds, including wounds to internal and external tissues. Wounds may be classified into one of four grades depending on the depth of the wound: i) Grade I: wounds limited to the epithelium; ii) Grade II: wounds extending into the dermis; iii) Grade III: wounds extending into the subcutaneous tissue; and iv) Grade IV (or full-thickness wounds): wounds wherein bones are exposed (e.g., a bony pressure point such as the greater trochanter or the sacrum).

The term "partial thickness wound" refers to wounds that encompass Grades I-III; examples of partial thickness wounds include burn wounds, pressure sores, venous stasis ulcers, and diabetic ulcers. The term "deep wound" is meant to include both Grade III and Grade IV wounds. The present invention contemplates treating all wound types, including deep wounds and chronic wounds.

The term "chronic wound" refers to a wound that has not healed within 30 days.

The phrases "promote wound healing," "enhance wound healing," and the like refer to either the induction of the formation of granulation tissue of wound contraction and/or the induction of epithelialization (i.e., the generation of new cells in the epithelium). Wound healing is conveniently measured by decreasing wound area.

The term "wound active agent" refers to known or potential chemical compounds that induce a desired pharmacological, physiological effect useful in the treatment and healing of a wound, wherein the effect may be prophylactic or therapeutic. The terms also encompass pharmaceutically acceptable, pharmacologically active derivatives of those active agents specifically mentioned herein, including, but not limited to, trophic factors, extracellular matrices, enzymes, enzyme inhibitors, defensins, polypeptides, anti-infective agents (including but not limited to ionic silver, elemental silver, and silver nanoparticles), buffering agents, vitamins and minerals, analgesics, anticoagulants, coagulation factors, anti-inflammatory agents, vasoconstrictors, vasodilators, diuretics, and anti-cancer agents.

The term "polymer multilayer" refers to the composition formed by sequential and repeated application of polymer(s) to form a multilayered structure. For example, polyelectrolyte multilayers are polymer multilayers are formed by the alternating addition of anionic and cationic polyelectrolytes to a wound or support. The term "polymer multilayer" also refers to the composition formed by sequential and repeated application of polymer(s) to a wound or to a solid support. In addition, the term "polymer layer" can refer to a single layer composed of polymer molecules, such as anionic or cationic polyelectrolyte molecules, existing either as one layer within multiple layers, or as a single layer of only one type of polyelectrolyte molecules on a wound or support. While the delivery of the polymers to the wound bed or support is sequential in preferred embodiments, the use of the term "polymer multilayer" is not limiting in terms of the resulting structure of the coating. It is well understood by those skilled in the art that inter-diffusion of polymers such as polyelectrolytes can take place leading to structures that may be well-mixed in terms of the distribution of anionic and cationic polyelectrolytes. It is also understood that the term polyelectrolyte includes polymer species as well as nanoparticulate species, and that it is not limiting in scope other than to indicate that the species possesses multiple charged or partially charged groups. It is also well understood by those skilled in the art that multilayer structures can be formed through a variety of interactions, including electrostatic interactions and others such as hydrogen bonding. Thus, the use of the term "polyelectrolyte" is not limiting in terms of the interactions leading to the formation of the wound bed constructs.

The term "crosslinked" herein refers to a composition containing intermolecular crosslinks and optionally intramolecular crosslinks as well, arising from the formation of covalent bonds. Covalent bonding between two crosslinkable components may be direct, in which case an atom in one component is directly bound to an atom in the other component, or it may be indirect, through a linking group. A crosslinked structure may, in addition to covalent bonds, also include intermolecular and/or intramolecular noncovalent bonds such as hydrogen bonds and electrostatic (ionic) bonds.

The term "covalent modification agent" refers to any molecule that covalently links molecules to each other. Covalent modification agents include homobifunctional and heterobifunctional cross-linkers as well as photoactivatable cross linkers.

The term "homobifunctional cross-linker" refers to a molecule used to covalently link identical or similar molecules to each other. Homobifunctional cross-linkers have two identical reactive groups; thus, a homobifunctional cross-linker can only link molecules of the same type to each other. Conversely, a "heterobifunctional cross-linker" refers to a molecule used to covalently link dissimilar molecules to each other, because it has two or more different reactive groups that can interact with various molecules of different types. Hetero- and homo-multifunctional crosslinkers refers to multivalent crosslinkers with both hetero- and homo- crosslinking functionalities. Activated dendrimers are an example of multifunctional crosslinkers.

The term "subject" refers to any animal (e.g., a mammal), including, but not limited to, humans, non-human primates, rodents, dogs, cats, and the like, which is to be the recipient of a particular treatment. Typically, the terms "subject" and "patient" are used interchangeably herein.

The term "surfactant" refers to an amphiphilic material that modifies the surface and interface properties of liquids or solids. Surfactants can reduce the surface tension between two liquids. Detergents, wetting agents, emulsifying agents, dispersion agents, and foam inhibitors are all surfactants.

The term "block copolymer" refers to a polymer consisting of at least two monomers. In a block copolymer, adjacent blocks are constitutionally different, i.e. adjacent blocks comprise constitutional units derived from different species of monomer or from the same species of monomer but with a different composition or sequence distribution of constitutional units. A block copolymer can be thought of as two homopolymers joined together at the ends.

The term "solvent" refers to a liquid that can dissolve a substance. The term "organic solvent" refers to a solvent derived from a petroleum-based product.

The term "polyelectrolyte" refers to a water-soluble macromolecular polymer substance containing many repeating ionic constituent units, including cations and anions.

The term "primary amine" refers to a derivative of ammonia in which a hydrogen has been replaced by a hydrocarbon unit. Primary amines have the general formula RNH₂ and examples include, but are not limited to, aniline, methylamine, and 1-propylamine.

The term "DNA delivery agent" refers to any molecule that can bring DNA into contact with an identified target. In some instances, a DNA delivery agent causes uptake of DNA into a cell or cells, *in vitro* or *in vivo.* DNA delivery agents can be viruses including, but not limited to, adenoviruses and retroviruses. DNA delivery agents can also be non-viral agents including, but not limited to, plasmids, lipids, liposomes, polymers and peptides.

The term "exposable" refers to anything that is capable of being exposed. An exposable surface or molecule is one that is made available to interaction with other surfaces or molecules. For example, in the context of the present invention, a covalent modification agent is exposable to a wound active agent; thus, the two agents can interact with each other and form covalent bonds.

The term "functionalized" refers to a modification of an existing molecular segment to generate or introduce a new reactive functional group (e.g., a maleimido or succinimidyl group) that is capable of undergoing reaction with another functional group (e.g., a sulfhydryl group) to form a covalent bond. For example, a component containing carboxylic acid (-COOH) groups can be functionalized by reaction with N-hydroxy-succinimide or N-hydroxysulfosuccinimide using known procedures, to form a new reactive functional group in the form of an activated carboxylate (which is a reactive electrophilic group), i.e., an N-hydroxysuccinimide ester or an N-hydroxysulfosuccinimide ester, respectively. In another example, carboxylic acid groups can be functionalized by reaction with an acyl halide, e.g., an acyl chloride, again using known procedures, to provide a new reactive functional group in the form of an anhydride.

As used herein, the term "aqueous solution" includes solutions, suspensions, dispersions, colloids, and the like containing water.

As used herein, the term "click chemistry" refers to the use of chemical building blocks with built-in high-energy content to drive a spontaneous and irreversible linkage reaction with appropriate complementary sites in other blocks. These chemical reactions (e.g., including, but not limited to, those between azide and acetylene groups that combine readily with each other) are specific and result in covalent linkage between the two molecules.

The term "native chemical ligation" refers to a chemoselective reaction of two unprotected peptide segments. The reaction results in an initial thioester-linked species, then spontaneous rearrangement of this transient intermediate occurs, yielding a full-length product with a native peptide bond at the ligation site.

The term "specific protein binding" refers to an interaction between two or more proteins that have high affinity and specificity for each other. Proteins must bind to specific other proteins *in vivo* in order to function. The proteins are required to bind to only one or a few other proteins of the few thousand proteins typically present *in vivo;* these interactions are employed *in vitro* in the present invention to attach wound active agents to the wound. In the context of the present invention, specific protein binding interactions include, but are not limited to, those between biotin and avidin, neutravidin, or streptavidin; glutathione-S-transferase and glutathione; and nickel-nitrilotriacetic acid and polyhistidine.

The term "device" refers to an object that contacts the body or bodily fluid of a subject for therapeutic or prophylactic purposes. Some devices may partially or indirectly contact the body or bodily fluid of a subject (e.g., catheter, dialysis tubing, diagnostic sensors, drug delivery devices), while other devices are completely imbedded in or encompassed by the body of a subject (e.g., stent, pacemaker, internally implanted defibrillator, angioplasty balloon, orthopedic device, spinal cage, implantable drug pump, artificial disc, ear disc).

The term "selective toxicity" refers to the property of differentially toxic effects on mammalian versus microbial cells. For example, a selectively toxic agent may effectively kill bacterial cells while permitting growth and viability of mammalian cells.

The term "toxic" refers to any detrimental or harmful effects on a subject, a cell, or a tissue as compared to the same cell or tissue prior to the administration of the toxicant.

As used herein, the terms "nanoparticle" and "nanoscale particles" are used interchangeably and refer to a nanoscale particle with a size that is measured in nanometers, for example, a nanoscopic particle that has at least one dimension of less than about 1000, 500, or 100 nm. Examples of nanoparticles include nanobeads, nanofibers, nanohoms, nano-onions, nanorods, and nanoropes.

As used herein, the term "microparticle" and "microscale particles" are used interchangeably and refers to a microscale particle with a size that is measured in micrometers, for example, a microscale particle that has at least one dimension of less than about 10 micrometers, 5 micrometers, or 2 micrometers.

The term "wound dressing" refers to materials placed proximal to a wound that have absorbent, adhesive, protective, osmoregulatory, pH-regulatory, or pressure-inducing properties. Wound dressings may be in direct or indirect contact with a wound. Wound dressings are not limited by size or shape. Indeed, many wound dressing materials may be cut or configured to conform to the dimensions of a wound. Examples of wound dressing materials include but are not limited to gauze, adhesive tape, bandages, and commercially available wound dressings including but not limited to adhesive bandages and pads from the Band-Aid^{®} line of wound dressings, adhesive bandages and pads from the Nexcare^{®} line of wound dressings, adhesive bandages and non-adhesive pads from the Kendall Curity Tefla^{®} line of wound dressings, adhesive bandages and pads from the Tegaderm^{®} line of wound dressings, adhesive bandages and pads from the Steri-Strip^{®} line of wound dressings, the COMFEEL^{®} line of wound dressings, adhesive bandages and pads, the Duoderm^{®} line of wound dressings, adhesive bandages and pads, the TEGADERM™ line of wound dressings, adhesive bandages and pads, the OPSITE^{®} line of wound dressings, adhesive bandages and pads, and biologic wound dressings. A "biologic wound dressing" is a type of wound dressing that comprises, e.g., is coated with or incorporates, cells and/or one or more biomolecules or fragments of biomolecules that can be placed in contact with the wound surface. The biomolecules may be provided in the form of an artificial tissue matrix. Examples of such biomolecules include, but are not limited, to collagen, hyaluronic acid, glycosaminoglycans, laminin, vitronectin, fibronectin, keratin, antimicrobial polypeptides and combinations thereof. Examples of suitable biologic wound dressings include, but are not limited to, BIOBRANE™, Integra™, Apligraf^{®}, Dermagraft^{®}, Oasis^{®}, Transcyte^{®}, Cryoskin^{®} and My skin^{®}.

As used herein, the term "antimicrobial silver composition" refers to a composition that comprises silver as an active antimicrobial agent. Examples of "antimicrobial silver compositions" include, but are not limited to silver nanoparticles, elemental silver, zero valent silver, multivalent silver ions carried by zirconium phosphate (ZP-Ag)(See, e.g., Wound Repair and Regeneration, 16: 800-804), and silver containing compounds such as silver sulfadiazine and related compounds. The term "releasable antimicrobial silver composition" refers to a antimicrobial silver composition that can be released from a material, for example, a polymer multilayer solid support, so that antimicrobial activity can be observed. The release of the antimicrobial silver composition can be defined as an amount of the composition released from a defined area or volume of the material.

### DETAILED DESCRIPTION OF THE INVENTION

The complex nature of wounds, and the lack of significant clinical progress based on current therapies, indicates the urgent need for new and unconventional approaches. The microenvironment of the pathologic/chronic wound bed is dysregulated with alterations in extracellular matrix constituents, degradative enzymes, growth factor and other cytoactive factor activity. The present invention provides a process for manufacturing a wound active nanoscale polymer matrix microsheet as claimed in the attached claims.

In normal wound healing, the coordinated interplay between fibroblasts, vascular cells, extracellular matrix components and epithelial cells results in a seamless progression through an inflammatory reaction, wound repair, contracture and coverage by an epithelial barrier. However, in many subjects with dysregulated wound microenvironment, systemic disease or other confounding circumstances, the wound healing processes become asynchronous resulting in an indolent ulcer (Pierce, 2001, Am. J. Pathol. 159:399). In other subjects, a loss or lack of regulatory responses to appropriately modulate cellular behaviors during healing causes an exuberant proliferative response that in itself is a problem for the subject. This is particularly true for patients prone to keloid formation or in burn patients where excessive fibroblastic proliferation and collagen production result in disfiguring and disabling scar formation.

It is clear that across the spectrum of non-healing wounds that there are a variety of inciting mechanisms and wound microenvironments. These wounds exhibit pathology at many junctures in the progression to closure. Deficits in angiogenesis, fibroblastic responses and re-epithelialization all play a role in chronic wounds. Thus, a single factor treatment approach to chronic wounds is not likely to be fruitful across the disparate array of wounds presented in the clinical milieu. In such a heterogeneous environment, a more promising strategy involves the identification of compounds that are able to modulate specific aspects of the cellular response and behavior in the wound environment, providing the potential for custom crafting of a healing response tailored to the individual wound and patient.

Due to the heterogeneous spectrum of wound environments, it is contemplated that stimulating a "desirable" healing response is best achieved by a differential modulation of the cellular responses within the wound.

In contrast, in some burns, such as deep second degree burns where dermal and hair shaft epithelial elements persist to replace lost tissues, a rich angiogenic and epithelial response is needed, but it is desirable to mitigate the fibroblastic reaction to reduce scar hypertrophy, contracture and disfigurement. A similar mitigation is desirable in healing subjects prone to keloid formation where the proliferative fibroblastic response in these wounds must be suppressed. It is also advantageous in wounds near joints or orifices to be able to promote rapid healing and coverage with epithelium but modulate the fibroblastic response so that improved suppleness of the tissue is retained. Modulation of the fibroblastic response in this way has the potential to provide superior clinical outcomes and reduce the need for subsequent reconstructive procedures targeted at recovery of limb or other critical bodily functions. The feasibility of such an approach has been demonstrated previously, such as in the report by Muehlberger and colleagues on the effects tretinoin on incisional wounds (Muehlberger et al., 2005, J. Am. Acad. Derm. 52:583). In that study, application of tretinoin resulted in an increased fibroblastic proliferation but the production of collagen was diminished.

The modification of surfaces has become an important challenge in the last decade for applications in implant materials, prostheses, and artificial organs, allowing broad medical applications for implant and tissue engineering (Langer and Vacanti, 1993, Science 260:920); Peppas and Langer, 1994, Science 263:1715; Angelova and Hunkeler, 1999, Trends Biotechnol. 17:409). For the improved integration efficiency of implants, several approaches, involving the alteration of physicochemical, morphological, and biochemical properties of device surfaces, have been investigated in an effort to obtain a suitable bone-implant interface. Self-assembled monolayers (SAMs) or Langmuir-Blodgett techniques have been commonly employed to produce new interfaces (Mrksich, 1997, Curr. Opin. Colloid Interface Sci. 2:83; Lösche, 1997, Curr. Opin. Solid State Mater. Sci. 2:546).

More recently, a new versatile method of self assembled architectures based on the alternate deposition of polyanions and polycations has been developed for the buildup of multilayered polyelectrolyte films (Decher, 1997, Science 277:1232). Besides varying film thickness, roughness, and porosity, it is also possible to incorporate in the film architecture functionalized macromolecules (Caruso et al., 1997, Langmuir 13:3427; Cassier et al.,1998, Supramol. Sci. 5:309). It has also been demonstrated that the layer-by-layer deposition process is not limited in applicability to polyelectrolytes, but can be applied to viruses, nanoparticles, non-ionic polymers, proteins and other forms of microscopic and nanoscopic matter. A recent review provides information on a wide range of species and interfacial structures that can be formed by the layer-by-layer deposition procedure. The scope of the invention described herein is not limited to polyelectrolytes but applies to all species that have been demonstrated to be incorporated into interfacial structures by the layer-by-layer deposition process.

In some embodiments of the present invention, the wound active agent is silver or a form of silver. Silver is a widely used nonspecific biocidal agent that acts against a very broad spectrum of bacterial species (Yin et al., 1999, J. Burn Care Rehabil. 20:195), yeast species, fungal species (Wright et al., 1999, Am. J Infect. Control 27:344), and viruses (Hussain et al., 1991, Biochem. Biophys. Res. Comm. 189:1444), including several antibiotic resistant strains (Wright et al., 1998, Am. J. Infect. Control 26:572). However, topical silver agents undergo fast inactivation in the wounds because of the highly reactive nature of silver ions. Frequent wound dressings result in large excess of silver being delivered to the wound, resulting in toxic effects from overdose. In vitro studies have shown cytotoxic effects of silver ions on fibroblasts (Poon et al., 2004, Burns 30:140; Hildago et al., Skin Pharmacol. Appl. Skin Physiol. 11:140), keratinocytes (Poon et al., 2004, Burns 30:140), hepatocytes (Baldi et al., 1988, Toxicol. Lett. 41:261), and lymphocytes (Hussain et al., 1991, Biochem. Biophys. Res. Comm. 189:1444).

The highly reactive nature of silver ions complicates their delivery to wound bed. The released silver ions become rapidly inactive as they readily bind to proteins and chloride within complex wound fluid (Mooney et al., 2006, Plastic and Reconstr. Surg. 117:666), resulting in unwanted adsorption of silver ions in epidermis cells and sweat glands (Klasen, 2000, Burns 26:117). Silver based topical solutions like 0.5*%* silver nitrate, or ointments like 1*%* silver sulfadiazine cream, that are currently used in clinics for wound healing, release silver at concentrations up to 3200 ppm but most of this is rapidly inactivated though the formation of chemical complexes in the wound (Dunn et al., Burns, 2004, 30(supplement 1):S1), necessitating frequent applications, up to 12 times a day for silver nitrate solutions and at least twice a day with silver sulfadiazine creams. Frequent dressings result in large excess of silver being delivered to the wound, resulting in toxic effects and discoloration from overdose (Atiyeh et al., 2007, Burns 33:139; Trop et al., 2006, J. Trauma 60:648). Wound-dressings with silver incorporated into the dressing itself have been recently introduced. They provide prolonged release of silver to the wound, allowing dressings to be changed less frequently. However, these reservoir-dressings have to be impregnated with large amount of silver, which results in cytotoxicity. Silver released from a leading commercial wound-dressing Acticoat™, that contains nanocrystalline silver (Dunn et al., Burns, 2004, 30(supplement 1):S1), was shown to be toxic to *in vitro* monolayer cell cultures of keratinocytes and fibroblasts (Poon et al., 2004, Burns 30:140; Trop et al., 2006, J. Trauma 60:648). Thus, the challenges with current topical silver antimicrobials lie in their low silver release levels, the lack of penetration, the rapid consumption of silver ions, and the presence of silver nitrate or cream bases that are pro-inflammatory, negatively affecting wound healing. Issues like wound staining, electrolyte imbalance, and patient discomfort also exist.

In experiments conducted during the course of developing certain embodiments of the present invention (Example 13), nanometer-thick polymeric films were assembled that were impregnated with silver nanoparticles to levels resulting in efficient killing of bacteria but no cytotoxic effects on the adherence and growth of mouse fibroblasts cells on the films. In some embodiments, the polymeric films are impregnated with an antimicrobial silver composition by incubating the film in an antimicrobial silver composition as described in Example 13. In other embodiments, the antimicrobial silver composition is directly incorporated into the polymer multilayer during synthesis of the multilayer. See, e.g., Langmuir. 2005 Dec 6;21(25): 11915-21.

The films produced with the process according to the invention can be applied directly on the exposed wound-bed, allowing them to release the silver ions locally into the wound. This reduces the amount of bactericidal silver needed to impregnate into the film by several orders compared to topical silver delivery agents, significantly reducing the silver-based toxicity. While the present invention is not limited to any particular mechanism, and an understanding of the mechanism is not necessary to practice the present invention, it is contemplated that while silver-impregnated dressings and ointments have to deliver high levels of silver into the wound bed to overcome rapid consumption of silver ions in wound-fluid that limits the penetration of released silver, the localized release of silver right into the wound-matrix by some polymeric thin film embodiments presented herein circumvents the use of large loadings of silver.

Some molecularly-thin composite film embodiments of the present invention can be tuned to contain as low as 0.4 µg/cm² of soluble silver and release a total of less than 1 ppm silver ions in buffers. Such films display 99.9999*%* bacteria-killing efficiency without any measurable cytotoxicity to mammalian cells. Furthermore, films containing these levels of silver allow adherence and growth of mammalian cells (e.g., NIH-3T3 mouse fibroblasts). In some embodiments, the releasable antimicrobial silver composition is loaded at an amount of from 1 or 10 to about 50, 100, 200, 300, 400, 500 or 1000 µg/cm² of the polymer multilayer and/or releasable in an amount of about 0.01 and 100 µg/cm² of the polymer multilayer per day. In some embodiments, the releasable antimicrobial silver composition is releasable in an amount of about 0.05 and 100 µg/cm² of the polymer multilayer. In some embodiments, the releasable antimicrobial silver composition is releasable in an amount of about 0.05 and 20 µg/cm² of the polymer multilayer. In some embodiments, the releasable antimicrobial silver composition is releasable in an amount of about 0.05 and 5 µg/cm² of the polymer multilayer. In some embodiments, the releasable antimicrobial silver composition is releasable in an amount of about 0.05 and 2 µg/cm² of the polymer multilayer. In some embodiments, the releasable antimicrobial silver composition is releasable in an amount of about 0.05 and 1 µg/cm² of the polymer multilayer. In some embodiments, the releasable antimicrobial silver composition is releasable in an amount of about 0.1 to 0.5 µg/cm² of the polymer multilayer.

Experiments conducted during the course of developing some embodiments of the present invention allowed systematic variation of the concentration of silver in the films and demonstrated their cytotoxicity and anti-bacterial efficiency to be dependent on the silver-loading. In comparison, the amount of soluble silver in the commercial wound dressing Acticoat™ is ~100 µg/cm² (Dunn et al., 2004, Burns 30(supplement 1):S1). Acticoat™ releases a total of over 120 ppm silver on dissolution in water for 24 hr (Taylor et al., 2005, Biomaterials 26:7221), and has been shown to be cytotoxic to keratinocytes and fibroblasts (Poon et al., 2005, Burns 30:140; Trop et al., 2006, J. Trauma 60:648).

Several reports exist on composite films of polyelectrolytes that incorporate silver and display anti-bacterial activity by releasing silver in the solution (Lee et al., 2005, Langmuir 21:9651; Li et al., 2006, Langmuir 22:9820; Grunlan et al., 2005, Biomacromolecules 6:1149; Yu et al., 2007, Bioconj. Chem. 18:1521; Shi et al., 2006, J. Biomed. Mat. Res. Part A 76A:826). However, all these studies have focused on controlling the volume fraction and concentration of silver in the films. Only films containing high loadings of silver (-5.5 µg/cm² or more) have been shown to demonstrate anti-bacterial activity (Wang et al., 2002, Langmuir 18:3370; Logar et al., 2007, Nanotechnol. 18:325601). Such polyelectrolyte films, when loaded with those levels of silver, display strong cytotoxicity and kill 100% NIH-3T3 cells seeded on them (e.g., reference Example 13).

In experiments conducted during the course of developing the process of the present invention (e.g., reference Example 13), loading levels of silver in PEMs were identified and achieved that lead to silver-impregnated antibacterial molecularly-thin films permitting adhesion of mammalian cells without exhibiting cytotoxicity. The films find use for the localized delivery of bactericidal silver to the wounds, reducing risks of toxicity from silver overdose observed with other topical delivery agents. Implantable medical devices can be coated with these silver-impregnated thin films to kill bacteria right during their initial attachment to these surfaces and prevent bacterial colonization, a leading cause of implant failure.

Thus, the silver-releasing moleculary-thin composite films as manufactured by the process of the present invention have advantage over: 1) other topical delivery agents of silver currently used in clinics; 2) wound-dressings that contain large amounts of silver in them; and 3) other bactericidal composite thin-films containing large amounts of silver, which are cytotoxic to mammalian cells and do not allow mammalian cells to adhere and grow over them.

Using silver-nanoparticle molecularly-thin films for localized delivery of bactericidal silver in wounds can overcome silver-related toxicity encountered with topical silver-delivery agents for wound healing (Poon et al., 2004, Burns 30:140; Baldi et al., 1988, Toxicol. Lett. 41:261; Atiyeh et al., 2007, Burns 33:139; Coombs et al., 1992, Burns 18:179). Furthermore, these polyelectrolyte multilayers (PEMs) are uniform, highly interpenetrated ultrathin nanocomposite films, typically far less than 1 µm thick (Decher, 1997, Science 277:1232; Hammond, 1999, Curr. Opin. Colloid Interface Sci. 4:430). Their porous and supramolecular architecture allows incorporating a variety of molecules including DNA, enzymes, viruses, dendrimers, colloids, inorganic particles, and dyes (Decher, 1997, Science 277:1232). Thus, along with delivering bactericidal silver, they can be used for simultaneous localized delivery of other bioactive agents in the wound-matrix (e.g., cell recruiting growth factors, DNA plasmids encoding such growth factors). Since PEMs can conformally coat substrates of any type, size or shape, including natural and synthetic polymers (Hammond, 1999, Curr. Opin. Colloid Interface Sci. 4:430), they can be constructed on any wound-bed or implantable medical device.

Figure 1 provides a schematic diagram 100 of a wound bed 110 on which a polyelectrolyte multilayer 130 has been deposited. The diagram 100 depicts that the wound bed 110 comprises a heterogeneous surface depicted by shapes 120 which represent different chemical moieties. The polyelectrolyte multilayer 130 provides a homogenous surface onto which functional groups can 140 can be attached to form a homogenous functionalized surface 150. In the embodiment depicted, the functional groups are uniform, however, in some preferred embodiments, different functional groups are utilized. A wide variety of active agents can then be attached to the surface via the functional groups 140. In other embodiments, the wound bed is covalently modified with covalent modification agents. The covalent modification agents include, but are not limited to, homobifunctional and heterobifunctional cross-linkers as well as photoactivatable cross linkers. Figure 2 provides a schematic diagram 200 of a wound bed 210 comprising a heterogeneous surface depicted by shapes 220 which represent different chemical moieties. The wound bed is covalently modified by reacting covalent modification agents 230 with the different chemical moieties to provide a relatively homogenous functionalized surface 250. In preferred embodiments, covalent modification agents 230 present functional groups 240. In the embodiment depicted, the functional groups are uniform, however, in some preferred embodiments, different functional groups are utilized. A wide variety of active agents can then be attached to the surface via the functional groups 240. These embodiments are discussed in more detail below.

### A. Multilayer deposition on wound bed

With the process of the present invention, a wound active nanoscale polymer matrix microsheet can be manufactured as claimed in claim 1, wherein the wound active nanoscale polymer layer is a polymer multilayer. In some embodiments, the multilayer structures comprise layers of polymers that form polyelectrolytes, while in other embodiments, the multilayers comprise polymers that do not have a charge (i.e., non-ionic polymers) or a combination of charged and uncharged polymer layers. In some embodiments, it is contemplated that polyelectrolyte films built-up by the alternated adsorption of cationic and anionic polyelectrolyte layers constitute a novel and promising technique to modify wound surfaces in a controlled way (Decher et al., 1992, Thin Solid Films 210/211:831; Decher, 1997, Science 277:1232). One of the most important properties of such multilayers is that they exhibit an excess of alternatively positive and negative charges (Caruso et al., 1999, J Am Chem Soc 121:6039; Ladam et al., 2000, Langmuir 16:1249). Not only can this constitute the motor of their buildup (Joanny, 1999, Eur. Phys. J. Biol. 9:117), but it allows, by simple contact, to adsorb a great variety of compounds such as dyes, particles(Cassagneau et al., 1998, J. Am. Chem. Soc. 120:7848; Caruso et al., 1999, Langmuir 15:8276; Lvov et al., 1997, Langmuir 13:6195), clay microplates (Ariga et al., 1999, Appl. Clay Sci. 15:137) and proteins (Keller et al., 1994, J. Am. Chem. Soc. 116:8817; Lvov et al., 1995, J. Am. Chem. Soc. 117:6117; Caruso et al., 1997, Langmuir 13:3427).

In some embodiments, the polymer multilayers, such as polyelectrolyte multilayers, are nanoscale in dimension. Accordingly, in some embodiments, the polymer multilayers are from about 1 nm to 1000 nm thick, from about 1 nm to 500 nm thick, from about 1 nm to 100 nm thick, from about 1 nm to about 25 nm thick, from about 1 nm to about 10 nm thick, or less than about 500 nm, 100 nm, 25 nm or 10 nm thick. It is contemplated that the nanoscale dimension of the polymer multilayers (i.e., the nanoscale thickness) allows for the loading of a lower total amount of an active agent while still allowing delivery of an effective amount (i.e., an amount of active agent that accelerates wound healing as compared to controls) of the active agent as compared to matrix structures with greater thickness. It is contemplated that the lower total loading levels result in reduced toxicity in the wound environment, especially when antimicrobial compounds are incorporated into the polymer multilayer.

In some embodiments, the compliance of the polymer multilayers is adjusted to facilitate cell migration in the wound. In some embodiments, the polymer multilayers exhibit a compliance, measured in kilopascals (kPa) of from about 3 to about 500 kPa, about 7 to about 250 kPa, about 10 to about 250 kPA or from about 10 to about 200 kPa.

### 1. Formation of polyelectrolyte layers

The cationic polyelectrolyte poly(L-lysine) (PLL) interacts with anionic sites on cell surfaces and in the extracellular matrix (Elbert and Hubbell, 1998, J. Biomed. Mater. Res. 42:55).

Polyelectrolyte layers are formed by alternating applications of anionic polyelectrolytes and cationic polyelectrolytes to surfaces to form a polyelectrolyte layer. The layers can be used to deliver a wound active agent to a wound. Preferably, at least four layers, and, more preferably, at least six layers are used to form the polyelectrolyte multilayer. In some embodiments, more than six layers are used, e.g., 7, 8, 9, 10, 15, 20, 25, 30, 35, 40, 45, 50 or more layers. In some preferred embodiments, a polyelectrolyte multilayer comprises 10.5 layers (e.g., comprising 10 bilayers of the two components and an additional layer of one of the two components).

In some embodiments, the cationic polyelectrolyte used is PLL and the anionic polyelectrolyte used is poly(L-glutamic acid) (PGA). Indeed, the use of a variety of polyelectrolytes is contemplated, including, but not limited to, poly(ethylene imine) (PEI), poly(allylamine hydrochloride) (PAH), poly(sodium 4-styrenesulfonate) (PSS), poly(acrylic acid) (PAC), poly(maleic acid-co-propylene) (PMA-P), poly(acrylic acid) (PAA), and poly(vinyl sulfate) (PVS). It is also possible to use naturally occurring polyelectrolytes, including hyaluronic acid and chondroitin sulfate.

In still further embodiments, the polymer is a dendrimer, grafted polymer, or star architecture polymer. In other embodiments, the multilayer responds to or is organized in the presence of an electric field, for example an electric field formed by placing electrodes on either side of a wound.

Referring to Figure 1, the polymer multilayer 130 can be comprised of polystyrene sulfonate, an amphiphillic polyelectrolyte with an affinity for hydrophobic regions of the wound bed, and an amphoteric polymer. The polymer multilayer is preferably functionalized with one or more crosslinking agents presenting an alkyne so that a uniform surface is presented (e.g., 140 in Figure 1 where x represents an alkyne group). From this point, widely available click chemistries can be used to add desired wound active agents to the modified surface of the wound. In some embodiments, the wound modifying agent is an azide conjugate or otherwise comprises an azide group and is reacted with the alkyne groups displayed on the wound bed in a Huisgen Cycloaddition.

Other suitable methods for preparing polyelectrolyte multilayers include those described, for example, in Cho and Char, Langmuir 20:4011-4016, 2004; Okamura et al., Adv. Mater. 21, 4388-92 (2009), Cho et al., Adv. Mat. 13(14):1076-1078 (2001); and U.S. pat. Publ. 2010/0062258. Suitable methods include layer by layer deposition, formation on SAMs, and spin coating assisted assembly.

### 2. Cationic Polymers

Cationic polymers useful in the present invention can be any biocompatible water-soluble polycationic polymer, for example, any polymer having protonated heterocycles attached as pendant groups. As used herein, "water soluble" means that the entire polymer must be soluble in aqueous solutions, such as buffered saline or buffered saline with small amounts of added organic solvents as co-solvents, at a temperature between 20 and 37°C. In some embodiments, the material will not be sufficiently soluble (defined herein as soluble to the extent of at least one gram per liter) in aqueous solutions per se but can be brought into solution by grafting the polycationic polymer with water-soluble polynonionic materials such as polyethylene glycol.

Representative cationic polymers include natural and unnatural polyamino acids having net positive charge at neutral pH, positively charged polysaccharides, and positively charged synthetic polymers. Examples of suitable polycationic materials include polyamines having amine groups on either the polymer backbone or the polymer side chains, such as poly-L-lysine (PLL) and other positively charged polyamino acids of natural or synthetic amino acids or mixtures of amino acids, including, but not limited to, poly(D-lysine), poly(ornithine), poly(arginine), and poly(histidine), and nonpeptide polyamines such as poly(aminostyrene), poly(aminoacrylate), poly (N-methyl aminoacrylate), poly (N-ethylaminoacrylate), poly(N,N-dimethyl aminoacrylate), poly(N,N-diethylaminoacrylate), poly(aminomethacrylate), poly(N-methyl amino-methacrylate), poly(N-ethyl amino methacrylate), poly(N,N-dimethyl amino methacrylate), poly(N,N-diethyl amino methacrylate), poly(ethyleneimine), polymers of quaternary amines, such as poly(N,N,N-trimethylaminoacrylate chloride), poly(methyacrylamidopropyltrimethyl ammonium chloride), and natural or synthetic polysaccharides such as chitosan. In some embodiments, PLL is a preferred material. In some preferred embodiments, the cationic polymer is poly(allylamine hydrochoride) (PAH).

In general, the polymers must include at least five charges, and the molecular weight of the polycationic material must be sufficient to yield the desired degree of binding to a tissue or other surface, having a molecular weight of at least 1000 g/mole.

### 3. Anionic Polymers

Polyanionic materials useful in the present invention can be any biocompatible water-soluble polyanionic polymer, for example, any polymer having carboxylic acid groups attached as pendant groups. Suitable materials include alginate, carrageenan, furcellaran, pectin, xanthan, hyaluronic acid, heparin, heparan sulfate, chondroitin s ulfate, dermatan sulfate, dextran sulfate, poly(meth)acrylic acid, oxidized cellulose, carboxymethyl cellulose and crosmarmelose, synthetic polymers and copolymers containing pendant carboxyl groups, such as those containing maleic acid or fumaric acid in the backbone. Polyaminoacids of predominantly negative charge are also suitable. Examples of these materials include polyaspartic acid, polyglutamic acid, and copolymers thereof with other natural and unnatural amino acids. Polyphenolic materials such as tannins and lignins can be used if they are sufficiently biocompatible. Preferred materials include alginate, pectin, carboxymethyl cellulose, heparin and hyaluronic acid. In some preferred embodiments, the anionic polymer is poly(acrylic acid) (PAA).

### 4. Nonionic Polymers

In some embodiments, the multilayer structures are formed from uncharged polymers or from a combination of charged and uncharged polymers. Examples of uncharged polymers include, but are not limited to, dextran, dextran sulfate, diethylaminoethyl (DEAE)-dextran, hydroxyethyl cellulose, ethyl(hydroxyethyl) cellulose, acrylamide, polyethylene oxide, polypropylene oxide, polyethylene oxide - polypropylene oxide copolymers, PAANₐ, Ficoll, polyvinylpyrolidine, and polyacrylic acid.

### 5. Amphoteric polymers

In some embodiments, the multilayer structures are formed from one or more amphoteric polymers, alone in combination with the other polymers described herein. In some embodiments, the amphoteric polymers comprise one or more of acrylic acid (AA), DMAEMA (dimethylaminoethyl methacrylate), APA (2-aminopropyl acrylate), MorphEMA (morpholinoethyl methacrylate), DEAEMA (diethylaminoethyl methacrylate), t-ButylAEMA (t-butylaminoethyl methacrylate), PipEMA (piperidinoethyl methacrylate), AEMA (aminoethyl methacrylate), HEMA (2-hydroxyethyl methacrylate), MA (methyl acrylate), MAA (methacrylic acid) APMA (2-aminopropyl methacrylate), AEA (aminoethyl acrylate). In some embodiments, the amphoteric polymer comprises (a) carboxylic acid, (b) primary amine, and (c) secondary and/or tertiary amine. The amphoteric polymers have an isoelectric point of 4 to 8, preferably 5 to 7 and have a number average molecular weight in the range of 10,000 to 150,000.

### 6. Application of multilayers

In some embodiments, a polymer multilayer is produced on a supporting substrate (e.g., PDMS), removed from the supporting substrate, then laid on a wound. In some embodiments, a polymer multilayer is produced on a supporting substrate (e.g., PDMS), overlaid with a dissolvable sacrificial support layer, e.g., comprising a biocompatible polymer (e.g., polyvinyl alcohol (PVA)). The sacrificial support layer facilitates the removal of the polymer multilayer from the supporting substrate, facilitates manual handling of the polymer multilayer, and/or facilitates application of the polymer multilayer on a wound. Then, after placement, the support layer dissolves and thus promotes the adherence of the polymer multilayer to the micro-topography and nano-topography of the wound.

### II. Wound Active Agents

Wound active agents can be delivered to a wound bed or incorporated into a wound bed using the systems described above that have been manufactured with the process of the invention . In some embodiments, the wound active agent is bound covalently or non-covalently with the polyelectrolyte layer,. The present invention is not limited to a particular mechanism by which the wound active agent binds to the polyelectrolyte layer.

The polyelectrolyte layer may function as a drug delivery scaffold to deliver one or more wound active agents to the wound. Wound active agents that may be desirable to deliver include, but are not limited to, trophic factors, extracellular matrices (ECMs), ECM fragments or synthetic constructs, enzymes, enzyme inhibitors, defensins, polypeptides, anti-infective agents (including antimicrobials, antivirals and antifungals), buffering agents, vitamins and minerals, analgesics, anticoagulants, coagulation factors, anti-inflammatory agents, vasoconstrictors, vasodilators, diuretics, and anti-cancer agents. In addition, would active agents include chlorhexidine, iodine based antimicrobials such as PVP-iodine; selenium based antimicrobials such as 7-azabenzisoselenazol-3(2H)-ones, selenium disulfide, and selenides; and silver based antimicrobials (e.g., silver sulfadiazine, ionic silver, elemental silver, silver nanoparticles)). With respect to selenides, with the use of standard and variations of typical protein and carbohydrate attachment chemistries, carboxyl and amino containing selenides may be routinely attached to many polymers, peptides, antibodies, steroids and drugs. Polymers and other molecules with attached selenides generate superoxide in a dose dependent manner in biological solutions, in cells or attached to insoluble matrixes such as silicones.

A wide variety of wound active agents can be incorporated into the polyelectrolyte layer, wherein the wound active agents are released from the polyelectrolyte layer into the wound.

A wide variety of wound active agents can be trophic factors, including, but not limited to, agrin, amphiregulin, artemin, cardiotrophin-1, epidermal growth factors including EGF; fibroblast growth factors (e.g., FGF-1, FGF-2, FGF-3, FGF-4, FGF-5, FGF-6, and FGF-7); LIF, CSF-1, CSF-2, CSF-3, erythropoietin, endothelial cell growth factors including ECGF; FGF- related and ECGF-related growth factors (e.g., endothelial cell stimulating angiogenesis factor, tumor angiogenesis factor, retina-derived growth factor (RDGF), vascular endothelium growth factor (VEGF), brain-derived growth factors (BDGF-A and B), astroglial growth factors (AGF 1 and 2), omentum-derived growth factor, fibroblast-stimulating factor (FSF), and embryonal carcinoma-derived growth factor (ECDGF)); neurotrophic growth factors (e.g, nerve growth factors (NGFs), neurturin, brain-derived neurotrophic factor (BDNF), neurotrophin-3, neurotrophin-4, and ciliary neurotrophic factor (CNTF)); glial growth factors (e.g., GGF-I, GGF-II, GGF-III, glia maturation factor (GMF), and glial-derived neurotrophic factor (GDNF)); liver growth factors (e.g., hepatopoietin A, hepatopoietin B, and hepatocyte growth factors including HGF); prostate growth factors including prostate-derived growth factors (PGFs); mammary growth factors including mammary-derived growth factor 1 (MDGF-1) and mammary tumor-derived factor (MTGF); heart growth factors including nonmyocyte-derived growth factor (NMDGF); melanocyte growth factors including melanocyte-stimulating hormone (MSH) and melanoma growth-stimulating activity (MGSA); angiogenic factors (e.g., angiogenin, angiotropin, platelet-derived ECGF, VEGF, and pleiotrophin); transforming growth factors including TGF-α and TGF-β; TGF-like growth factors (e.g., TGF-beta₁, TGF-beta₂, TGF-beta₃, GDF-1, CDGF, tumor-derived TGF-like factors, ND-TGF, and human epithelial transforming factor); regulatory peptides with growth factor-like properties (e.g., bombesin and bombesin-like peptides ranatensin and litorin, angiotensin, endothelin, atrial natriuretic factor, vasoactive intestinal peptide, and bradykinin); platelet-derived growth factors including PDGF-A, PDGF-B, and PDGF-AB; neuropeptides (e.g., substance P, calcitonin gene-regulated peptide (CGRP), and neuropeptide Y); neurotransmitters and their analogs including norepinephrine, acetylcholine and carbachol; hedgehog, heregulin/neuregulin, IL-1, osteoclast-activating factor (OAF), lymphocyte-activating factor (LAF), hepatocyte-stimulating factor (HSF), B-cell-activating factor (BAF), tumor inhibitory factor 2 (TIF-2), keratinocyte-derived T-cell growth factor (KD-TCGF), IL-2, IL-3, IL-4, IL-5, IL-6, IL-7, IL-8, IL-9, IL-10, IL-11, stromal cell-derived cytokine (SCDC), IL-12, IL-13, IL-14, IL-15, insulin, insulin-like growth factors including IGF-1, IGF-2, and IGF-BP; interferons including INF-alpha, INF-beta, and INF-gamma; leptin, midkine, tumor necrosis factors (TNF-alpha and beta), netrins, saposins, semaphorins, somatrem, somatropin, stem cell factor, VVGF, bone morphogenetic proteins (BMPs), adhesion molecules, other cytokines, heparin-binding growth factors, and tyrosine kinase receptor ligands. In some embodiments, the wound active agent is a peptide such as AcEEED, which is the N terminal peptide for alpha smooth muscle actin and has been shown to inhibit contractile properties of myofibroblasts.

In some embodiments, the wound active agents are integrin binding sequences exemplified by, but not limited to RGD, EILDV, VCAM-1 and their recombined or synthetic analogs, enzymes, enzyme inhibitors, and polypeptides.

In some embodiments, the enzymes include exopeptidases and endopeptidases (also known as proteases and proteinases), including but not limited to the serine proteinases chymotrypsin, trypsin, elastase, and kallikrein, bacterial enzymes, the cysteine proteases papain, actinin, bromelain, cathepsins, cytosolic calpains, parasitic proteases, aspartic proteinases, the pepsin family of proteases pepsin and chymosin, lysosomal cathepsins D, renin, fungal proteases, the viral proteases, AIDS virus retropepsin, and the metalloproteinases (MMPs), collagenases, Maggott enzyme, MMP1, MMP2, MMP8, MMP13, gelatinases, MMP2, MMP9, MMP3, MMP7, MMP10, MMP11, and MMP12.

In some embodiments, the enzyme inhibitors include captopril, thiorphan, phosphoramidon, teprotide, protease and proteinase inhibitors, metalloproteinase inhibitors and exopeptidase inhibitors.

In some embodiments, defensins, including, but not limited to, alpha-defensins HNP 1, 2, 3 and 4, and beta-defensins HBD-1 and HBD-2 are used, or polypeptides such as fibronectin, serotonin, PAF, PDEGF, TNFa, IL1, IL6, IGF, IGF-1, IGF-2, IL-1, PDGF, FGF, KGF, VEGF, bradykinin, prothymosin-alpha, and thymosin-alpha1, or antimicrobials, including, but not limited to, magainin (e.g., magainin I, magainin II, xenopsin, xenopsin precursor fragment, caerulein precursor fragment), magainin I and II analogs (e.g., PGLa, magainin A, magainin G, pexiganin, Z-12, pexigainin acetate, D35, MSI-78A, MG0 (K10E, K11E, F12W-magainin 2), MG2+ (K10E, F12W-magainin-2), MG4+ (F12W-magainin 2), MG6+ (f12W, E19Q-magainin 2 amide), MSI-238, reversed magainin II analogs (e.g., 53D, 87-ISM, and A87-ISM), Ala-magainin II amide, magainin II amide), cecropin P1, cecropin A, cecropin B, indolicidin, nisin, ranalexin, lactoferricin B, poly-L-lysine, cecropin A (1-8)-magainin II (1-12), cecropin A (1-8)-melittin (1-12), CA(1-13)-MA(1-13), CA(1-13)-ME(1-13), gramicidin, gramicidin A, gramicidin D, gramicidin S, alamethicin, protegrin, histatin, dermaseptin, lentivirus amphipathic peptide or analog, parasin I, lycotoxin I or II, globomycin, gramicidin S, surfactin, ralinomycin, valinomycin, polymyxin B, PM2 ((+/-) 1-(4-aminobutyl)-6-benzylindane), PM2c ((+/-) -6-benzyl-1-(3-carboxypropyl)indane), PM3 ((+/-) 1-benzyl-6-(4-aminobutyl)indane), tachyplesin, buforin I or II, misgurin, melittin, PR-39, PR-26, 9-phenylnonylamine, (KLAKKLA)n, (KLAKLAK)n, where n = 1, 2, or 3, (KALKALK)3, KLGKKLG)n, and KAAKKAA)n, wherein N = 1, 2, or 3, paradaxin, Bac 5, Bac 7, ceratoxin, mdelin 1 and 5, bombin-like peptides, PGQ, cathelicidin, HD-5, Oabac5alpha, ChBac5, SMAP-29, Bac7.5, lactoferrin, granulysin, thionin, hevein and knottin-like peptides, MPG1, 1bAMP, snakin, lipid transfer proteins, and plant defensins. Exemplary sequences for the above compounds are provided in Table 1. In some embodiments, the antimicrobial peptides are synthesized from L-amino acids, while in other embodiments, the peptides are synthesized from, or comprise, D-amino acids. Additional antimicrobial polypeptides of use in the present invention are listed in Figure 7.

**TABLE 1**

| Antimicrobial Peptides | | | |
|---|---|---|---|
| SEQ ID NO: | Name | Organism | Sequence |
| 1 | lingual antimicrobial peptide precursor (Magainin) | *Bos taurus* | |
| 2 | antimicrobial peptide PGQ | *Xenopus laevis* | gvlsnvigylkklgtgalnavlkq |
| 3 | Xenopsin | *Xenopus laevis* | |
| 4 | magainin precursor | *Xenopus laevis* | |
| 5 | tachyplesin I | *Tachypleus gigas* | kwcfrvcyrgicyrrcr |
| 6 | tachyplesin II | *Tachypleus gigas* | rwcfrvcyrgicyrkcr |
| 7 | buforin I | *Bufo bufo gagarizans* | |
| 8 | buforin II | *Bufo bufo gagarizans* | trssraglqfpvgrvhrllrk |
| 9 | cecropin A | *Bombyx mori* | |
| 10 | cecropin B | *Bombyx mori* | |
| 11 | cecropin C | *Drosophila melanogaster* | |
| 12 | cecropin PI | *Sus scrofa* | swlsktakklensakkrisegiaiaiqggpr |
| 13 | indolicidin | *Bos taurus* | ilpwkwpwwpwrr |
| 14 | nisin | *Lactococcus lactis* | itsislctpgcktgalmgcnmktatchcsihvsk |
| 15 | ran alexin | *Rana catesbeiana* | flgglikivpamicavtkkc |
| 16 | lactoferricin B | *Bos taurus* | fkcrrwqwrmkklgapsitcvrraf |
| 17 | protegrin -1 | *Sus scrofa* | rggrlcycrrrfcvcvgrx |
| 18 | protegrin-2 | *Sus scrofa* | ggrlcycrrrfcicvg |
| 19 | histatin precursor | *Homo sapiens* | |
| 20 | histatin 1 | *Macaca fascicularis* | dsheerhhgrhghhkygrkthekhhshrgyrsnylydn |
| 21 | dermaseptin | *Phyllomedusa sauvagei* | alwktmlkklgtmalhagkaalgaaadtisqtq |
| 22 | dermaseptin 2 | *Phyllomedusa sauvagei* | alwftmlkklgtmalhagkaalgaaantisqgtq |
| 23 | dermaseptin 3 | *Phyllomedusa sauvagei* | alwknmlkgigklagkaalgavkklvgaes |
| 24 | misgurin | *Misgurnus anguillicaudatu s* | rqrveelskfskkgaaarrrk |
| 25 | melittin | *Apis mellifera* | gigavlkvlttglpaliswisrkkrqq |
| 26 | pardaxin-1 | *Pardachirus pavoninus* | gffalipkiissplfktllsavgsalsssgeqe |
| 27 | pardaxin-2 | *Pardachirus pavoninus* | gffalipkiisspifktllsavgsalsssggqe |
| 28 | bactenecin 5 precursor | *Bos taurus* | |
| 29 | bactenecin precursor | *Bos taurus* | |
| 30 | ceratotoxin A | *Ceratitis capitata* | sigsalkkalpvakkigkialpiakaalp |
| 31 | ceratotoxin B | *Ceratitis capitata* | sigsafkkalpvakkigkaalpiakaalp |
| 32 | cathelicidin antimicrobial peptide | *Homo sapiens* | |
| 33 | myeloid cathelicidin 3 | *Equus caballus* | |
| 34 | myeloid antimicrobial peptide BMAP-28 | *Bos taurus* | |
| 35 | myeloid cathelicidin 1 | *Equus caballus* | |
| 36 | SMAP 29 | *Ovis aries* | |
| 37 | BNP-1 | *Bos taurus* | rlcrivvirvcr |
| 38 | HNP-1 | *Homo sapiens* | acycripaciagerrygtciyqgrlwafcc |
| 39 | HNP-2 | *Homo sapiens* | cycripaciagerrygtciyqgrlwafcc |
| 40 | HNP-3 | *Homo sapiens* | dcycripaciagerrygtciyqgrlwafcc |
| 41 | HNP-4 | *Homo sapiens* | vcscrlvfcrrtelrvgncliggvsftycctrv |
| 42 | NP-1 | *Oryctolagus cuniculus* | vvcacrralclprerragfcrirgrihplccrr |
| 43 | NP-2 | *Oryctolagus cuniculus* | vvcacrralclplerragfcrirgrihplccrr |
| 44 | NP-3A | *Oryctolagus cuniculus* | gicacrrrfcpnserfsgycrvngaryvrccsrr |
| 45 | NP-3B | *Oryctolagus cuniculus* | grcvcrkqllcs yrerrigdckirgvrfpfccpr |
| 46 | NP-4 | *Oryctolagus cuniculus* | vsctcrrfscgfgerasgsctvnggvrhtlccrr |
| 47 | NP-5 | *Oryctolagus cuniculus* | vfctcrgflcgsgerasgsctingvrhtlccrr |
| 48 | RatNP-1 | *Rattus norvegicus* | vtcycrrtrcgfrerlsgacgyrgriyrlccr |
| 49 | Rat-NP-3 | *Rattus norvegicus* | cscrysscrfgerllsgacrlngriyrlcc |
| 50 | Rat-NP-4 | *Rattus norvegicus* | actcrigacvsgerltgacglngriyrlccr |
| 51 | GPNP | Guinea pig | rrcicttrtcrfpyrrlgtcifqnrvytfcc |
| 52 | beta defensin-3 | Homo sapiens | |
| 53 | theta defensin-1 | Macaca mulatta | rcictrgfcrclcrrgvc |
| 54 | defensin CUA1 | Helianthus annuus | |
| 55 | defensin SD2 | Helianthus annuus | |
| 56 | neutrophil defensin 2 | Macaca mulatta | acycripaclagerrygtcfymgrvwafcc |
| 57 | 4 KDA defensin | Androctonus australis hector | gfgcpfnqgachrhcrsirrrggycaglfkqtctcyr |
| 58 | defensin | *Mytilus galloprovinciali s* | gfgcpnnyqchrhcksipgrcggycggxhrlrctcyrc |
| 59 | defensin AMP1 | Heuchera sanguinea | |
| 60 | defensin AMP1 | Clitoria ternatea | |
| 61 | cysteine-rich cryptdin-1 homolog | Mus musculus | |
| 62 | beta-defensin-9 | Bos taurus | qgvrnfvtcrinrgfcvpircpghrrqigtclgpqikccr |
| 63 | beta-defensin-7 | Bos taurus | qgvrnfvtcrinrgfcvpircpghrrqigtclgprikccr |
| 64 | beta-defensin-6 | Bos taurus | qgvrnhvtcriyggfcvpircpgrtrqigtcfgrpvkccrrw |
| 65 | beta-defensin-5 | Bos taurus | qvvrnpqscrwnmgvcipiscpgnmrqigtcfgprvpccr |
| 66 | beta-defensin-4 | Bos taurus | qrvrnpqscrwnmgvcipflcrvgmrqigtcfgprvpccrr |
| 67 | beta-defensin-3 | *Bos taurus* | qgvrnhvtcrinrgfcvpircpgrtrqigtcfgprikccrsw |
| 68 | beta-defensin-10 | *Bos taurus* | qgvrsylscwgnrgicllnrcpgrmrqigtclaprvkccr |
| 69 | beta-defensin-13 | *Bos taurus* | sgisgplscgrnggvcipircpvpmrqigtcfgrpvkccrsw |
| 70 | beta-defensin-1 | *Bos taurus* | dfaschtnggiclpnrcpghmiqigicfrprvkccrsw |
| 71 | coleoptericin | *Zophobas atratus* | |
| 72 | beta defensin-3 | *Homo sapiens* | |
| 73 | defensin C | *Aedes aegypti* | atcdllsgfgvgdsacaahciargnrggycnskkvcvcrn |
| 74 | defensin B | *Mytilus edulis* | gfgcpndypchrhcksipgryggycggxhrlrctc |
| 75 | sapecin C | *Sarcophaga peregrina* | atcdllsgigvqhsacalhcvfrgnrggyctgkgicvcrn |
| 76 | macrophage antibiotic peptide MCP-1 | *Oryctolagus cuniculus* | |
| 77 | cryptdin-2 | *Mus musculus* | |
| 78 | cryptdin-5 | *Mus musculus* | |
| 79 | cryptdin 12 | *Mus musculus* | lrdlvcycrargckgrermngtcrkghllymlccr |
| 80 | defensin | *Pyrrhocoris apterus* | atcdilsfqsqwvtpnhagcalhcvikgykggqckitvchcrr |
| 81 | defensin R-5 | *Rattus norvegicus* | vtcycrstrcgfrerlsgacgyrgriyrlccr |
| 82 | defensin R-2 | *Rattus norvegicus* | vtcscrtsscrfgerlsgacrlngriyrlcc |
| 83 | defensin NP-6 | *Oryctolagus cuniculus* | gicacrrrfclnfeqfsgycrvngaryvrccsrr |
| 84 | beta-defensin-2 | *Pan troglodytes* | |
| 85 | beta-defensin-2 | *Homo sapiens* | |
| 86 | beta-defensin-1 | *Homo sapiens* | |
| 87 | beta-defensin-1 | *Capra hircus* | |
| 88 | beta defensin-2 | *Capra hircus* | |
| 89 | defensin-3 | *Macaca mulatta* | |
| 90 | defensin -1 | *Macaca mulatta* | |
| 91 | neutrophil defensin 1 | *Mesocricetus auratus* | vtcfcrrrgcasrerhigycrfgntiyrlccrr |
| 92 | neutrophil defensin 1 | *Mesocricetus auratus* | cfckrpvcdsgetqigycrlgntfyrlccrq |
| 93 | Gallinacin 1-alpha | *Gallus gallus* | grksdcfrkngfcaflkcpyltlisgkcsrfhlcckriw |
| 94 | defensin | *Allomyrina dichotoma* | vtcdllsfeakgfaanhslcaahclaigrrggscergvcicrr |
| 95 | neutrophil cationic peptide 1 | *Cavia porcellus* | rrcicttrtcrfpyrrlgtcifqnrvytfcc |

In some embodiments, the antimicrobials include loracarbef, cephalexin, cefadroxil, cefixime, ceftibuten, cefprozil, cefpodoxime, cephradine, cefuroxime, cefaclor, neomycin/polymyxin/bacitracin, dicloxacillin, nitrofurantoin, nitrofurantoin macrocrystal, nitrofurantoin/nitrofuran mac, dirithromycin, gemifloxacin, ampicillin, gatifloxacin, penicillin V potassium, ciprofloxacin, enoxacin, amoxicillin, amoxicillin/clavulanate potassium, clarithromycin, levofloxacin, moxifloxacin, azithromycin, sparfloxacin, cefdinir, ofloxacin, trovafloxacin, lomefloxacin, methenamine, erythromycin, norfloxacin, clindamycin/benzoyl peroxide, quinupristin/dalfopristin, doxycycline, amikacin sulfate, vancomycin, kanamycin, netilmicin, streptomycin, tobramycin sulfate, gentamicin sulfate, tetracyclines, framycetin, minocycline, nalidixic acid, demeclocycline, trimethoprim, miconazole, colistimethate, piperacillin sodium/tazobactam sodium, paromomycin, colistin/neomycin/hydrocortisone, amebicides, sulfisoxazole, pentamidine, sulfadiazine, clindamycin phosphate, metronidazole, oxacillin sodium, nafcillin sodium, vancomycin hydrochloride, clindamycin, cefotaxime sodium, co-trimoxazole, ticarcillin disodium, piperacillin sodium, ticarcillin disodium/clavulanate potassium, neomycin, daptomycin, cefazolin sodium, cefoxitin sodium, ceftizoxime sodium, penicillin G potassium and sodium, ceftriaxone sodium, ceftazidime, imipenem/cilastatin sodium, aztreonam, cinoxacin, erythromycin/sulfisoxazole, cefotetan disodium, ampicillin sodium/sulbactam sodium, cefoperazone sodium, cefamandole nafate, gentamicin, sulfisoxazole/phenazopyridine, tobramycin, lincomycin, neomycin/polymyxin B/gramicidin, clindamycin hydrochloride, lansoprazole/clarithromycin/amoxicillin, alatrofloxacin, linezolid, bismuth subsalicylate/metronidazole/tetracycline, erythromycin/benzoyl peroxide, mupirocin, fosfomycin, pentamidine isethionate, imipenem/cilastatin, troleandomycin, gatifloxacin, chloramphenicol, cycloserine, neomycin/polymyxin B/hydrocortisone, ertapenem, meropenem, cephalosporins, fluconazole, cefepime, sulfamethoxazole, sulfamethoxazole/trimethoprim, neomycin/polymyxin B, penicillins, rifampin/isoniazid, erythromycin estolate, erythromycin ethylsuccinate, erythromycin stearate, ampicillin trihydrate, ampicillin/probenecid, sulfasalazine, sulfanilamide, sodium sulfacetamide, dapsone, doxycycline hyclate, trimenthoprim/sulfa, methenamine mandelate, plasmodicides, pyrimethamine, hydroxychloroquine, chloroquine phosphate, trichomonocides, anthelmintics, atovaquone, bacitracin, bacitracin/polymyxin b, gentamycin, neomycin/polymyxin/dexameth, neomycin sulf/dexameth, sulfacetamide/prednisolone, sulfacetamide/phenylephrine, tobramycin sulfate/dexameth, bismuth tribromophenate, silver ion compounds, silver nanoparticles, zerovalent silver, multivalent silver, elemental silver, and silver containing compounds such as silver sulfadiazine and related compounds.

In some embodiments, antivirals include amantadine, acyclovir, foscarnet, indinavir, ribavirin, enfuvirtide, emtricitabine, lamivudine, abacavir sulfate, fomivirsen, valacyclovir, tenofovir, cidofovir, atazanavir, amprenavir, delavirdine mesylate, famciclovir, adefovir, didanosine, efavirenz, trifluridine, inidinavir, lamivudine, vidarabine, lopinavir/ritonavir, ganciclovir, zanamivir, abacavir/lamivudine/zidovudine, lamivudine/zidovudine, nelfinavir, nelfinavir mesylate, nevirapine, ritonavir, saquinavir, saquinavir mesylate, rimantadine, stavudine, docosanol, zalcitabine, idoxuridine, zidovudine, zidovudine/didanosine, valganciclovir, penciclovir, lamivudine, and oseltamivir.

In some embodiments, antifungals includeamphotericin B, nystatin, nystatin/triamcinolone, itraconazole, ketoconazole, miconazole, sulconazole, clotrimazole, clotrimazole/betamethasone, enilconazole, econazole, oxiconazole, tioconazole, terconazole, butoconazole, thiabendazole, flucytosine, butenafine, ciclopirox, haloprogin, naftifine, tolnaftate, natamycin, undecylenic acid, mafenide, dapsone, clioquinol, clioquinol/hydrocortisone, potassium iodide, silver sulfadiazine, gentian violet, carbol-fuchsin, cilofungin, sertaconazole, voriconazole, fluconazole, terbinafine, caspofungin, other topical azole drugs, and griseofulvin.

In some embodiments, the buffering agents include Maleic acid, Phosphoric acid, Glycine, Chloroacetic acid, Formic acid, Benzoic acid, Acetic acid, Pyridine, Piperazine, MES, Bis-tris, Carbonate, ACES, ADA MOPSO, PIPES, Phosphoric acid, BES, MOPS, TES, HEPES, DIPSO, TAPSO, Triethanolamine, HEPSO, Tris, Tricine, Bicine, TAPS, Borate, Ammonia, CHES, Ethanolamine, CAPSO, Glycine, Carbonate, CAPS, Methylamine, Piperidine, and Phosphoric acid.

In some embodiments, the vitamins and minerals, include Vitamin A, Carotenoids, Vitamin D, Vitamin E, Vitamin K, Vitamin C/ascorbic acid, B1/thiamin, B2/riboflavin, B3/niacin, B5/pantothenic acid, B6/pyridoxine, B12/cobalamin, Biotin, Calcium, Magnesium, Phosphorus, Sodium, Chloride, Potassium, Boron, Chromium, Copper, Iodine, Iron, Manganese, Selenium, and Zinc.

In some embodiments, the the analgesics include acetaminophen, anileridine, acetylsalicylic acid, buprenorphine, butorphanol, fentanyl, fentanyl citrate, codeine, rofecoxib, hydrocodone, hydromorphone, hydromorphone hydrochloride, levorphanol, alfentanil hydrochloride, meperidine, meperidine hydrochloride, methadone, morphine, nalbuphine, opium, levomethadyl, hyaluronate sodium, sufentanil citrate, capsaicin, tramadol, leflunomide, oxycodone, oxymorphone, celecoxib, pentazocine, propoxyphene, benzocaine, lidocaine, dezocine, clonidine, butalbital, phenobarbital, tetracaine, phenazopyridine, sulfamethoxazole/phenazopyridine, and sulfisoxazole/phenazopyridine.

In some embodiments, the anticoagulants include coumarins, 1,3-indandione, anisindione, fondaparinux, heparin, lepirudin, antithrombin, warfarin, enoxaparin, dipyridamole, dalteparin, ardeparin, nadroparin, and tinzaparin.

In some embodiments, the coagulation factors includeFactor I (fibrinogen), Factor II (prothrombin), Factor III (thromboplastin, tissue factor), Factor IV (calcium), Factor V (labile factor), Factor VII (stable factor), Factor VIII (antihemophilic globulin, antihemophilic globulin, antihemophilic factor A), Factor IX (plasma thromboplastin component, Christmas factor, antihemophilic factor B), Factor X (Stuart factor, Prower factor, Stuart-Prower factor), Factor XI (plasma thromboplastin antecedent, antihemophilic factor C), Factor XII (Hageman factor, surface factor, contact factor), and Factor XIII (fibrin stabilizing factor, fibrin stabilizing enzyme, fibri-nase).

In some embodiments, the anti-inflammatory agents include non steroidal anti-inflammatory drugs (NSAIDs) including diclofenac (also known as Voltaren, Abitren, Allvoran, Almiral, Alonpin, Anfenax, Artrites, Betaren, Blesin, Bolabomin, Cataflam, Clofec, Clofen, Cordralan, Curinflam, Diclomax, Diclosian, Dicsnal, Difenac, Ecofenac, Hizemin, Inflamac, Inflanac, Klotaren, Lidonin, Monoflam, Naboal, Oritaren, Remethan, Savismin, Silino, Staren, Tsudohmin, Voltarol, Voren, Voveran, and Vurdon), diflunisal (also known as Dolobid, Adomal, Diflonid, Diflunil, Dolisal, Dolobis, Dolocid, Donobid, Dopanone, Dorbid, Dugodol, Flovacil, Fluniget, Fluodonil, Flustar, Ilacen, Noaldol, Reuflos, and Unisal), etodolac (also known as Lodine), fenoprofen (also known as Nalfon, Fenoprex, Fenopron, Fepron, Nalgesic, and Progesic), flurbiprofen (also known as Ansaid and Ocuflur), ibuprofen (also known as Rufen, Motrin, Aches-N-Pain, Advil, Nuprin, Dolgesic, Genpril, Haltran, Ibifon, Ibren, Ibumed, Ibuprin, Ibupro-600, Ibuprohm, Ibu-Tab, Ibutex, Ifen, Medipren, Midol 200, Motrin-IB, Cramp End, Profen, Ro-Profen, Trendar, Alaxan, Brofen, Alfam, Brufen, Algofen, Brufort, Amersol, Bruzon, Andran, Buburone, Anflagen, Butacortelone, Apsifen, Deflem, Artofen, Dolgit, Artril, Dolocyl, Bloom, Donjust, Bluton, Easifon, Ebufac, Emflam, Emodin, Fenbid, Fenspan, Focus, Ibosure, Ibufen, Ibufug, Ibugen, Ibumetin, Ibupirac, Imbun, Inabrin, Inflam, Irfen, Librofen, Limidon, Lopane, Mynosedin, Napacetin, Nobafon, Nobgen, Novogent, Novoprofen, Nurofen, Optifen, Paduden, Paxofen, Perofen, Proartinal, Prontalgin, Q-Profen, Relcofen, Remofen, Roidenin, Seclodin, Tarein, and Zofen), indomethacin (also known as Indameth, Indocin, Amuno, Antalgin, Areumatin, Argilex, Artherexin, Arthrexin, Artrinovo, Bavilon, Bonidon, Boutycin, Chrono-Indocid, Cidalgon, Confortid, Confortind, Domecid, Durametacin, Elemetacin, Idicin, Imbrilon, Inacid, Indacin, Indecin, Indocap, Indocen, Indocid, Indoflex, Indolag, Indolar, Indomed, Indomee, Indometacinum, Indometicina, Indometin, Indovis, Indox, Indozu, Indrenin, Indylon, Inflazon, Inpan, Lauzit, Liometace, Metacen, Metindon, Metocid, Mezolin, Mobilan, Novomethacin, Peralgon, Reflox, Rheumacid, Rheumacin, Salinac, Servindomet, Toshisan, and Vonum), ketoprofen (also known as Orudis, Alrheumat, Alrheumun, Alrhumat, Aneol, Arcental, Dexal, Epatec, Fastum, Keduril, Kefenid, Keprofen, Ketofen, Ketonal, Ketosolan, Kevadon, Mero, Naxal, Oruvail, Profenid, Salient, Tofen, and Treosin), ketorolac (also known as Toradol),
meclofenamate (also known as Meclofen, Meclomen, and Movens), mefenamic acid (also known as Ponstel, Alpain, Aprostal, Benostan, Bonabol, Coslan, Dysman, Dyspen, Ecopan, Lysalgo, Manic, Mefac, Mefic, Mefix, Parkemed, Pondex, Ponsfen, Ponstan, Ponstyl, Pontal, Ralgec, and Youfenam), nabumetone (also known as Relafen), naproxen (also known as Naprosyn, Anaprox, Aleve, Apranax, Apronax, Arthrisil, Artrixen, Artroxen, Bonyl, Congex, Danaprox, Diocodal, Dysmenalgit, Femex, Flanax, Flexipen, Floginax, Gibixen, Headlon, Laraflex, Laser, Leniartil, Nafasol, Naixan, Nalyxan, Napoton, Napren, Naprelan, Naprium, Naprius, Naprontag, Naprux, Napxen, Narma, Naxen, Naxid, Novonaprox, Nycopren, Patxen, Prexan, Prodexin, Rahsen, Roxen, Saritilron, Sinartrin, Sinton, Sutony, Synflex, Tohexen, Veradol, Vinsen, and Xenar), oxaprozin (also known as Daypro), piroxicam (also known as Feldene, Algidol, Antiflog, Arpyrox, Atidem, Bestocam, Butacinon, Desinflam, Dixonal, Doblexan, Dolonex, Feline, Felrox, Fuldin, Indene, Infeld, Inflamene, Lampoflex, Larapam, Medoptil, Novopirocam, Osteral, Pilox, Piraldene, Piram, Pirax, Piricam, Pirocam, Pirocaps, Piroxan, Piroxedol, Piroxim, Piton, Posidene, Pyroxy, Reucam, Rexicam, Riacen, Rosic, Sinalgico, Sotilen, Stopen, and Zunden), sulindac (also known as Clinoril, Aflodac, Algocetil, Antribid, Arthridex, Arthrocine, Biflace, Citireuma, Clisundac, Imbaral, Lindak, Lyndak, Mobilin, Reumofil, Sudac, Sulene, Sulic, Sulindal, Suloril, and Sulreuma), tolmetin (also known as Tolectin, Donison, Midocil, Reutol, and Safitex), celecoxib (also known as Celebrex), meloxicam (also known as Mobic), rofecoxib (also known as Vioxx), valdecoxib (also known as Bextra), aspirin (also known as Anacin, Ascriptin, Bayer, Bufferin, Ecotrin, and Excedrin) and steroidal anti-inflammatory drugs including cortisone, prednisone and dexamethasone.

In some embodiments, vasoconstrictors include epinephrine (adrenaline, Susphrine), phenylephrine hydrochloride (Neo-Synephrine), oxymetazoline hydrochloride (Afrin), norepinephrine (Levophed), and caffeine.

In some embodiments, vasodilators include bosentan (Tracleer), epoprostenol (Flolan), treprostinil (Remodulin), sitaxsentan, nifedipine (Adalat, Procardia), nicardipine (Cardene), verapamil (Calan, Covera-HS, Isoptin, Verelan), diltiazem (Dilacor XR, Diltia XT, Tiamate, Tiazac, Cardizem), isradipine (DynaCirc), nimodipine (Nimotop), amlodipine (Norvasc), felodipine (Plendil), nisoldipine (Sular), bepridil (Vascor), hydralazine (Apresoline), minoxidil (Loniten), isosorbide dinitrate (Dilatrate-SR, Iso-Bid, Isonate, Isorbid, Isordil, Isotrate, Sorbitrate), isorbide mononitrate (IMDUR), prazosin (Minipress), cilostazol (Pletal), treprostinil (Remodulin), cyclandelate, isoxsuprine (Vasodilan), nylidrin (Arlidin), nitrates (Deponit, Minitran, Nitro-Bid, Nitrodisc, Nitro-Dur, Nitrol, Transderm-Nitro), benazepril (Lotensin), benazepril and hydrochlorothiazide (Lotensin HCT), captopril (Capoten), captopril and hydrochlorothiazide (Capozide), enalapril (Vasotec), enalapril and hydrochlorothiazide (Vaseretic), fosinopril (Monopril), lisinopril (Prinivil, Zestril), lisinopril and hydrochlorothiazide (Prinzide, Zestoretic), moexipril (Univasc), moexipril and hydrochlorothiazide (Uniretic), perindopril (Aceon), quinapril (Accupril), quinapril and hydrochlorothiazide (Accuretic), ramipril (Altace), trandolapril (Mavik), papaverine (Cerespan, Genabid, Pavabid, Pavabid HP, Pavacels, Pavacot, Pavagen, Pavarine, Pavased, Pavatine, Pavatym, Paverolan).

In some embodiments, diuretics include acetazolamide (Diamox), dichlorphenamide (Daranide), methazolamide (Neptazane), bendroflumethiazide (Naturetin), benzthiazide (Exna), chlorothiazide (Diuril), chlorthalidone (Hygroton), hydrochlorothiazide (Esidrix, HydroDiuril, Microzide), hydroflumethiazide (Diucardin), indapamide (Lozol), methyclothiazide (Enduron), metolazone (Zaroxolyn, Mykrox), polythiazide (Renese), quinethazone (Hydromox), trichlormethiazide (Naqua), bumetanide (Bumex), ethacrynic acid (Edecrin), furosemide (Lasix), torsemide (Demadex), amiloride (Midamor), amiloride and hydrochlorothiazide (Moduretic), spironolactone (Aldactone), spironolactone and hydrochlorothiazide (Aldactazide), triamterene (Dyrenium), triamterene and hydrochlorothiazide (Dyazide, Maxzide).

In some embodiments, anti-cancer agents include aldesleukin, alemtuzumab, alitretinoin, allopurinol, altretamine, amifostine, anagrelide, anastrozole, arsenic trioxide, asparaginase, bexarotene, bicalutamide, bleomycin, busulfan, calusterone, capecitabine, carboplatin, carmustine, celecoxib, chlorambucil, cisplatin, cladribine, cyclophosphamide, cytarabine, dacarbazine, dactinomycin, darbepoetin alpha, daunorubicin, daunomycin, dexrazoxane, docetaxel, doxorubicin, epoetin alpha, estramustine, etoposide, etoposide phosphate, exemestane, filgrastim, floxuridine, fludarabine, flutamide, fulvestrant, gemcitabine, gemtuzumab ozogamicin, goserelin acetate, hydroxyurea, ibritumomab tiuxetan, idarubicin, ifosfamide, imatinib mesylate, interferon alpha-2a, interferon alpha-2b, irinotecan, leflunomide, letrozole, leucovorin, levamisole, lomustine, meclorethamine (nitrogen mustard), megestrol acetate, melphalan, mercaptopurine, mesna, methotrexate, methoxsalen, mitomycin C, mitotane, mitoxantrone, mycophenolate mofetil, nandrolone phenpropionate, nilutamide, nofetumomab, oprelvekin, oxaliplatin, paclitaxel, pamidronate, pegademase, pegaspargase, pegfilgrastim, pentostatin, pipobroman, plicamycin, porfimer sodium, procarbazine, quinacrine, rasburicase rituximab, sargramostim, streptozocin, tacrolimus, tamoxifen, temozolomide, teniposide, testolactone, thioguanine, thiotepa, topotecan, toremifene, tositumomab, trastuzumab, tretinoin, uracil mustard, valrubicin, vinblastine, vincristine, vinorelbine, and zoledronate.

In other embodiments, the wound active agent is an siRNA. The RNAi constructs of the present invention are gene(s) that express RNAs that base pair to form a dsRNA RNA region. The RNAs may be a part of the same molecule or different molecules. In preferred embodiments, the RNAi construct comprises a promoter operably linked to a nucleic acid sequence encoding two complementary sequences separated by a loop sequence. The complementary regions correspond to a target RNA sequence separated by a loop sequence. When the RNAi construct is expressed, the complementary regions of the resulting RNA molecule pair with one another to form a double stranded RNA region. The present invention is not limited to loop sequences of any particular length. In some preferred embodiments, the loop sequences range from about 4 to about 20 nucleotides in length. In more preferred embodiments, the loop sequences are from about 6 to about 12 nucleotides in length. In other preferred embodiments, the dsRNA regions are from about 19 to about 23 in length.

In other embodiments, the dsRNA is formed from RNA transcribed from a vector as two separate stands. In other embodiments, the two strands of DNA used to form the dsRNA may belong to the same or two different duplexes in which they each form with a DNA strand of at least partially complementary sequence. When the dsRNA is thus-produced, the DNA sequence to be transcribed is flanked by two promoters, one controlling the transcription of one of the strands, and the other that of the complementary strand. These two promoters may be identical or different. In some embodiments, a DNA duplex provided at each end with a promoter sequence can directly generate RNAs of defined length, and which can join in pairs to form a dsRNA. See, e.g., U.S. Pat. No. 5,795,715. RNA duplex formation may be initiated either inside or outside the cell.

It will be recognized that after processing the resulting siRNA can comprise two blunt ends, one blunt end and one end with an overhang, or two ends with overhangs. In some embodiments, the end or ends with overhangs comprise an overhang of either one or two nucleotides. As a non-limiting example, a siRNA of 23 nucleotides in length comprises two 19mers with a two nucleotide overhang at each end. As another non-limiting example, a siRNA of 21 nucleotides in length comprises two 19mers with a single nucleotide overhang at each end. As still another non-limiting example, a siRNA of 22 nucleotides in length comprises two 22mers with no overhangs at either end.

Inhibition is sequence-specific in that nucleotide sequences corresponding to the duplex region of the RNA are targeted for genetic inhibition. RNA molecules containing a nucleotide sequence identical to a portion of the target gene are preferred for inhibition. RNA sequences with insertions, deletions, and single point mutations relative to the target sequence have also been found to be effective for inhibition. Thus, sequence identity may optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript.

There is no upper limit on the length of the dsRNA that can be used. For example, the dsRNA can range from about 21 base pairs (bp) of the gene to the full length of the gene or more. In one embodiment, the dsRNA used in the methods of the present invention is about 1000 bp in length. In another embodiment, the dsRNA is about 500 bp in length. In yet another embodiment, the dsRNA is about 22 bp in length. In some preferred embodiments, the sequences that mediate RNAi are from about 21 to about 23 nucleotides. The isolated iRNAs of the present invention mediate degradation of the target RNA.

The double stranded RNA of the present invention need only be sufficiently similar to natural RNA that it has the ability to mediate RNAi for the target RNA. In one embodiment, the present invention relates to RNA molecules of varying lengths that direct cleavage of specific mRNA to which their sequence corresponds. It is not necessary that there be perfect correspondence of the sequences, but the correspondence must be sufficient to enable the RNA to direct RNAi cleavage of the target mRNA. In a particular embodiment, the RNA molecules of the present invention comprise a 3' hydroxyl group.

### III. Nanoscale polymer matrix compositions

In some embodiments, with the process of the present invention as claimed in the attached claims, microsheets are provided comprising a nanoscale polymer matrix that can be applied to a wound, a biologic tissue, a cornea, a lens, a bone, a tendon, a surgical mesh, a wound dressing, a biomedical device, a device used for healthcare, or other surface. In some embodiments, the nanoscale polymer matrix is functionalized. In some embodiments, the nanoscale polymer matrix is not functionalized. In some embodiments, the nanoscale polymer matrix comprises one or more polymers, preferably biocompatible, or is formed from one or more proteins, or is a combination of polymers and proteins. In some embodiments, the nanoscale polymer layer is formed from synthetic polymers such as synthetic polyelectrolytes. In other embodiments, the nanoscale polymer layer is formed from naturally occurring polymers such as polysaccharides. In some embodiments, the nanoscale polymer matrix is functionalized to allow for covalent interaction and/or binding to the tissue surface or the wound bed, or to allow application of - bioactive agents to the nanoscale polymer matrix. In some embodiments, a - bioactive agent, being a wound active agent, for example an antimicrobial agent such as silver, polyhexamethylene biguanide (PHMB), - chlorhexidine, or iodine compound, or an antibiotic, is incorporated into the nanoscale polymer matrix. The - bioactive agent is preferably impregnated, incorporated or interspersed throughout the three dimensional structure of the nanoscale polymer matrix. For example, if the nanoscale polymer matrix is polyelectrolyte multilayer (PEM), the - bioactive agent is preferably incorporated between or within the layers of the polymer multilayer.

In some embodiments, a polymer matrix (e.g., a nanoscale polymer matrix and/or a a sacrificial polymer layer) comprising an antibiofilm agent is manufactured. The technology is not limited in the antibiofilm agent that is used in embodiments of the device and associated method, kit, and method of treatment embodiments. For example, in some embodiments the antibiofilm agent is a small molecule antibiofilm agent, a charged small molecule antibiofilm agent, an antibiofilm polypeptide, an antibiofilm enzyme (e.g., Dispersin B), a metallic particle, or a metal ion antibiofilm agent (e.g., a metal ion, metal ion salt, or metal ion nanoparticle). Further, in some embodiments, the metal ion antibiofilm agent is a gallium ion, a gallium ion salt, a gallium ion nanoparticle, an alloy of gallium, or an alloy of gallium and silver.

In some embodiments, the nanoscale polymer matrices, such as polymer multilayers, are nanoscale to microscale in dimension. Accordingly, in some embodiments, the nanoscale polymer matrices are from about 1 nm to 1000 nm thick, from about 1 nm to 500 nm thick, from about 1 nm to 100 nm thick, from about 1 nm to about 25 nm thick, from about 1 nm to about 10 nm thick, or less than about 500 nm, 100 nm, 25 nm or 10 nm thick. It is contemplated that the nanoscale dimension of the matrices (i.e., the nanoscale thickness) allows for the loading of a lower total amount of an active agent while still allowing delivery of an effective amount (i.e., an amount of active agent that accelerates wound healing as compared to controls) of the active agent as compared to matrix structures with greater thickness. It is contemplated that the lower total loading levels result in reduced toxicity in the wound environment, especially when antimicrobial compounds are incorporated into the polymer multilayer.

In some embodiments, the compliance of the nanoscale polymer matrices, such as polymer multilayers, is adjusted to facilitate cell migration in the wound. In some embodiments, the matrices have a compliance, measured in kilopascals (kPa) of from about 100 Pa to about 500 kPa, about 7 to about 250 kPa, about 10 to about 250 kPA or from about 10 to about 200 kPa. In some embodiments, the matrices, e.g., polymer multilayers, have a compliance of from about 3 kPa to 5, 4, 3, 2, 1, 0.5, 0.1 and 0.05 GPa (gigapascals).

According to the process of the invention, the nanoscale polymer matrix is supported on a sacrificial polymer layer, formed from a degradable or dissolvable support material such as a dissolvable polymer. According to the process of the invention the nanoscale polymer matrix is first formed on a solid support and then a sacrificial polymer layer is formed on the nanoscale polymer matrix, for example, by spin coating, spraying or casting the sacrificial polymer layer material on the nanoscale polymer matrix. The sacrificial polymer layer material is dissolvable in aqueous environments or environments where moisture is present, such as moist surfaces like wound beds, internal body surfaces, epithelial surfaces and the like. In preferred embodiments, the sacrificial polymer layer material is dissolvable in aqueous solutions after application of the nanoscale polymer matrix on the surface. Thus, the present process providesa microsheet article comprising two layers, a first nanoscale polymer matrix layer adjacent to a sacrificial polymer layer. The sacrificial polymer layer dissolves when applied to a moist surface so that the nanoscale polymer matrix is left in contact with the moist surface. The nanoscale polymer matrix can be transferred to a wound or tissue using a sacrificial polymer layer that lies on top of nanoscale polymer matrix after transfer to the wound or tissue, or the nanoscale polymer matrix is transferred to a wound or tissue using a sacrificial polymer layer that lies underneath the nanoscale polymer matrix after transfer to the wound or tissue. A nanoscale polymer matrix made with PEMs according to a process of the invention and a dissolvable sacrificial cast can be transferred to a wound or tissue surface such that sacrificial cast dissolves completely in wound and PEMs are in direct contact with the wound tissue and a primary/secondary wound can be placed over the wound.

The nanoscale polymer matrix containing one or more bioactive agents can be transferred to a wound or tissue using a sacrificial polymer layer that lies on top of nanoscale polymer matrix after transfer to the wound or tissue. A wound dressing can be placed on top of the sacrificial polymer layer before or after the sacrificial polymer layer is dissolved.

In some embodiments, the nanoscale polymer matrix is a polyelectrolyte multilayer (PEM).

In some embodiments, the sacrificial polymer layer of a microsheet contains bioactive agents, antimicrobial agents, antibiofilm agents, microparticles, nanoparticles, magnetic particles. In some embodiments, microparticles or nanoparticles in the sacrificial polymer layer contain bioactive agents or antimicrobial agents.

In some embodiments, the sacrificial layer comprises an antibiofilm agent. The technology is not limited in the antibiofilm agent that is used in embodiments of the device and associated method, kit, and method of treatment embodiments. For example, in some embodiments the antibiofilm agent is a small molecule antibiofilm agent, a charged small molecule antibiofilm agent, an antibiofilm polypeptide, an antibiofilm enzyme (e.g., Dispersin B), a metallic particle, or a metal ion antibiofilm agent (e.g., a metal ion, metal ion salt, or metal ion nanoparticle). Further, in some embodiments, the metal ion antibiofilm agent is a gallium ion, a gallium ion salt, a gallium ion nanoparticle, an alloy of gallium, or an alloy of gallium and silver.

The sacrificial polymer layer of a microsheet is water soluble. In some embodiments, the sacrificial polymer layer is made of non-toxic polymer, and in some embodiments the sacrificial polymer layer is poly vinyl alcohol (PVA). In some embodiments the sacrificial polymer layer is made of polyacrylic acid (PAA).

### A. Polymer matrix materials

In some embodiments of the process of the invention, the matrix is a polymer multilayer. In some embodiments, the multilayer structures comprise layers of polyelectrolytes (i.e., forming a polyelectrolyte multilayer), while in other embodiments, the multilayers comprise polymers that do not have a charge (i.e., non-ionic polymers) or a combination of charged and uncharged polymer layers. In some embodiments, it is contemplated that polyelectrolyte films built-up by the alternated adsorption of cationic and anionic polyelectrolyte layers constitute a novel and promising technique to modify wound surfaces in a controlled way (Decher et al., 1992, Thin Solid Films 210/211:831; Decher, 1997, Science 277:1232). One of the most important properties of such multilayers is that they exhibit an excess of alternatively positive and negative charges (Caruso et al., 1999, J Am Chem Soc 121:6039; Ladam et al., 2000, Langmuir 16:1249). Not only can this constitute the motor of their buildup (Joanny, 1999, Eur. Phys. J. Biol. 9:117), but it allows, by simple contact, to adsorb a great variety of compounds such as dyes, particles(Cassagneau et al., 1998, J. Am. Chem. Soc. 120:7848; Caruso et al., 1999, Langmuir 15:8276; Lvov et al., 1997, Langmuir 13:6195), clay microplates (Ariga et al., 1999, Appl. Clay Sci. 15:137) and proteins (Keller et al., 1994, J. Am. Chem. Soc. 116:8817; Lvov et al., 1995, J. Am. Chem. Soc. 117:6117; Caruso et al., 1997, Langmuir 13:3427).

Polyelectrolyte layers are formed by alternating applications of anionic polyelectrolytes and cationic polyelectrolytes to surfaces to form a polyelectrolyte multilayer. In some embodiments, one or more wound active agents, such as those described above, are incorporated into the multilayer. Preferably, at least four layers, and, more preferably, at least six layers are used to form the polyelectrolyte multilayer.

Cationic polyelectrolytes useful in the present invention can be any biocompatible water-soluble polycationic polymer, for example, any polymer having protonated heterocycles attached as pendant groups. As used herein, "water soluble" means that the entire polymer must be soluble in aqueous solutions, such as buffered saline or buffered saline with small amounts of added organic solvents as co-solvents, at a temperature between 20 and 37° Centigrade. In some embodiments, the material will not be sufficiently soluble (defined herein as soluble to the extent of at least one gram per liter) in aqueous solutions per se but can be brought into solution by grafting the polycationic polymer with water-soluble polynonionic materials such as polyethylene glycol.

Representative cationic polyelectrolytes include natural and unnatural polyamino acids having net positive charge at neutral pH, positively charged polysaccharides, and positively charged synthetic polymers. Examples of suitable polycationic materials include polyamines having amine groups on either the polymer backbone or the polymer side chains, such as poly-L-lysine (PLL) and other positively charged polyamino acids of natural or synthetic amino acids or mixtures of amino acids, including, but not limited to, poly(D-lysine), poly(ornithine), poly(arginine), and poly(histidine), and nonpeptide polyamines such as poly(aminostyrene), poly(aminoacrylate), poly (N-methyl aminoacrylate), poly (N-ethylaminoacrylate), poly(N,N-dimethyl aminoacrylate), poly(N,N-diethylaminoacrylate), poly(aminomethacrylate), poly(N-methyl amino-methacrylate), poly(N-ethyl amino methacrylate), poly(N,N-dimethyl amino methacrylate), poly(N,N-diethyl amino methacrylate), poly(ethyleneimine), polymers of quaternary amines, such as poly(N,N,N-trimethylaminoacrylate chloride), poly(methyacrylamidopropyltrimethyl ammonium chloride), and natural or synthetic polysaccharides such as chitosan.

In general, the polymers must include at least five charges, and the molecular weight of the polycationic material must be sufficient to yield the desired degree of binding to a tissue or other surface, having a molecular weight of at least 1000 g/mole.

Polyanionic materials useful in the present invention can be any biocompatible water-soluble polyanionic polymer, for example, any polymer having carboxylic acid groups attached as pendant groups. Suitable materials include alginate, carrageenan, furcellaran, pectin, xanthan, hyaluronic acid, heparin, heparan sulfate, chondroitin sulfate, polyacrylic acid (PAA), dermatan sulfate, dextran sulfate, poly(meth)acrylic acid, oxidized cellulose, carboxymethyl cellulose and crosmarmelose, synthetic polymers and copolymers containing pendant carboxyl groups, such as those containing maleic acid or fumaric acid in the backbone. Polyaminoacids of predominantly negative charge are also suitable. Examples of these materials include polyaspartic acid, polyglutamic acid, and copolymers thereof with other natural and unnatural amino acids. Polyphenolic materials such as tannins and lignins can be used if they are sufficiently biocompatible. Preferred materials include alginate, pectin, carboxymethyl cellulose, heparin and hyaluronic acid.

In some embodiments, the cationic polyelectrolyte used is PLL and the anionic polyelectrolyte used is poly(L-glutamic acid) (PGA). Indeed, the use of a variety of polyelectrolytes is contemplated, including, but not limited to, poly(ethylene imine) (PEI), poly(allylamine hydrochloride) (PAH), poly(sodium 4-styrenesulfonate) (PSS), poly(acrylic acid) (PAC), poly(maleic acid-co-propylene) (PMA-P), and poly(vinyl sulfate) (PVS). It is also possible to use naturally occurring polyelectrolytes, including hyaluronic acid and chondroitin sulfate. In still further embodiments, the polymer is a dendrimer, grafted polymer, or star architecture polymer.

In some embodiments, the multilayer structures are formed from uncharged polymers or from a combination of charged and uncharged polymers. Examples of uncharged polymers include, but are not limited to, dextran, dextran sulfate, diethylaminoethyl (DEAE)-dextran, hydroxyethyl cellulose, ethyl(hydroxyethyl) cellulose, acrylamide, polyethylene oxide, polypropylene oxide, polyethylene oxide - polypropylene oxide copolymers, PAANₐ, Ficoll, polyvinylpyrolidine, and polyacrylic acid.

In some embodiments, the multilayer structures are formed from one or more amphoteric polymers, alone in combination with the other polymers described herein. In some embodiments, the amphoteric polymers comprise one or more of acrylic acid (AA), DMAEMA (dimethylaminoethyl methacrylate), APA (2-aminopropyl acrylate), MorphEMA (morpholinoethyl methacrylate), DEAEMA (diethylaminoethyl methacrylate), t-ButylAEMA (t-butylaminoethyl methacrylate), PipEMA (piperidinoethyl methacrylate), AEMA (aminoethyl methacrylate), HEMA (2-hydroxyethyl methacrylate), MA (methyl acrylate), MAA (methacrylic acid) APMA (2-aminopropyl methacrylate), AEA (aminoethyl acrylate). In some embodiments, the amphoteric polymer comprises (a) carboxylic acid, (b) primary amine, and (c) secondary and/or tertiary amine. The amphoteric polymers have an isoelectric point of 4 to 8, preferably 5 to 7 and have a number average molecular weight in the range of 10,000 to 150,000.

Polymer layers may be formed by a variety of methods.The polymer layers are formed on solid supports as described in detail below. In some embodiments, it is contemplated that the polymer or polymer multilayer is formed by sequential application of polymers using either a pump (including syringes, ink jet printers, and electrojets) or spray, such as an aerosol spray, or by dip coating. In other embodiments, particle bombardment is utilized. In other embodiments, the use of a brush including an air brush is contemplated. In an embodiment of the process of the invention , the nanoscale polymer matrix is formed on a solid support, a sacrificial polymer layer is formed on the nanoscale polymer matrix, and the resulting microsheet is peeled from the solid support.

In some embodiments, the matrix comprises one or more proteins. In preferred embodiments, the proteins form a hydrogel. In some preferred embodiments, the matrix comprises one or more extracellular matrix proteins. In some embodiments, the matrix comprises at least one of collagen, laminin, vitronectin, fibronectin, keratin, and combinations thereof. As described above, the protein matrix may preferably be formed by a variety of methods. In some embodiments, the protein matrix is formed on solid supports as described in detail below. In some embodiments, it is contemplated that the protein matrix is formed by application of proteins or solutions or gels of proteins using either a pump (including syringes, ink jet printers, and electrojets) or spray, such as an aerosol spray. In other embodiments, the use of a brush including an air brush is contemplated. In other embodiments, a sponge is utilized. In other embodiments a solid support or stamp such as an elastomeric material, for example, PDMS (polydimethylsiloxane), silicone, hydrogel or latex, is used to support the protein matrix and mechanically transfer the protein matrix into or onto the wound bed.

In some embodiments, the matrix is further modified to include cells, including, but not limited to, stem cells, keratinocytes, 3-D skin constructs, corneal epithelial cells, conjunctival cells, corneal limbal stem cell, human embryonic stem cells, pluripotential stem cells, adult induced stem cells, hematopoietic stem cells, hepatocytes, pancreatic cells and the like. In some embodiments, the compositions and methods described herein are used to functionalize harvested and processed (frozen, freeze dried, fixed and stored dry or wet, fresh tissue for direct transplant) animal and human tissue (pig/human aortic valve, harvested human amniotic membrane, human cadaver skin, harvested sclera and cornea, harvested cadaver bone, harvested blood vessels and the like. In some embodiments, the compositions and methods described herein are used to functionalize skin constructs, including, but not limited to, autograft skin (i.e., skin is harvested from a patient, functionalized as described herein, and returned to the patient), organotypically cultured human skin equivalents, and other keratinocyte products such as Dermagraft™.

### C. Microsheets comprising a nanoscale polymer matrix and a sacrificial polymer layer

As detailed above, the present invention provides a process for manufacturing microsheets comprising at least two layers, the first layer being a nanoscale polymer matrix and the second layer being a sacrificial polymer layer. Microsheets and methods for making the microsheets are provided in Figures 13, 16 and 17. Referring to Figure 13, a nanoscale polymer matrix is formed on a solid support such as PDMS. The nanoscale polymer matrix may comprise any of the polymers described in detail above. Additional support materials are described in more detail below. In some embodiments, a wound active agent is incorporated into the nanoscale polymer matrix during formation of the matrix. It can also be impregnated into the matrix following formation of the matrix to provide a bioactive nanoscale polymer matrix. As described above, the wound active agent is preferably incorporated or interspersed within the three dimensional structure of the matrix. Figure 16 shows a depiction wherein silver ions and nanoparticles are interspersed within the three dimensional structure of a PAH/PAA nanoscale polyelectrolyte multilayer (PEM) matrix. In preferred embodiments, the nanoscale polymer matrix is impregnated with silver nitrate solution to impregnate silver ions; the matrix is then incubated in a solution of a reducing agent to form silver nanoparticles within the matrix. Figure 17 shows a depiction wherein chlorhexidine, a positively charged antimicrobial agent, is interspersed between negatively charged polyelectrolyte (PAA) layers in a PAH/PAA nanoscale polyelectrolyte multilayer (PEM) matrix.

Referring again to Figure 13, in some embodiments, a sacrificial polymer layer is then cast on the nanoscale polymer matrix. The sacrificial polymer layer preferably comprises a degradable or dissolvable material as described above. In some preferred embodiments, the sacrificial polymer layer comprises polyvinyl alcohol (PVA). In some embodiments the sacrificial polymer layer is made of polyacrylic acid (PAA). In some embodiments, the sacrificial polymer layer is preferably cast by dip coating, spin coating or spraying. It is contemplated that dip coating, spin coating or spraying provides a sacrificial polymer layer of uniform thickness. In some preferred embodiments, the microsheet is from about 0.2 cm² to about 600 cm². In some embodiments, the sacrificial polymer layer has a thickness variation across the entire microsheet of less than 500, 400, 300, 200, 100, 50, 20 or 10% of the average thickness when measured in cross section. Still referring to Figure 13, the microsheet may be peeled from the solid support to provide a free standing microsheet which can be applied to a surface such as a device or wound tissue as depicted in Figures 13 and 14, respectively. The microsheets of the present invention have excellent mechanical strength even though they are microscopically thin and can be handled by hand or by machines. In some embodiments, the microsheets have a Young modulus of from about 0.1 GPa to about 10 GPa. As can be seen from Figures 13 and 14, the exposed nanoscale polymer matrix is brought into direct contact with the surface of the device or a wound surface. In the presence of moisture, the sacrificial polymer layer dissolves leaving the nanoscale polymer matrix displayed on or adhered to the surface.

### D. Functionalization agents

In some embodiments, the matrices described above are functionalized. In preferred embodiments, the matrices are functionalized with one or more covalent modification agents. In some preferred embodiments, the crosslinkers comprise either an azide group or an alkyne group so that suitable click chemistries can be utilized. In some embodiments, the at least one covalent modification agent is a homobifunctional cross-linker. In other embodiments, the at least one covalent modification agent is a heterobifunctional cross-linker. For example, in some embodiments, the homobifunctional cross-linker is an N-hydroxysuccinimidyl ester (e.g., including, but not limited to, disuccinimidyl ester, dithiobis(succinimidylpropionate), 3,3'-dithiobis(sulfosuccinimidylpropionate), disuccinimidyl suberate, bis(sulfosuccinimidyl)suberate, disuccinimidyl tartarate, disulfosuccinimidyl tartarate, bis[2-(succinimidyloxycarbonyloxy) ethyl]sulfone, bis[2-(sulfosuccinimidooxycarbonyloxy) ethyl]sulfone, ethylene glycolbis(succinimidyl-succinate), ethylene glycolbis(sulfosuccinimidylsuccinate), disuccinimidyl glutarate, and N,N'-disuccinimidylcarbonate). In some embodiments, the homobifunctional cross-linker is at a concentration between 1 nanomolar and 10 millimolar. In some preferred embodiments, the homobifunctional cross-linker is at a concentration between 10 micromolar and 1 millimolar. In other embodiments, the at least one covalent modification agent is a heterobifunctional cross-linker (e.g., including, but not limited to, N-succinimidyl 3-(2-pyridyldithio)propionate, succinimidyl 6-(3-[2-pyridyldithio]-propionamido)hexanoate, sulfosuccinimidyl 6-(3'-[2-pyridyldithio]-propionamido)hexanoate, succinimidyloxycarbonyl-α-methyl-α-(2-pyridyldithio)toluene, sulfosuccinimidyl-6-[α-methyl-α-(2-pyridyldithio) toluamido]hexanoate, succinimidyl 4-(N-maleimidomethyl)cyclohexane- 1-carboxylate, sulfosuccinimidyl 4-(N-maleimidomethyl)cyclohexane- 1-carboxylate, m-maleimidobenzoyl-N-hydroxysuccinimide ester, m-maleimidobenzoyl-N-hydroxy-sulfosuccinimide ester, N-succinimidyl(4-iodoacetyl)aminobenzoate, sulfo-succinimidyl(4-iodoacetyl)aminobenzoate, succinimidyl-4-(p-maleimidophenyl)butyrate, sulfosuccinimidyl-4-(p-maleimidophenyl)butyrate, N-(y-maleimidobutyryloxy)succinimide ester, N-(γ-maleimidobutyryloxy)sulfosuccinimide ester, succinimidyl 6-((iodoacetyl)amino)hexanoate, succinimidyl 6-(6-(((4-iodoacetyl)amino)hexanoyl)amino)hexanoate, succinimidyl 4-(((iodoacetyl)amino)methyl) cyclohexane-1-carboxylate, succinimidyl 6-((((4-iodoacetyl)amino)methyl)cyclohexane-1-carbonyl)amino)-hexanoate, and p-nitrophenyl iodoacetate). In some embodiments, the heterobifunctional cross-linker is modified with functional groups, rendering it soluble in aqueous solvents for delivery as an aqueous solution. Furthermore, in some embodiments, the aqueous solution contains additives (e.g., including, but not limited to, surfactants and block copolymers). In other embodiments, a multiplicity of hetero-bifunctional cross-linkers can be attached to a molecule, polymer or particle to serve as the cross-linking agent. In other embodiments, the heterobifunctional cross-linker is dissolved in an organic solvent (e.g., including, but not limited to, dimethyl sulfoxide).

In some embodiments, the covalent modifier is a photoactivatable crosslinker. Suitable photoactivatable crosslinkers include, but are not limited to, aryl azide N-((2-pyridyldithio)ethyl)-4-azidosalicylamide, 4-azido-2,3,5,6-tetrafluorobenzoic acid, succinimidyl ester, 4-azido-2,3,5,6-tetrafluorobenzoic acid, STP ester, benzophenone maleimide, succinimidyl ester of 4-benzoylbenzoic acid, N-5-azido-2-Nitrobenzoyloxysuccinimide, N-Hydroxysulfo succinimidyl-4- azidobenzo ate, N-hydroxysuccinimidyl-4-azidosalicylic acid, and (4-[p-azidosalicylamido]butylamine).

The covalent modification agent may be applied to the matrix by any suitable method. . In some embodiments, it is contemplated that the covalent modification agent is applied using either a pump (including syringes, ink jet printers, and electrojets) or spray, such as an aerosol spray. In other embodiments, the use of a brush including an air brush is contemplated. In other embodiments, a sponge is utilized.

Following application of the covalent modification agent to the matrix, a functionalized matrix is formed. In some embodiments, the functionalized matrix displays one or more reactive moieties, for example, azide moieties, succinimidyl moieties, alkyne moieties or any of the other reactive groups of the compounds described above.

In some embodiments, the matrix is modified by PEGylation with polyethylene glycol. It is contemplated that PEGylation can be used to control interaction of the matrix with the wound bed and to prevent nonspecific adsorption as desired. PEGylation can also be used to control delivery of the wound active agent from the matrix.

### E. Wound active agents

The matrices comprise one or more wound active agents as described in detail above. In some embodiments, the wound active agent or agents are noncovalently incorporated into the matrix. In some preferred embodiments, silver containing antimicrobials are incorporated into the functionalized matrix, for example, a polyelectrolyte multilayer as described above. In some embodiments, the wound active agent or agents are covalently immobilized on the matrix, for example, via a covalent modification agent.

In some embodiments, the one or more wound active agents are applied to form a gradient with respect to the wound modifying agent. In general, the gradients present a higher contraction of wound active agent at one or more first desired locations in the wound following application of the wound modifying agent to the wound and a lower concentration of wound active agent at one or second location in the wound following application of the matrix to the wound. For example, the concentrations of the wound active agents are layered in a wound bed in a gradient such that higher concentrations of a particular composition is greater proximal to the wound bed than distal to the wound bed in a vertical fashion. The converse, where concentrations of compositions is greater distal to the wound bed than proximal, is also contemplated. Concentration of compositions in a wound bed wherein a horizontal gradient is deposited is also contemplated. Topographical gradients are also contemplated, wherein compositions are deposited such that the concentrations of compositions in a wound bed or on a biocompatible particle follow the topography of the substrate, for example, a higher concentration of compositions is deposited in the valleys of undulations of an exemplary substrate compared to the peaks of the undulations.

In some embodiments, the gradient comprises a higher concentration of the wound active agent in the center of the matrix which transitions to a lower concentration of the wound active agent away from the center of the matrix. Accordingly, when the matrix is applied to a wound, the gradient results in a higher concentration of wound active agent in the center of the wound and a lower concentration of wound active agent as one moves to the periphery of the wound. In some embodiments, the gradient comprises a lower concentration of the wound active agent in the center of the matrix which transitions to a higher concentration of the wound active agent away from the center of the matrix. Accordingly, the gradient results in a lower concentration of wound active agent in the center of the wound and a higher concentration of wound active agent as one moves to the periphery of the wound. If two or more wound active agents are utilized, they can be presented as similar gradients or the gradients can be varied so that the concentrations of the two or more wound active agents vary across the wound. The gradients of high or low concentration can be any shape, such as circular, square, rectangular, oval, oblong, etc. so that the matrix and gradient can conform to a variety of wound shapes. For example, for long, incision type wound, the gradient may be centered on a longitudinal axis that extends along the length of the wound and can be centered on the wound. As another example, the gradient can be circular or oval-shaped for application to open type wounds, burns, sores and ulcers that are roughly circular or oval. In other embodiments, the gradients comprise a series of features arranged in a pattern. For example, the gradients can form a series of stripes or high and low concentrations of one or more wound active agents along a longitudinal axis of the matrix. Alternatively, the gradients can form a checkerboard pattern, array, concentric circles, overlapping circles or oval, etc.

### F. Support materials

The matrices are formed on a support material. Suitable support materials include, but not limited to, solid supports formed from polymeric materials, elastomeric materials such PDMS, nylon, Teflon^{®}, Gortex^{®}, silk, polyurethane, and silicon, preferably medical grade silicon, PVDF, polyethylene oxide, and water soluble materials and polymers such as polyvinyl alcohol. In some embodiments, the polymer(s) used to form the polymer multilayer are different than the polymer(s) forming the support. In some embodiments, an additional layer of material, such as a water soluble material or polymer, is disposed between the polymer multilayer and the support. In some embodiments, the solid support is a wound dressing or biologic wound dressing that is compatible with functionalization by addition of a matrix material. Examples of commercially available wound dressings that can be modified by addition of a matrix as described below include, but are not limited to, Biobrane™, gauze, adhesive tape, bandages such as Band-Aids^{®}, and other commercially available wound dressings including but not limited to COMPEEL^{®}, DUODERM™, TAGADERM™, and OPSITE^{®}. In some embodiments, the support is a silicon wafer, surfaces, such as glass or silicon wafers, that are coated with hydrophobic self assembled monolayer, fabrics, such as nylon fabrics, absorbent material, foam, a transparent thin film, a hydrogel, hydro fibers, hydrocolloids, alginate fibers, silica gel, sodium polyacrylate, potassium polyacrylamides and combinations thereof. In some embodiments, the supports comprise (e.g., is coated with or displays) a biological molecule selected from the group consisting of collagen, hyaluronic acid, glycosaminoglycans, keratin, fibronectin, vitronectin, laminin, and combinations or fragments thereof.

There are several different methods that can be used to deposit polymer multilayers on a support. In some embodiments, the polymer multilayers are deposited onto a support by adsorption from solution, spraying, or spin coating. As described above, the matrices are preferably polymer multilayers, for example, polyelectrolyte multilayers. In some embodiments, the matrices preferably comprise one more wound active agents as described above and/or or nanoscale or microscale beads as described above. In some preferred embodiments, the wound active agent is one or more of silver nanoparticles, elemental silver, and silver containing compounds such as silver sulfadiazine and related compounds, preferably included in the concentration ranges described above.

The microsheets as manufactured by the process of the present invention can be applied to all types of wounds. Furthermore, a wound modifying agent with one or more wound active agents can be applied to skin, mucous membranes, body cavities, and to internal surfaces of bones, tissues, etc. that have been damaged. A wound modifying agent with one or more wound active agents can be used on wounds such as cuts, abrasions, ulcers, surgical incision sites, burns, and to treat other types of tissue damage. The microsheets as manufactured by the process of the present invention described above enhance wound healing.

As discussed herein and supported by the experimental examples, embodiments of the technology provide for the modification of wound-beds using microfilms comprising PEMs loaded with silver-nanoparticles and a dissolvable layer of PVA. Following dissolution of the PVA, the conformal contact of the PEMs to the wound-bed provides for the localized delivery of silver to wounds (see, e.g., Figure 49). Conventional silver dressings rest on top of a wound and have to release large concentrations of silver ions to diffuse through the wound exudate and kill microbes on the wound bed (see, e.g., Figure 49A). Most of the silver ions released are inactivated in the wound exudate. In contrast, microfilm silver dressings conform around the micro-contours of wound, providing intimate contact of active silver ions with wound bed, requiring up to 100× less silver than conventional dressings to kill microbes on wound-bed (see, e.g., Figure 49B). As such, microfilm dressings reduce loss of silver ions in wound exudate, reducing silver cytotoxicity and tissue staining.

This improved technology reduced the loss of silver in the wound exudate and thus minimized the need for high silver loadings within the nanofilms (A. Agarwal, T. L. Weis, M. J. Schurr, N. G. Faith, C. J. Czuprynski, J. F. McAnulty, C. J. Murphy, N. L. Abbott, Biomaterials 2010, 31, 680). Experiments conducted during the development of embodiments of the technology indicate that PEMs releasing up to 100 times less silver than conventional silver dressings (e.g., Acticoat® dressings with nanocrystalline silver release -100 µg cm⁻² per day) (P. L. Taylor, a L. Ussher, R. E. Burrell, Biomaterials 2005, 26, 7221) effectively reduced microbial colonization in contaminated murine wounds. Whereas conventional silver dressings (including Acticoat®) significantly inhibit re-epithelialization of excisional splinted wounds in mice, (A. Burd, C. H. Kwok, S. C. Hung, H. S. Chan, H. Gu, W. K. Lam, L. Huang, Wound Repair. Regen. 2007, 15, 94) embodiments of the technology described herein comprising silver did not inhibit wound re-epithelialization in a similar murine wound-model and did not cause excessive inflammation. Accordingly, the PEMs/PVA microfilms find use, e.g., as a proactive application over clean surgical wounds to prevent microbial infections. In contrast, conventional silver dressings are not recommended for use until a 'critical colonization' is established (M. H. Hermans, Am. J. Nurs. 2006, 106, 69)*.*

The PEM/PVA microfilm constructs used herein for wound microbial management possess a number of improved and/or novel attributes that are useful in clinical contexts. (1) Embodiments of the microfilm constructs are mechanically strong and translucent, allowing clinicians to handle the films and to see through them, thus providing for easy placement on a wound-bed. In contrast, conventional silver-dressings are opaque and are typically removed to examine wounds. Accordingly, embodiments of the PEM/PVA microfilms described herein reduce patient pain, nursing time, and costs associated with dressing changes. (2) As described earlier, conventional silver dressings are designed to release large concentrations of silver to replenish silver ions lost to proteins in wound fluid (M. Trop, M. Novak, S. Rodl, B. Hellbom, W. Kroell, W. Goessler, J. Trauma 2006, 60, 648; E. K. Mooney, C. Lippitt, J. Friedman, Plast. Reconstr. Surg. 2006, 117, 666). This results in excessive accumulation of silver aggregates in the epithelium, causing tissue staining and irritation (B. S. Atiyeh, M. Costagliola, S. N. Hayek, S. A. Dibo, Burns 2007, 33, 139), and impairing re-epithelialization (A. Burd, C. H. Kwok, S. C. Hung, H. S. Chan, H. Gu, W. K. Lam, L. Huang, Wound Repair. Regen. 2007, 15, 94). In contrast, the low rates of release of silver from embodiments of the polymer nanofilms described herein significantly reduce silver accumulation in the wound, reducing the potential for tissue staining and irritation. (3) As demonstrated previously, PEMs can be impregnated with an array of bioactive or antimicrobial agents (T. Boudou, T. Crouzier, K. Ren, G. Blin, C. Picart, Adv. Mater. 2010, 22, 441; J. a. Lichter, K. J. Van Vliet, M. F. Rubner, Macromolecules 2009, 42, 8573). The embodiments of the technology provided herein are practical for use in a clinical environment with no loss of efficacy of the nanofilm component of the construct. Accordingly, the approach is generalizable, thus providing embodiments applicable to a heterogeneous variety of wounds and, in some embodiments, to simultaneous release of multiple bioactive agents in wound-beds to support different phases of wound healing. The technology contemplates the modification of wound-beds with PEMs comprising multiple bioactive agents.

The technology provides significant improvements over conventional technologies in several aspects. For example, some conventional technologies use sacrificial polymer layers for the transfer of polymer nanofilms to wounds (R. Vendamme, S.-Y. Onoue, A. Nakao, T. Kunitake, Nat. Mater. 2006, 5, 494; W. Cheng, M. J. Campolongo, S. J. Tan, D. Luo, Nano Today 2009, 4, 482). In particular, some conventional technologies use PEMs comprised of chitosan and sodium alginate, and the transfer the PEMs to wounds. In some conventional approaches, an antibiotic is included to manage microbial burden. In particular, conventional technologies use a processes of fabrication comprising (i) formation of a PEM on a substrate and coating of the PEM with a film of PVA; (ii) mechanical removal of the PEM/PVA, inversion of the construct, and replacement attachment of the PEM/PVA constructs onto a substrate with the PEM surface exposed, (iii) deposition of antibiotic (tetracycline, TC) from acetone onto the surface of the PEM and encapsulation of the deposited layer of acetone by a layer of hydrophobic polyvinylacetate. Release of the TC from the construct occurred over 6 hours in a manner that was dependent on the thickness of the polyvinylacetate layer (layers of polyvinylacetate thicker than 200 nm were reported to delaminate during experiments).

In contrast, embodiments of the techonology provided herein provide a fabrication procedure to prepare PEMs capable of delivering bioactive agents that is novel and provides significant improvement over conventional technologies (T. Fujie, A. Saito, M. Kinoshita, H. Miyazaki, S. Ohtsubo, D. Saitoh, S. Takeoka, Biomaterials 2010, 31, 6269; A. Saito, H. Miyazaki, T. Fujie, S. Ohtsubo, M. Kinoshita, D. Saitoh, S. Takeoka, Acta Biomater. 2012, 8, 2932). Specifically, the embodiments of the procedure occur on a single surface, and do not require mechanical removal and replacement of the construct during fabrication. In addition, embodiments of the technology provide for the controlled release of bioactive agents over 30 days, a period of time that encompasses the time typically involved in wound healing. In some embodiments, advantages are provided in the use of broad spectrum antimicrobial agents (e.g., silver) for routine management of microbial burden rather than antibiotics such as TC due to the increasing concerns of antibiotic-resistant bacteria to specific antibiotics. In some embodiments, the silver-loaded PEMs provided herein are more effective in reducing bacterial colonization both *in vitro* (bacterial reduction of 5.8 and 2.7 log₁₀ CFU for silver and TC, respectively) and *in vivo* (bacterial reduction of 2.4 and 1 log₁₀ CFU for silver and TC, respectively) compared to conventional PEMs.

Experiments conducted during the development of embodiments of the technology described herein indicated the efficacy of antimicrobial silver nanofilms in contaminated wounds under Biobrane® dressing. In the experiments, Biobrane was used as an example of a biosynthetic dressing with a high incidence of wound infection rates in patients (K. Nichols, Z. Moaveni, A. Alkadhi, W. Mcewan, ANZ J. Surg. 2009, 79, A7). Because Biobrane exemplifies a primary dressing with high infection rates, the data collected indicate that wound treatment with silver nanofilms is broadly useful for management of microbial burden in combination with a range of moist wound dressings such as silicon films, collagen scaffolds, hydrogel sheets, hydrofibers, and hydrocolloids. The technology contemplates the use of silver nanofilms in combination with these primary wound dressings to reduce microbial burden in contaminated wounds to provide, e.g., a reduction of microbial colonization in contaminated wounds to expedite wound closure as compared to unmodified wounds.

### EXPERIMENTAL

The examples below serve to further illustrate the invention.

### Refercence Example 13. Nanometer-thick silver-impregnated polymeric films showing selective toxicity towards bacterial growth and that support mammalian cell growth.

Polyelectrolyte multilayers electrostatically assembled alternately from such weak polyacids as poly(allylamine hydrochloride) (PAH) and poly(acrylic acid) (PAA) were tuned to impregnate post-assembly with silver nanoparticles to levels where they kill bacteria effectively yet allow adhesion of mammalian cells on the films without measurable cytotoxicity. Silver ions (Ag⁺) were incorporated in the films by incubating them with bulk solution of a silver salt (e.g., silver nitrate). While the present invention is not limited to any particular mechanism, and an understanding of the mechanism is not necessary to practice the present invention, it is contemplated that Ag⁺ ions bound to the free carboxylic acid groups of PAA in the films by ion exchange with the acidic protons. Post binding, Ag⁺ in the films were reduced to zerovalent Ag nanoparticles by using an aqueous solution of a reducing agent. By changing the pH of the polyelectrolyte assembly solutions, the number of nonionized carboxylic acid groups (and hence the amount of silver incorporated) in the PEMs post-assembly was effectively controlled. These silver-impregnated nanometer-thick films were then investigated for their interactions with NIH-3T3 cells, a highly adhesive murine fibroblast cell line, and *Staphylococcus epidermidis,* a gram-positive bacterium that occurs frequently on skin and in mucous membranes.

Silver loading in the PEMs could be varied from -24.2 µg/cm² to 0.4 µg/cm². Cytotoxicity to NIH-3T3 cells seeded on these films was correlated with the concentration of soluble silver in the PEMs, with augmented toxicity at higher silver concentrations, as shown in Figure 8. PEMs prepared with relatively more ionized PAA entrapped lower amounts of silver and demonstrated reduced cytotoxicity. Further, their strongly ionically crosslinked architecture allowed better attachment and spreading of fibroblasts than PEMs prepared using weakly ionized PAA, as shown in Figure 10. Bacteria killing efficiency of PEMs correlated to the amount of silver entrapped in the films (Figure 9), with PEMs containing as little as -0.4 µg/cm² of silver providing up to 99.9999% kill in a water-borne assay. Furthermore, PEMs with this silver-loading did not exhibit measurable cytotoxicity to NIH-3T3 cells.

### Reference Example 14. Transfer to a soft surface with microscale beads.

Introducing rigid microspheres in the polymer multilayers facilitates their transfer onto the dermis layer of a commercially available human skin graft- GammaGraft®. A PAH(PAA/PAH)₁₀ multilayer, with fluorescent PAH and a PAH(PAA/PAH)₅(PS µ-spheres)(PAH/PAA)₅PAH multilayer, with fluorescent PS microspheres were stamped onto skin grafts. The portion of the multilayers that adhered to the grafts were accessed by fluorescence microscopy and imaging. The results (Figure 12) showed that the polymer multilayers with microscale beads showed much better transfer to the skin graft than did the polymer multilayer without microscale beads.

### Example 15. Synthesis and use of nanoscale polymer matrices with an accompanying sacrificial polymer layer (microsheet).

As illustrated in Figure 13, polymer matrices are assembled as polymer multilayer nanofilms over polydimethylsiloxane (PDMS) sheets; post-assembly, a sacrificial polymer layer of water-soluble polymer polyvinylalcohol (PVA) is casted (by spin coating) over the nanofilms; microsheets are peeled-off the PDMS sheets; sacrificial cast of microsheets dissolves quickly in drops of water when placed over a device surface; polymer nanofilm is immobilized on the device surface. Polymer nanofilms synthesized in this manner and loaded with silver nanoparticles or chlorhexidine were tested in-vitro and in an in-vivo wound healing model.

### Materials and methods

### Synthesis of PEMs with Silver-Nanoparticles:

Polyelectrolyte multilayers (PEMs) of the oppositely charged polyelectrolytes poly(allylaminehydrochloride) (PAH) (MW 70 kDa; Sigma Aldrich St. Louis, MO) and poly(acrylic acid) (PAA) (MW 60 kDa; Polysciences, Warrington, PA) were assembled on elastomeric poly(dimethylsiloxane) (PDMS) (Dow Chemical, Midland, MI) sheets, or on plasma-cleaned silicon wafers, by 'layer-by-layer' deposition as described in detail elsewhere. Briefly, PEMs with the desired number of multilayers were assembled on PDMS sheets using a StratoSequence Robot (nanoStrata Inc, Tallahassee, FL), by sequential incubation in solutions (0.01 M by repeat unit) of PAA (between pH 2.5 and pH 7.5) and PAH (pH 7.5) for 10 minutes each. The formation of the PEMs onto PDMS was initiated by the adsorption of PAH. PDMS sheets were rinsed with DI water 3 times for 1 minute each after immersion in each polyelectrolyte solution. After assembly, the PEMs were dried in vacuum at 60°C for 1 hour. Number of bilayers (n) fabricated are denoted as (PAH/PAA)*ₙ*. Fluorescently labeled PEMs were assembly using fluorescein-5-isothiocynate (FITC) (Ext/Em-492/518)-labeled PAH. The PEMs were nanometer-thick (10-200 nm). Their porous and supramolecular architecture allows them to be impregnated with a range of bioactive agents post-assembly or during assembly.

Synthesis of the silver nanoparticles within the PEMs was initiated by incubation of the pre-assembled PEMs on PDMS in an aqueous solution of AgNO₃ (10mM) for 1 hour, followed by rinsing in water. Silver ions (Ag⁺) diffuse into the PEMs and exchange with the carboxylic protons of the PAA. The carboxylate-bound silver ions (Ag⁺) within the PEMs were then subsequently reduced to form Ag(0) nanoparticles by incubating PEMs in NaBH₄ (2 mM) aqueous solution (pH 7.0) for 10 min and again rinsing with water. Besides forming the AgNPs, this reducing condition regenerates the carboxylic acid groups within the PEMs. Therefore, additional Ag⁺ could be loaded into the PEMs. This process of silvr-ion exchange and reduction in PEMs was repeated to obtain PEMs with desired silver loading. Silver loading in PEMs was determined by extracting the silver ions from PEMs into 2% nitric acid for 24 h. The concentration of Ag⁺ extracted from the PEMs was measured by elemental analysis using an inductively coupled plasma (ICP) emission spectrometer (Perkin Elmer Optima 3000 DV) at the wavelength of 328.068 nm.

In some PEMs, micropartilces or nanoparticles were impregnated during fabrication. Negatively charged, crimson fluorescent (Ext/Em- 625/645 nm) carboxylate-modified polystyrene (PS) microspheres, 2 µm in diameter (cat# F8816, Invitrogen, Carlsbad, CA), were incorporated into the PEMs as described elsewhere. Briefly, PAH(PAA/PAH)₁₀ multilayers deposited on PDMS sheets and terminating in a positively charged PAH layer were incubated with a 1 wt% suspension of microspheres for 2 h, and then washed 3 times with water. Subsequently, another set of (PAH/PSS)₂ multilayers was deposited over the microspheres (PSS= poly(styrenesulfonate), MW 70 kDa, Sigma). This was followed by the deposition of a layer of positively charged 20 nm diameter poly(vinylpyrrolidone) (PVP)-coated silver nanoparticles (NanoAmor Inc, Houston, TX)²²⁻²⁴ Briefly, a 0.2 wt% suspension of nanoparticles in aqueous solution, adjusted to pH 2.0 using dilute nitric acid, was sonicated and vortexed for 30 min several times for homogenous suspension. The suspension was then incubated over the PEMs ending with negatively charged PSS for 2 min, and then rinsed off with water 3 times. To increase the loading of silver in the PEMs, another set of PSS(PAH/PSS)₂ multilayer was deposited over the first layer of silver nanoparticles, followed by deposition of the second layer of silver nanoparticles. Finally, the PEMs were capped with a set of (PAA/PAA)₂ multilayer.

The final structure of the PEMs was denoted as PAH(PAA/PAH)₁₀(PS-microspheres) (PAH/PSS)₂(Ag-NP)PSS(PAH/PSS)₂(Ag-NP)(PAA/PAH)₂.

### Synthesis of PEMs with Chlorhexidine:

PAA (MW 60 kDa) was obtained from Polysciences (Warrington, PA). PAH (MW 70 kDa), Chlorhexidine diacetate (CX) (MW 625 Da) and all other reagents were obtained from Sigma Aldrich (St. Louis, MO). PEMs with desired number of multilayers were assembled on Poly(dimethylsiloxane) (PDMS) sheets, or on plasma-cleaned silicon wafers, using a StratoSequence Robot (nanoStrata Inc, Tallahassee, FL), by sequential incubation in solutions of PAA and PAH (0.01 M by repeat unit) and/or chlorhexidine diacetate (0.1mM) for 10 min each. Polyelectrolytes solutions were adjusted to the desired pH using either 1 M HCL or 1 M NaOH; eg: PAH (adjusted to pH 7.5), PAA (adjusted to pH 5.5) and CX (pH of -8.0). The formation of the PEMs onto PDMS was initiated by the adsorption of PAH. Stamps were rinsed with DI water 3 times for 1 min each after immersion in each polyelectrolyte solution. After assembly, the PEMs were dried in vacuum at 60°C for 1 hour.

PEMs with CX were assembled in different architectures, including (PAH/PAA)*₅*(CX/PAA)*ₙ* architecture with successive depositions of CX and PAA, and a 'tetralayer' structure of (PAH/PAA/CX/PAA)*ₙ*) with incorporation of cationic chlorhexidine molecules between alternating PAH and PAA bilayers.

PAH labeled with fluorescein-5-isothiocyanate (FITC) (Ext/Em- 488/510) (cat# F1906, Invitrogen, Carlsbad, CA) was used to prepare fluorescent films. Crimson fluorescent (Ext/Em- 625/645) FluoSpheres^{®} carboxylate-modified polystyrene (PS) microspheres, 2 µm in diameter (cat# F8816, Invitrogen), or non-fluorescent carboxyl latex microspheres, 2 µm in diameter (cat# C37274, Invitrogen) were used to prepare PEMs containing microspheres. The microspheres were washed three times with DI water in Eppendorf tubes by centrifuging for 5 min at 9000g, and re-suspended in fresh DI water by sonication and vortexing before use. To deposit a monolayer of microspheres on the PEMs, PAH/PAA multilayers ending in cationic PAH layer were incubated with a suspension of negatively charged microspheres for 2 h, and then washed 3x with water. Additional layers of polyelectrolytes were assembled subsequently over the microspheres.

To determine the release profile of chlorhexidine in solution from PEMs, substrates containing PEMs were carved out into 2 cm diameter pieces and incubated with 2 mL PBS buffer (pH 7.4) in a 12-well (non-tissue culture treated) polystyrene plate. At defined time points, the entire solution was collected from the wells of the plate and replaced with fresh buffer. The samples were stored at 4°C until characterized. The raw data in µg/mL of the chlorhexidine concentration in the eluted solution was multiplied the total elution volume and normalized by the surface area of the substrate to be reported as drug released per unit surface area (µg/cm²). Concentration of chlorhexidine in aqueous solutions was quantified by measuring their UV absorbance at 255 nm wavelength on a UV-Vis spectrophotometer (Beckman Coulter, Fullerton, CA). Fresh standard calibration curves of CX concentrations in solution were prepared for each experiment, with 0.1 µg/mL CX used as minimum standard concentration.

### Microsheet with PEMs and a sacrificial polymer layer

To fabricate a microsheet with PEMs, a water-soluble sacrificial film of non-toxic polymer- polyvinyl alcohol (PVA) (Mw= 22 kDa) was deposited over the PEMs. Briefly, a PVA solution (2.5 wt% - 25 wt%) was spin coated (2000 rpm; acceleration=27) for 10s. PEMs/PVA microsheet was oven dried for 5-10 min at 70°C. After baking, the PVA/PEMs microsheet, with PEMs and PVA film, was peeled off the supporting PDMS sheets using tweezers. The freestanding microsheet were mechanically robust and could be handled and placed over a secondary device or tissue surface. The water-soluble cast quickly dissolved in aqueous solution, or in moisture on a device surface, or in the moist wound tissue, leaving the polymer nanofilms floating in a solution or adhered to the device/tissue surface.

### In-vitro antibacterial assay

The strain of *Staphylococcus aureus* subsp. *aureus* ATCC 25923 was obtained from The American Type Culture Collection (Manassas, VA). Bacteria were grown in Tryptic Soy Broth with Yeast Extract (BD, Franklin Lakes, NJ) overnight at 37°C with shaking (200 rpm) until a cell density of approximately 4×10⁹ CFU/mL was reached, as determined from optical density (600 nm) measured on a UV-vis spectrometer (Beckman Coulter, Fullerton, CA). The bacterial suspensions were centrifuged at 2700 rpm for 10 min and the pellet washed and resuspended in PBS. In antibacterial assays, test substrates were placed in the wells of 96-well plates and incubated with 100 µL HBSS (Hank's Buffered Salt Solution) (pH 7.4) containing 10⁷ CFU of S. *aureus.* Plates were incubated with shaking (200 rpm) at 37°C for 24 h. After incubation, the fluid in each well was collected, the wells rinsed twice with 200 µL ice cold PBS, and the fluid and washes from each well pooled and brought to 1 mL in PBS. Serial dilutions of the solutions in PBS were spread onto Trypticase Soy Blood Agar plates (#221261, BD, Franklin Lakes, NJ) and incubated at 37°C. Viable bacterial colonies were counted on agar plates after 24 h incubation. All assays were carried out on at least three different days, with at 3-6 replicates of each test sample in each experiment.

### Wound healing in excisional wounds in mice:

All experimental protocols were approved by the Institutional Animal Care and Use Committee (IACUC) of the University of Wisconsin-Madison. Phenotypically normal male mice (heterozygous for *Lepr^{db},* Jackson Laboratories, Inc.) between the ages of 8-12 weeks were used. Mice were housed in groups during a one week acclimation period prior to the study and housed individually thereafter. Throughout the study period, mice were maintained in a temperature-controlled facility with a standard light/dark cycle. All mice were provided with environmental enrichment and food and water *ad libitum.* Mice were randomly assigned to either control or experimental groups on the day of surgery. For wounding, mice were anaesthetized with inhaled isoflurane, administered using an induction chamber. The mice were injected subcutaneously with buprenorphine (0.1 mg/kg) for pain control and the cranial thoracodorsal region was shaved and aseptically prepared for surgery. Wounds were splinted to prevent wound contracture and promote wound closure by epithelialiation. Silicone O-rings (McMaster-Carr®, inner diameter 11 mm, outer diameter 15 mm) were applied to the skin 4 mm caudal to the base of the ears on each side of the dorsal midline and secured with tissue glue (Tissumend II) and six 5-0 interrupted nylon sutures. Wounds were centered in the skin circumscribed by the O-ring. A 6-mm biopsy punch was used to create two symmetrical wounds. Each wound was then covered with an 8-mm disc of test dressing (eg. Biobrane™) that had been sterilized by UV light for 30 minutes. Sterile non-adherent padding was placed on top of the Biobrane, and the entire construct was covered with Tegaderm™, secured with tissue glue. Mice were recovered from anesthesia on a warming pad. Wounds were photographed using a digital camera and body weights of mice were registered on post-operative day 1, and every 2-3 days thereafter until the end of the study. Upon completion of the study, mice were killed by intra-peritoneal injection of Beuthanasia^{®}-D (Schering-Plough) solution (0.5 ml/mouse) after induction of anesthesia.

For the duration of the studies, the splints and sutures were monitored daily. Any loss of contact between the splint and skin was repaired with glue. Broken or missing sutures were replaced under anesthesia as described above, and a dose of buprenorphine was administered subcutaneously (0.1 mg/kg) for pain control. Wounds were removed from analysis if two or more sutures were broken or missing within a 24-hour period. Wounds were digitally traced in the photographs and the area calculated using NIH ImageJ software. Wound closure was calculated at each time point as the percentage of the original wound area.

For microbial-burden studies, a bacterial suspension of 10⁶ CFU of S. *aureus* in 10 µL PBS was applied topically to the wound and allowed to absorb for 15 minutes before application of a wound dressing (e.g., Biobrane). At the end of the study, all mice were euthanized and the wounds with the attached Biobrane were excised aseptically with a 6 mm biopsy punch. Wounds and Biobrane were homogenized for 15 minutes in 1 mL PBS. Homogenates were serially diluted in PBS and plated on blood agar. Bacterial quantification was performed for each wound and reported as CFU per cm² of the wound surface area. If a wound yielded no bacterial colonies, a value of 1.0 CFU per cm² was assigned to it for purposes of statistical analysis. Bacterial counts for the two wounds on each mouse were averaged and the latter used for statistical analysis in each experimental group. Statistical analysis was performed using SigmaPlot^{®} software (Systat Software, Inc.). All normally distributed data were analyzed by Student's t-test. All non-normally distributed data were analyzed by Mann-Whitney U test. Significance was set at p < 0.05.

### Results

The synthesis of polymer nanofilms as microsheets with a sacrificial supporting layer was efficient and produced nanosheets with excellent bioactivity. Figure 15 provides images of microsheets composed of a sacrificial water-soluble cast of PVA and a nanofilm made with PEMs containing silver-nanoparticles. Panel A provides an image of a microsheet (1" × 1") placed on a glass dish. Panel B provides an image of a polymer nanofilm matrix (1" × 1") floating on a water surface after the dissolution of sacrificial cast of the microsheet in solution.

Sustained release of bioactive agents from the polymer nanofilms was assayed in vitro. Figure 18 demonstrates sustained release of silver-ions in PBS from polymer nanofilms assembled as PEMs of PAH and PAA containing 11 ± 2.1 ug/cm² of silver-nanoparticles. Figure 19 demonstrates sustained release of chlorhexidine (CX) in PBS from polymer nanofilms fabricated as PEMs containing chlorhexidine acetate molecules.

The use of the bioactive polymer nanofilms was assessed in vivo in a mouse wound model. Figure 20 provides images of application of a microsheet as a silver-microfilm wound dressing on excisional wounds in mice. (A) A 6-mm diameter full-thickness splinted wound on the flank of a mice. (B) Coverage of wound by a silver impregnated microfilm wound dressing. The sacrificial cast dissolved in moist wound to immobilize silver-nanofilm polymer matrix on the wound surface. Figure 21 provides data showing reduction in the bacterial colonization of excisional wounds in mice on day 3 post-surgery by a microsheet used as silver-microfilm wound dressing containing silver-nanoparticles. All wounds were inoculated with S. aureus on day 0 and covered with Integra™ bilayer wound dressing, or covered with silver-microfilm wound dressing (microsheet) along with Integra™ dressing. The bioactive plymer nanofilms expedited wound closure in vivo.

Figure 22 provides data demonstrating that silver-microfilm wound dressing (microsheet) prevents infection and expedites closure of contaminated wounds in mice. Wounds were inoculated with S. *aureus* and covered with a biosynthetic dressing (Biobrane™) in one group (see Figure 22A) or covered with a silver-microfilm wound dressing (microsheet) before placement of Biobrane™ on the wounds (see Figure 22B). Figure 22A and Figure 22B show representative images of wounds in group A and group B on day 9 post surgery. In Figure 22C, the plot shows the area (average ± SEM) of wounds (as percentage of original wound size) on day 9 post surgery in mice wounds that were treated either with only Biobrane dressing or with silver-microfilm wound dressing and Biobrane (n = 10 mice/group; p < 0.05). In Figure 22D, the plot shows the microbial burden (average ± SEM) of S. *aureus* in wound biopsies harvested on day 9 post surgery from the two groups of mice. Microbial colonization of wounds was significantly less in the group treated with silver microfilm dressing (n=10 mice/group; p<0.05; Mann-Whitney U test). In Figure 22E, the plot shows the percentage wound closure (average ± SEM) (as percentage of original wound size) on day 9 post surgery. In sum, these data indicated that wound closure was significantly better (e.g., faster) and infection significantly lower in the group treated with silver microfilm dressing (n=10 mice/group; p<0.05; Mann-Whitney U test).

The use of the microsheets to modify medical surfaces on medical devices was also assessed, as illustrated in Figure 23. Figure 24 shows immobilization of polymer nanofilms by a microsheet on the surface of moist collagen matrix of Integra™ bilayer wound dressing. (A) Micrograph of the surface of collagen matrix of Integra™ wound dressing. (B) Micrograph of fluorescent polymer nanofilm immobilized on the collagen matrix of Integra™ wound dressing after the dissolution of the sacrificial cast of a microsheet. Scale bar= 200 um. Figure 25 shows immobilization of a polymer nanofilm containing microspheres by a microsheet on the moist collagen matrix of Integra wound dressing. (A) Micrograph of a microsheet with a polymer nanofilm containing 2 µm size fluorescent microsphers, as assembled on an elastomeric sheet. (B) Micrograph of polymer nanofilm (with fluorescent microspheres) immobilized on the moist collagen matrix of Integra dressing after dissolution of the sacrificial cast of the microsheet. Scale bar= 20 µm.

Modification of medical devices such as wound dressings with the bioactive nanoscale polymer layers provides for a reduction in bacterial colonization following use of the modified device. Figure 26 shows reduction in bacterial colonization in solutions incubated on the surface of Integra™ Bilayer wound dressing modified by silver-nano films (AgNP) using microsheets with water-soluble sacrificial casts of PVA. Solutions over test dressings were repeatedly inoculated with10⁷ CFU/ml of S. aureus every 24 h. Similarly, Figure 26 shows reduction in the bacterial colonization of excisional wounds in mice on day 3 post-surgery by Integra™ Bilayer wound dressing in which collagen matrix surface was modified with silver-microsheets containing silver-nanoparticles (AgNP). All wounds were inoculated with S. aureus on day 0 and covered with either Integra™ wound dressing, or Integra™ dressing surface modified with silver-microsheets.

### Reference Example 16. Fabrication of nanoscale polymer matrix by spray coating.

In some examples, PEMs of PAA and PAH were assembled on PDMS sheets, glass substrates, and silicon wafers, by spray coating polyelectrolytes using a multiarm spraying robot. An automated SPALAS™ (Spray Assisted Layer-by-layer Assembly) coating system was employed.

Polyelectrolyte solutions, rinse water and air were delivered through four different spray nozzles onto the substrate. Substrates of up to 6" × 10" were coated with PEMs using this method. PEMs fabrication on a PDMS sheet was initiated by first spraying PAH. After that, negatively charged polyelectrolyte solution (PAA) and positively charged polyelectrolyte solution (PAH) was sprayed in alternative cycles with intermittent water rinsing and drying cycles. PEMs with (PAH/PAA)₂₀ bilayers were fabricated in an example. PEMs were subsequently impregnated with silver-ions;that were subsequently reduced to silver-nanoparticles in-situ. Silver loading in PEMs was characterized using inductively coupled plasma emission spectroscopy (ICPES) to range from 1 µg/cm² to 21 µg/cm². The thickness of (PAH/PAA)₂₀ nanofilms, fabricated by spray coating on silicon wafers and characterized using ellipsometry, was in the range of 100-200 nm.

### Example 17. Mechanical strength of microsheets.

Figure 27 provides a stress strain curve showing mechanical strength of a silver-microsheet at 25°C in a Dynamic Strain Sweep test at 0.1 Hz frequency, as measured using a Film and Fiber tool on a Dynamic Mechanical Analyzer (DMA). Elastic Young storage modulus (E') was ~10⁹ Pa and elastic loss modulus (E") was ~10⁸ Pa. Microsheet was made of a PVA cast over nanofilms (PEMs) of (PAH/PAA)₂₀ impregnated with silver-nanoparticles. Rectangular microsheet strips of width 3 mm were used for this tensile testing. Microsheet was clamped on the tension clamp such as to provide 10 mm of specimen length.

### Example 18. Polymeric multilayer nanofilms with silver nanoparticles reduce microbial burden in contaminated wounds

### A. Summary

During developments of particular embodiments of the technology provided herein, data were collected indicating that polymeric multilayer nanofilms comprising antimicrobial agents, when immobilized on wound-beds through use of a free-standing composite microfilm delivery construct, provide sustained and intimate contact of antimicrobials with the wound-bed. This approach provided therapeutic activity at significantly lower antimicrobial loadings than in conventional dressings (thus lowering potential for tissue toxicity and treatment costs). Specifically, nanometer-thick polyelectrolyte multilayers (PEMs) were assembled by sequential deposition of poly(allylamine hydrochloride) and poly(acrylic acid) by dip coating on polydimethylsiloxane (PDMS) sheets. Post-fabrication, nanofilms were impregnated with silver ions that were subsequently reduced in situ to silver nanoparticles. In some embodiments, a dissolvable cast of poly(vinylalcohol) PVA was spin-coated over the PEM to create a PEM/PVA composite that is peeled from the underlying support to create an easily handled microfilm.

When placed on a moist wound, the PVA cast of the composite microfilm dissolved, leaving the PEMs immobilized on the wound-bed (Figure 28). In vitro, PEMs (releasing 1-2 µg cm^{―2} day^{―1} of silver ions for up to 30 days) immobilized by the microfilms on human cadaver skin-dermis killed 5 log₁₀ CFUs of pathogenic bacteria *S. aureus* in 24 hours. In BALB/c mice, full-thickness splinted wounds topically inoculated with 10⁵ CFU of S. *aureus* and covered with a collagen dressing (Biobrane®), presented up to 3 log₁₀ CFUs less bacterial burden when treated with silver/microfilms for 3 days, as compared to unmodified wounds. In uncontaminated wounds, silver/microfilms allowed normal and complete wound closure by re-epithelialization. These results indicate that modification of wound-beds with PEMs that contain silver nanoparticles and are delivered using a dissolvable microfilm construct does not impair wound healing but effectively reduces microbial colonization under primary dressings using low loadings of antimicrobial agent. Details of these experiments are provided below:

### B. Materials & Methods

*Materials:* PAH (Mw = 65 kDa), sodium borohydride, and PVA (Mw = 13-23 kDa, 98% hydrolyzed) were obtained from Sigma Aldrich (St. Louis, MO), silver nitrate from Alfa Aesar (Ward Hill, MA), and PAA (Mw = 50 kDa) from Polysciences (Warrington, PA). For the fluorescent study, PAH was labeled with fluorescein-5-isothiocyanate (FITC) (excitation and emission wavelengths 492 nm and 518 nm, respectively) (Sigma Aldrich) using procedures described elsewhere (J. K. Gupta, E. Tjipto, A. N. Zelikin, F. Caruso, N. L. Abbott, Langmuir 2008, 24, 5534). Polished silicon wafers were purchased from Silicon Sense (Nashua, NH). Terminally gamma irradiated human cadaver skin allograft, GammaGraft®, was obtained from Promethean LifeSciences Inc, Pittsburgh, PA. All work with GammaGraft was performed under sterile conditions in an air-flow hood.

*Cleaning of silicon wafer or glass slides:* Glass microscope slides and silicon wafers were cleaned sequentially in acidic piranha solution (70:30 (% v/v) H₂SO₄: H₂O₂) and alkaline piranha solution (70:30 (% v/v) KOH: H₂O₂) for 1 hour at 80°C, according to published procedures (J. J. Skaife, N. L. Abbott, Chem. Mater. 1999, 11, 612). When required, cleaned glass slides and silicon wafers were coated with octadecyltrichlorosilane (OTS) (using procedures described elsewhere; see, e.g., J. M. Brake, N. L. Abbott, Langmuir 2002, 18, 6101) to generate a low-energy surface.

*Preparation of PEMs:* Poly(dimethylsiloxane) (PDMS) sheets were fabricated by curing Sylgard 184 (10:1 base to catalyst; Dow Chemical, Midland, MI) on OTS-functionalized silicon wafers or glass slides at 60°C for 24 hours. After 24 hours, PDMS was released from the silicon wafers/glass slides and PEMs were assembled on the side of the PDMS that previously was in contact with the OTS-coated silicon wafer/glass slide. The OTS-coated silicon wafer was used to ensure formation of a smooth surface on each PDMS sheet. Aqueous solutions of polyelectrolytes were adjusted to the desired pH using either 1 M HCl or 1 M NaOH for layer-by-layer assembly. The PEMs were assembled on PDMS sheet using a StratoSequence IV, a robotic dipping machine, from nanoStrata Inc, Tallahassee, FL. The PDMS sheets were first immersed into a PAH solution (0.01 M) for 10 min followed by three 1-minute rinses with deionized water (Millipore, 18.2 MΩ). Next, the substrates were immersed in PAA solution (0.01 M) for 10 minutes followed by the rinsing steps described above. The adsorption and rinsing steps were repeated until the desired number of multilayers was deposited, as described elsewhere (A. Agarwal, K. M. Guthrie, C. J. Czuprynski, M. J. Schurr, J. F. McAnulty, C. J. Murphy, N. L. Abbott, Adv. Funct. Mater. 2011, 21, 1863). Post fabrication, the PEMs were dried in vacuum at 60°C for 1 hour and then stored at ambient conditions.

*Characterization of PEMs:* An Olympus IX70 inverted microscope equipped with Chroma Technology Corp. (Rockingham, VT) fluorescence filter cubes was used to image the fluorescence from fluorescent PAH. Images were captured and analyzed using the Metavue version 7.1.2.0 software package (Molecular Devices, Toronto, Canada). A Gaertner LSE ellipsometer (λ = 632.8 nm, ψ = 70°) was used to measure the thickness of PEMs prepared on silicon wafers. The effective substrate parameters (nₛ= 3.85, kₛ = -0.02) were obtained by averaging twelve measurements on the silicon wafers. The refractive index of the PEMs was assumed to be 1.55 (N. a. Lockwood, K. D. Cadwell, F. Caruso, N. L. Abbott, Adv. Mater. 2006, 18, 850).

*Loading of silver nanoparticles in PEMs and quantification of silver release from PEMs:* Synthesis of silver nanoparticles (AgNPs) within the PEMs was initiated by incubation of the pre-assembled PEMs on PDMS in an aqueous solution of AgNO₃ (10 mM) for 1 hour, followed by rinsing in water. As described in past reports (T. C. Wang, M. F. Rubner, R. E. Cohen, Langmuir 2002, 18, 3370; S. Joly, R. Kane, L. Radzilowski, T. Wang, A. Wu, R. E. Cohen, E. L. Thomas, M. F. Rubner, Langmuir 2000, 16, 1354), Ag⁺ ions diffuse into the PEMs and exchange with the carboxylic protons of the PAA. The carboxylate-bound Ag⁺ ions within the PEMs were subsequently reduced to form Ag(0) nanoparticles by incubating PEMs in aqueous NaBH₄ (2 mM) solution for 1 minute and again rinsing with water (M. Logar, B. Jancar, D. Suvorov, R. Kostanjšek, Nanotechnology 2007, 18, 325601). In addition to forming the AgNPs, the acidic solutions conditions regenerate the carboxylic acid groups within the PEMs. This allows additional Ag⁺ to be loaded into the PEMs. Repeated incubation in Ag⁺ solutions followed by reducing agent solutions can be used to increase the loading of silver in the PEMs.

The loading of silver incorporated into the PEMs was determined by extracting the silver from the PEMs (which were punched into circles of 6 mm diameter using a biopsy punch) into 5 mL of 2% nitric acid by incubation for 24 hours. The concentration of Ag⁺ extracted from the PEMs was measured by elemental analysis using an inductively coupled plasma (ICP) emission spectrometer (Perkin Elmer Optima 3000 DV) at the wavelength of 328.068 nm (Z. Shi, K. G. Neoh, S. P. Zhong, L. Y. L. Yung, E. T. Kang, W. Wang, J. Biomed. Mater. Res. A. 2006, 76, 826). The detection limit of the instrument was specified to be -0.1 ppb. Details regarding the analytical method can be found elsewhere (A. Agarwal, T. L. Weis, M. J. Schurr, N. G. Faith, C. J. Czuprynski, J. F. McAnulty, C. J. Murphy, N. L. Abbott, Biomaterials 2010, 31, 680).

Similarly, the release of silver from the PEMs into aqueous solution was quantified by ICP. Sample substrates were individually placed into 15 mL centrifuge tubes, immersed in 3 mL of PBS buffer and incubated without agitation. Substrates were removed daily and placed into new vials containing 3 mL of fresh PBS to simulate sink conditions, with at least 3 samples prepared per experimental group. For ICP analysis, 1 mL of solution from each vial was combined with 2 mL of 3% nitric acid.

*In vitro antibacterial activity: S. aureus* (ATCC 6538) and *P. aeruginosa* (ATCC 27853) were obtained from ATCC (Manassas, VA). Bacteria were grown in Tryptic Soy Broth Yeast Extract (TSBYE) (BD, Franklin Lakes, NJ) overnight at 37°C with shaking at 200 rpm until a cell density of approximately 4 × 10⁹ CFU/mL was reached. The cell density was calculated from a standard curve for optical density (600 nm) of bacterial suspensions prepared using a UV-Vis spectrometer (Beckman Coulter, Fullerton, CA). The bacterial suspensions were centrifuged at 4000 rpm for 10 minutes and the pellet washed and resuspended in Hank's Balance Salt Solutions (HBSS) buffer. For antibacterial assays, the test samples: 1) GammaGraft without PEMs, 2) PEMs without silver on GammaGraft, and 3) PEMs with silver on GammaGraft, were placed at the bottom of the wells of 96-well plates and were incubated with 100 µL PBS buffer (pH 7.4) containing 10⁷ CFU of bacteria. Plates were incubated with shaking (150 rpm) at 37°C for 24 to 48 hours. After the incubation period, the solution in each well was collected in Eppendorf tubes pre-filled with 900 µL of ice cold PBS to make a 1 mL suspension of bacteria. To ensure collection of most of the bacteria present in the well, each well was rinsed twice with 200 µL diluted bacterial solution collected previously. The viable bacterial cells in the washings were determined by a surface spread-plate method (B. Herigstad, M. Hamilton, J. Heersink, J. Microbiol. Methods 2001, 44, 121). Dilutions of the samples were prepared in PBS and 100 µl of each diluted sample was spread onto Trypticase Soy Blood Agar plates (#221261, BD, Franklin Lakes, NJ). After incubation in a 37°C incubator for 24 hours, bacterial colonies were counted and used to calculate the mean colony forming units (CFU) per ml. All assays were carried out on at least three different days, with at least three replicates of each test sample performed on each day.

*In vivo antibacterial and wound healing study in mice full-thickness wound model:*
All experimental protocols were approved by the Institutional Animal Care and Use Committee (IACUC) of the School of Veterinary Medicine, University of Wisconsin-Madison. Phenotypically normal male mice (heterozygous for Lepr^{db}, Jackson Laboratories, Inc.) or BALB/c mice, 8-12 weeks old, were used in this study. In order to prevent other mice from disturbing the wounds, mice were housed individually in ventilated cages. All mice were acclimatized for a minimum of one week before the surgery. Throughout the study period, mice were maintained in a temperature-controlled facility with a standard light/dark cycle. All mice were provided with environmental enrichment and food and water *ad libitum.* Mice were randomly assigned to either control or experimental groups on the day of surgery. For wounding, mice were anaesthetized with inhaled isoflurane, administered using an induction chamber. After buprenorphine (0.001 mg) delivery to the mice (used for pain control), hair on the left and right thorax immediately behind the shoulder was removed using electric clippers. The area was swabbed with 4% chlorhexidine gluconate surgical scrub alternating with 0.9% sterile saline (3 times each). Subsequently, silicone O-rings (McMaster-Carr, inner diameter 11 mm, outer diameter 15 mm) were applied to the skin 4-mm caudal to the base of the ears on each side of the dorsal midline and secured with tissue glue (Tissumend II) and six 5-0 interrupted nylon sutures. Two 6 mm diameter full thickness excisional wounds were created on the back of each mouse using a biopsy punch. Wounds were centered in the skin circumscribed by the O-ring. O-ring splint application was used to decrease contracture of the wound margins over the course of the study (R. D. Galiano, J. Michaels, M. Dobryansky, J. P. Levine, G. C. Gurtner, Wound Repair. Regen. 2004, 12, 485). Following wounding, 8 mm diameter PEM/PVA composite microfilms (held in tweezers) were placed onto the wound-beds. Wounds were covered with secondary dressings, as described in the text. Mice were recovered from anesthesia on a warming pad. Wounds were photographed using a digital camera and body weights of mice were registered on post-operative day 1, and every 2-3 days thereafter until the end of the study. Upon completion of the study, mice were euthanized by intra-peritoneal injection of Beuthanasia®-D (Schering-Plough) solution (0.5 mL/mouse) after induction of anesthesia.

For the wound healing study, the splints and sutures were monitored daily for the duration of the study. Similar to our previous animal study (K. M. Guthrie, A. Agarwal, D. S. Tackes, K. W. Johnson, N. L. Abbott, C. J. Murphy, C. J. Czuprynski, P. R. Kierski, M. J. Schurr, J. F. McAnulty, Ann. Surg. 2012, 256, 371), any loss of contact between the splint and skin was repaired with glue. Broken or missing sutures were replaced under anesthesia as described above, and a dose of buprenorphine was administered subcutaneously for pain control. Wounds were removed from analysis if two or more sutures were broken or missing within a 24-hour period. Wounds were digitally traced in the photographs and the area calculated using NIH ImageJ software. Wound closure was calculated at each time point as the percentage of the original wound area.

For evaluation of antibacterial efficacy, each wound was inoculated with 10 µL of a bacterial suspension containing ~10⁶ CFU of S. *aureus.* After the bacterial suspension was incubated on the wound for 15 min, the PEM/PVA microfilms (8 mm diameter) were applied to the surgical site and covered with an 8-mm disc of Biobrane® (UDL Labs). PEM/PVA microfilms (with and without silver) and Biobrane were sterilized by UV light for 30 minutes before application to the wounds. Sterile non-adherent padding (Telfa) was placed on top of the Biobrane, and the entire dressing construct was covered with Tegaderm (3M). These covers were used to protect the wound from rubbing and to create a moist environment to encourage bacterial growth. Mice were recovered from anesthesia on a warming pad. After three days, the mice were euthanized. The wounds (including the Biobrane cover) were harvested with a 6-mm biopsy punch, placed into an Eppendorf tube with 1 mL sterile PBS, and homogenized with a bullet blender for 15 minutes. The viable bacterial cells in the homogenate were determined by a surface-spread plate method as described in the *in vitro* assay.

*Histological examinations:* On day 7 post-operative, 2, 5 and 5 mice from the group without microfilms, from the group with microfilms without silver, and from the group with microfilms with silver, respectively, were euthanized. On day 14, after operation, 2, 3 and 3 mice from each group, respectively, were euthanized. For the histopathological study, the entire wound with adjacent normal skin was excised and fixed in 10% buffered formalin. The specimen included the epidermis, dermis and the subcutaneous tissue. Excised wound sites fixed in formalin were processed and embedded in paraffin, and wound sections were stained with hematoxylin and eosin (H&E). Under light microscopy, the H&E sections were photographed using a mounted digital camera (Olympus DP72, Melville, NY) and images were analyzed using an image analysis software (CellSence Dimension 1.4, Olympus, Melville, NY) for length of re-epithelialization, epithelial gap, amount of fibrovascular proliferation in the dermis, and inflammatory response.

*Statistical analysis:* Data are presented as means with standard error of the mean (SEM) as error bars, calculated for three or more data points. Significant difference between two groups were evaluated by Student's t-test and between more than two groups by one-way ANOVA analysis of variance, followed by Tukey's test. The level of significance was set at p < 0.05.

### C. Results

*Fabrication of free standing PEM*/*PVA microfilms:* During the development of embodiments of the technology described herein, layer-by-layer deposition was used to fabricate PEMs consisting of PAH (pH 7.5) and PAA (pH 2.5 and 5.5), starting with deposition of a layer of PAH on a poly(dimethylsiloxane) (PDMS) sheet (see Materials & Methods in Section B for details). Below, PEMs are identified using the notation (Poly1_{pH1}/Poly2_{pH2})ₙ, where Poly1 and Poly2 are the polymers used and pH1 and pH2 are the respective solution conditions at which those polymers were adsorbed; n is the number of bilayers, where one bilayer comprises or consists of one adsorbed layer of Poly1 and one adsorbed layer of Poly2. Here, it is understood that in some embodiments the layers of such "bilayers" are substantially intermixed in PEMs. Growth of (PAH_{7.5}/PAA_{2.5 and 5}.₅)_{10.5} multilayers (vacuum dried after fabrication) on piranha-cleaned silicon wafers were confirmed by performing measurements of ellipsometric thicknesses (Figure 29).

PVA is a non-toxic biodegradable polymer widely used in fabrication of biomaterials including hydrogels (C. R. Nuttelman, S. M. Henry, K. S. Anseth, Biomaterials 2002, 23, 3617; R. H. Schmedlen, K. S. Masters, J. L. West, Biomaterials 2002, 23, 4325). PVA with MW = 22 kDa was employed in these studies so that any polymer absorbed systemically would be removed through renal filtration (Y. Jiang, A. Schädlich, E. Amado, C. Weis, E. Odermatt, K. Mäder, J. Kressler, J. Biomed. Mater. Res. B. Appl. Biomater. 2010, 93, 275). To assemble the microfilms, 25-50 µL of 2.5 wt.% PVA solution was pipetted over PEMs supported on PDMS sheets (6-8 mm diameter), wetting all their surface area. The PDMS disk was subsequently spun at 1500 rpm (ac = 27) for 10 seconds to facilitate deposition of a uniformly thick microfilm of PVA over the PEM. Next, the microfilm construct was dried in an oven for 5 minutes at 70°C. After drying, the composite PEM/PVA construct was peeled from the PDMS surface, e.g., using tweezers. The thickness of the PVA microfilm was measured to be 20-40 µm using an electric digital micrometer (Marathon Watch Co., Richmond Hill, Ontario, Canada). The final PEM/PVA microfilm was measured to have an elastic Young's modulus (E') of 1 GPa (Figure 27). The microfilms were sufficiently robust that they could be easily handled, e.g., by hand or tweezers, and placed on wound surfaces or skin grafts.

The effectiveness of the removal of PEMs from the PDMS sheets was quantified using (PAH/PAA_{5.5})_{10.5} nanofilms that were assembled with fluorescein-5-isothiocynate (FITC) (Ext/Em-492/518)-labeled PAH. Montage fluorescent micrographs were acquired of a PEM/PVA microfilm before and after peeling from the PDMS substrate (Figure 30a, Figure 30b, and Figure 30c). The fluorescent micrographs (imaged using 4× objective) were stitched together using MosaicJ, a plugin available in Fiji software, to create a composite image of the polymer film over a large area (~6 mm diameter). The results demonstrate that 99.3 ± 1.1% of the PEM was peeled from the PDMS sheet (quantified using the fluorescent area measured from micrographs using Fiji software, averaged over 23 samples).

These results were highly reproducible and were obtained using multiple batches of PEMs and PVA. Also, PEMs fabricated on bare glass and coated with PVA could not be peeled from the bare glass substrates. As such, in some embodiments glass substrates are functionalized with octadecyltrichlorosilane (OTS) before deposition of the PEMs. Using the OTS-functionalized glass, it was possible to peel the PEM/PVA microfilm from the rigid substrate (Figure 31). While not being bound by theory (and while an understanding of the mechanism is not required to practice the technology provided herein), this result is consistent with OTS treatment of the glass leading to a low energy surface that only weakly adheres to the PEMs (A. D. Stroock, R. S. Kane, M. Weck, S. J. Metallo, G. M. Whitesides, Langmuir 2003, 19, 2466; T. Fujie, N. Matsutani, M. Kinoshita, Y. Okamura, A. Saito, S. Takeoka, Adv. Funct. Mater. 2009, 19, 2560).

After fabrication, PEMs were impregnated with silver by the exchange of silver ions with protons of the carboxylic groups of the PAA in the PEMs. The silver ions in the PEMs were subsequently reduced *in situ* to form silver nanoparticles (see Materials & Methods in Section B for details). The silver loading in each PEM was tailored by controlling: (i) the number of bilayers in the PEM, (ii) the pH of the assembly solution of the weak polyelectrolyte PAA (pH = 5.5 or pH = 2.5), and (iii) the number of silver ion exchange and reduction cycles. For *in vitro* studies, PEMs with a single cycle of silver ion exchange and reduction were tested and characterized for short-term (1 to 4 days) silver release profiles and antibacterial efficacy. Constructs identified from the *in vitro* studies were impregnated with higher silver loadings by using multiple silver ion exchange and reduction cycles to provide long-term (> 10 days) release of antibacterial silver in murine wounds. As shown in Figure 32, the loading of silver in PEMs of (PAH/PAA)_{10.5} could be varied systematically from 0.04 ± <0.01 to 2.9 ± 0.1 µg cm⁻² by changing the pH of the PAA assembly solution from pH 5.5 to pH 2.5. The PEMs made from (PAH/PAA_{2.5})_{5.5} provided intermediate loadings of silver (1.3 ± 0.01 µg cm⁻²). These PEMs with 3 different loadings of silver were chosen for further *in vitro* antibacterial testing. It is noted that the 0.04 ± <0.01 µg cm⁻² loading was chosen as a control because the PEMs were subjected to the same processing conditions as the other two loadings yet contain minimal active agent. Figure 33 shows that the loadings of silver within the PEMs were measured to be invariant during three months of storage.

Experiments were also conducted to quantify the amount of silver that remained on the PDMS sheets after peeling of the PEM/PVA composite microfilms from the PDMS (second column of Figure 32). For all silver loadings used in this study, only trace amounts of silver were determined to remain on the PDMS (<0.01, 0.05 ± <0.01 and 0.07 ± <0.01 µg cm^{―2}, respectively, for the three loadings described above). The loadings of silver in the free standing PEM/PVA composite microfilms were determined to be 0.04 ± <0.01, 1.2 ± 0.04, and 2.7 ± 0.2 µg cm^{―2}, respectively (third column of Figure 32). These results indicate that most (93 ± 3 %, 95 ± 4 %, and 94 ± 8 %, respectively) of the silver present within the PEMs on the PDMS support was retained in the free-standing PEM/PVA microfilms.

*Transfer of PEMs onto Skin Grafts:* During the development of embodiments of the technology described herein, experiments were performed to simulate the mechanical characteristics and surface topography of a partial thickness wound, experiments were conducted to evaluate the transfer of PEMs onto the dermal side of terminally irradiated human cadaver skin grafts (GammaGraft®, Promethean Lifesciences). To this end, PEM/PVA microfilms with a diameter of 6 mm were peeled from PDMS sheets and were placed onto the moist skin dermis (6 or 8 mm diameter) with the PEMs facing the surface of the tissue. The skin dermis was maintained moist before the placement of the free standing PEM/PVA microfilm using PBS, mimicking moist wounds. PEM/PVA microfilms were held by tweezers (with PEMs side facing the skin dermis) and contacted with the dermis by first placing an edge of the microfilm into contact with the skin dermis and then releasing the microfilm from the tweezers to allow the remainder of the microfilm to adhere to the skin dermis. The PVA cast of each microfilm was observed to dissolve gradually over 10 minutes on the moist dermis and the PEMs were observed to adhere to the dermis.

The efficiency of transfer of the PEMs from the PDMS support onto the skin dermis was evaluated using fluorescent microscopy. To visualize fluorescent PEMs over the background auto-fluorescence of the skin graft, PEMs with 40.5 multilayers of FITC-PAH were assembled. Fluorescent imaging was performed immediately after the PEMs were transferred onto the skin dermis. Quantification of the transfer efficiency was performed using Fiji software by comparing the projected fluorescent area of the PEMs on the PDMS sheets (Figure 34a) with that of the PEMs after transfer onto the skin dermis (Figure 34b), averaged over 13 samples. Repeat experiments confirmed that 99.7 ± 0.5% of the fluorescent area that was projected by the PEMs on the PDMS sheets was transferred onto the skin dermis. The experiments were performed on several different days using different batches of microfilms.

The rate of dissolution of the PVA cast within a composite microfilm was studied in excess PBS using a Malachite Green spectroscopic dye (0.01 wt% dye) mixed into the PVA solution used for casting. The 8 mm diameter composite microfilms were incubated in a multiwell plate in 3 mL PBS, and the cumulative concentration of Malachite Green that was released into the PBS was determined at different time points by measuring the absorbance of the solution at 595 nm wavelength, as shown in Figure 35. From these measurements, we concluded that -98% of the PVA cast dissolves within 10 minutes of contact with the PBS.

The persistence of the PEMs that were immobilized on the skin-dermis following dissolution of PVA was evaluated using fluorescent microscopy. The fluorescent PEMs were observed to remain adhered to the skin-dermis after repeated pipetting of 1 ml PBS (20 µL each) onto the PEMs (Figure 34c). The modified skin-dermis was, subsequently, incubated in multiwell plates for 1 to 3 days in excess PBS solution on shaker plates. Fluorescent micrographs (n = 21) showed that 98.4 ± 1.4% of the PEMs remained adhered to skin dermis (Figure 34d). Additionally, the adherence of the PEMs on the skin dermis was tested against an external vertically applied pressure (~8 kPa) and lateral abrasion generated by the motion of Telfa dressing (a commonly used non-adherent dressing) at ~1 mm/s (Figure 36). In all treatments, >95% of the fluorescent area of the PEMs remained intact on the skin dermis, indicating good adherence of the PEMs to the skin dermis. Without being bound by theory, the strong adherence of the nanofilms on soft tissue is attributed to strong physical interactions, such as through Van der Walls interactions between the PEM and tissue surface (T. Fujie, N. Matsutani, M. Kinoshita, Y. Okamura, A. Saito, S. Takeoka, Adv. Funct. Mater. 2009, 19, 2560). However, an understanding of the mechanism is not required to practice the technology. Some cracks were observed in the PEMs with 40.5 bilayers of (PAH/PAA_{2.5}) (see Figure 37). However, such cracks were not observed in PEMs with only 10.5 bilayers of (PAH/PAA_{2.5}). The presence of the cracks in the thick PEMs, however, did not prevent their efficient transfer onto the skin dermis by the composite microfilms.

The release profile of silver ions in PBS from modified skin dermis is presented in Figure 34e. The amount of silver released in 24 hours was 0.04 ± <0.01, 0.20 ± 0.01, and 0.44 ± 0.04 µg cm^{―2} from the dermis modified with the three PEM constructs described above, respectively. Subsequently, the dermis released none, 0.07 ± 0.01, or 0.18 ± 0.03 µg cm⁻²/day of silver, respectively, for the next 2 days. The total amount of silver released, at saturation, over 6 days was 0.04 ± <0.01, 0.41 ± 0.01, and 0.80 ± 0.03 µg cm⁻², respectively. These results indicate that, (i) the rate of release of silver ions from the modified dermis depends on the loadings of silver within the PEMs, and (ii) the total amount of silver released from the dermis was lower than the total loading of silver in the PEMs determined before their transfer on to the dermis.

*Antibacterial activity of skin dermis modified with composite PEM*/*PVA microfilms:*
During the development of embodiments of the technology provided herein, experiments were performed using skin dermis (GammaGraft) modified with PEM/PVA microfilms to test for antimicrobial activity against suspensions of either Gram-positive bacteria (*Staphylococcus aureus*) or Gram-negative bacteria (*Pseudomonas aeruginosa*)*.* Briefly, biopsy punches (6 mm diameter) of dermis placed at the bottom of the wells of a 96-well plate were incubated with 100 µL HBSS containing ~10⁷ CFU of bacteria on a shaker plate (150 rpm) in a humidified incubator at 37°C. Negative controls in each experiment were no skin dermis, unmodified skin dermis, and skin dermis modified with PEMs without silver. Viable bacterial counts remaining in suspensions were determined by plating, and are reported as colony forming units (CFU).

Bacterial counts in suspension after 24 hours and 48 hours incubation are summarized in Figure 38. Dermis modified by PEMs with no silver or PEMs with 0.04 ± <0.01 µg cm^{―2} of silver resulted in bacterial counts in suspensions that were similar to unmodified skin dermis (as shown in the first two columns of Figure 38). The PEMs containing 1.3 ± 0.01 µg cm^{―2} silver caused 3 log₁₀ and 5 log₁₀ CFU decrease in the suspensions within 24 hours and 48 hours, respectively. Finally, the PEMs containing 2.9 ± 0.1 µg cm⁻² silver caused more than 5 log₁₀ CFU decrease within 24 hours of incubation. PEMs with this latter silver loading also mediated a 5 log₁₀ CFU decrease in bacterial counts in suspensions of *P. aeruginosa* (Figure 39). Experiments indicated that the dermis modified with PEMs containing 2.9 ± 0.1 µg cm^{―2} silver was found to release only 0.44 ± 0.04 µg cm^{―2} of silver in PBS within 24 hours (see Figure 34). This amount of silver (-0.4 µg cm^{―2}) is sufficient to have a potent antibacterial effect while allowing normal growth and proliferation of NIH 3T3 mouse fibroblast cells (A. Agarwal, T. L. Weis, M. J. Schurr, N. G. Faith, C. J. Czuprynski, J. F. McAnulty, C. J. Murphy, N. L. Abbott, Biomaterials 2010, 31, 680).

In summary, the *in vitro* experiments described above demonstrate that PEM/PVA microfilms that adhere to the surface of human cadaver skin dermis release sufficient silver in 24 hours and 48 hours to cause a >5 log₁₀ CFU decrease in bacterial counts in suspensions of *S. aureus* and *P. aeruginosa.* These constructs of (PAH/PAA_{2.5})_{10.5} were subsequently prepared with a range of silver loadings (including higher loadings achieved by repeated silver ion exchange and reduction cycles, see above) to provide sustained release of antibacterial silver from contaminated murine wound beds described below.

*Modification of excisional wounds in mice using PEM*/*PVA microfilms:* During the development of embodiments of the technology provided herein, experiments were conducted to test modification of excisional wounds in mice using PEM/PVA microfilms. All *in vivo* experiments were performed in an excisional full-thickness wound model in mice, as described in the Materials & Method Section B. Briefly, wounds (diameter 6-8 mm) were surgically created on the left and right flanks of mice and were splinted, as described previously (R. D. Galiano, J. Michaels, M. Dobryansky, J. P. Levine, G. C. Gurtner, Wound Repair. Regen. 2004, 12, 485) and shown in Figure 40a. The wounds were 'splinted' to minimize wound closure by contraction and, thus, allow wound closure primarily by epithelialization as in human dermal healing (R. D. Galiano, J. Michaels, M. Dobryansky, J. P. Levine, G. C. Gurtner, Wound Repair. Regen. 2004, 12, 485). Initial *in vivo* studies determined the transfer and persistence of PEMs on the wound-bed. Freshly prepared surgical wounds (6 mm diameter) were allowed to exude excess fluid for 5 minutes and then were covered with the PEM/PVA microfilms, with the PEMs facing the wound-bed, as shown in Figure 40a. Briefly, the edge of the composite microfilm was held firmly in tweezers and one edge was brought into contact with the wound-bed. Release of the microfilm from the tweezers resulted in adherence of the remaining film to the wound-bed. The PVA cast of each microfilm was observed to start dissolving immediately after contact with the moist wound environment. The PEMs were found to adhere conformally to the wound-bed within 10 minutes of dissolution of the PVA cast of the composite microfilms, as shown in Figure 40b.

Transfer of the PEMs onto the wound-bed using the PEM/PVA microfilm was quantified using fluorescent microscopy and PEMs assembled using FITC-labeled PAH (as in studies with skin dermis described above). Fluorescent micrographs of FITC-PEMs in Figure 40c, Figure 40d, and Figure 40e depict the transfer and persistence of PEMs on murine wounds for up to 3 days post-surgery. To quantify the amount of PEM adhered initially to the wound-bed, wounds were harvested within 30 minutes of treatment and imaged to measure the fluorescent area (n = 4). These measurements revealed that the area of the wound covered by the PEM was 107 ± 5 % of the area of the PEM/PVA microfilm prior to transfer onto the wound (shown in Figure 40c and Figure 40d). This result indicates that minor stretching of the PEMs may occur on the elastic tissue surface during harvesting and sample preparation. The PEMs, however, remained firmly attached on the wound-bed.

In one set of mice, splinted wounds modified with fluorescent PEMs were allowed to heal for 3 days and then were harvested for fluorescent imaging. After deposition of these PEMs, the wounds were covered with a plastic cover slip such that the cover slip did not touch the wound-bed but prevented external disturbance to the wound-bed (such as scratching by mice). In addition, an aluminum foil was glued over the cover slips to limit exposure to light. The entire construct was then covered with Tegaderm^{®}. As shown in Figure 40e, approximately 90 ± 2 % (n = 4) of the wound-bed was covered by fluorescent PEMs after 3 days post-immobilization. Approximately 10% of the fluorescent PEM was found to be lost or detached from the tissue surface. Without being bound by theory, it is hypothesized that the PEMs are removed from the tissue surface and dispersed as microscopic fragments as the tissue regenerates. However, an understanding if the mechanism is not required to practice the technology.

*Reduction in microbial colonization in contaminated wounds modified with silverlPEMs using microfilms:* During the development of embodiments of the technology provided herein, experiments were conducted to test the reduction in microbial colonization in contaminated wounds modified with silver/PEMs using microfilms. Splinted wounds in mice were topically inoculated with ~10⁶ CFU S. *aureus* in 10 µl of PBS. Following bacterial inoculation, the wounds were treated with the PEM/PVA microfilms (8 mm diameter) and then covered with the biosynthetic wound dressing Biobrane® (8-mm diameter). Sterile nonadherent padding (Telfa) was placed on top of the Biobrane, and the entire construct was secured with Tegaderm (3M). It is noted here that Biobrane® is a transparent biosynthetic dressing widely used in hospitals for the treatment of burn wounds (I. S. Whitaker, S. Prowse, T. S. Potokar, Ann. Plast. Surg. 2008, 60, 333; E. M. Lang, C. a. Eiberg, M. Brandis, G. B. Stark, Ann. Plast. Surg. 2005, 55, 485). It is composed of a silicone membrane with nylon fibers coated with collagen peptides. Biobrane® seals the wounds and promotes tissue growth. A key advantage of Biobrane is that it integrates into wounds and thus circumvents painful dressing changes (R. L. Klein, B. F. Rothmann, R. Marshall, J. Pediatr. Surg. 1984, 19, 846; M. Tavis, J. Thornto, R. Bartlett, J. Roth, E. Woodroof, Burns 1980, 7, 123). However, it also seals into the wound peri-wound and/or endogenous bacteria, resulting in bacterial colonization and failure of therapy in up to 20% of the applications of the dressing (K. Nichols, Z. Moaveni, A. Alkadhi, W. Mcewan, ANZ J. Surg. 2009, 79, A7). Standard of care is to treat wounds with topical solutions of antimicrobial agents before use of Biobrane, or to cover Biobrane with silver dressings that deliver silver to the wound-bed by diffusion across Biobrane. Accordingly, experiments were conducted to assess if wound-beds modified with silver/PEMs prevented and/or reduced bacterial colonization in wounds under Biobrane. Three groups of n = 8 mice each (e.g., a total of 16 wounds/group) were employed: unmodified wounds, wounds modified with PEMs without silver, and wounds modified with PEMs containing silver nanoparticles.

Extrapolating from the *in vitro* studies, PEMs of (PAH/PAA_{2.5})_{10.5} were used in mouse experiments. To ensure prolonged release of antimicrobial silver in the murine wounds over 14 days (the average time it takes for complete closure of 6 mm diameter wounds), the silver loading in the (PAH/PAA_{2.5})_{10.5} was increased to 16.8 ± 0.5 µg cm⁻² by using three cycles of silver ion exchange and reduction, as shown in Figure 41. The silver loading cycles ended with a final silver ion exchange, such that the PEMs provided an initial burst release of silver ions (bound to carboxylic groups of PAA) that was followed by sustained release of silver ions via dissolution of silver nanoparticles entrapped in the PEMs. A typical silver release profile for these PEMs (from PDMS sheets into PBS) is presented in Figure 42 As shown, the PEMs provided an initial burst release of 3.1 ± 0.1 µg cm⁻² of silver in the first 24 hours, followed by a release of ~1.5 µg cm⁻² silver /day for 4 days, and a release rate of -0.4 µg cm⁻²/day thereafter. The cumulative release of silver over the 30 days period was measured to be 17.2 ± 0.6 µg cm⁻².

Several clinical studies have documented that it takes 48 hours to 72 hours for Biobrane to integrate into wound-beds (R. L. Klein, B. F. Rothmann, R. Marshall, J. Pediatr. Surg. 1984, 19, 846; M. Tavis, J. Thornto, R. Bartlett, J. Roth, E. Woodroof, Burns 1980, 7, 123). If wounds are infected during this period, they form exudate pockets under Biobrane where the dressing does not integrate into the wound-bed (J. E. Greenwood, J. Clausen, S. Kavanagh, Eplasty 2009, 9, 243). Therefore, experiments were conducted and data were collected that demonstrate that modification of contaminated wounds with PEM/PVA microfilms reduce colonization of *S*. *aureus* under Biobrane within the first 3 days post-surgery and ensure normal wound closure thereafter. To that end, all mice were euthanized on day 3 post surgery and wounds were harvested, along with the Biobrane, and homogenized for bacterial quantification. Wounds were also imaged on day 0 and day 3 before euthanizing the mice. Figure 43 shows that an average of 1.8 × 10⁶ CFU/cm² of S. *aureus* was recovered from unmodified wounds on day 3 post surgery. Similarly, 3.1× 10⁵ CFU/cm² of S. *aureus* was recovered from the wounds that were modified with PEMs containing no silver. The bacterial burden of wounds in those two groups was not significantly different (p > 0.05). In contrast, wounds that were modified by silver/PEMs using the PEM/PVA composite microfilms had an average of only 6.1× 10³ CFU/cm² of S. *aureus,* e.g., an average of 2.4 log₁₀ reduction in bacterial burden of the wounds under Biobrane as compared to unmodified wounds. A repeat experiment with a different batch of silver/PEMs and a larger size of n = 10 mice/group provided evidence of a similar reduction in the microbial burden of the wounds under Biobrane (Figure 44). Overall, these results indicate that modification of wound-beds with silver/PEMs is effective in reducing bacterial colonization of contaminated wounds under Biobrane.

*Promotion of normal healing in mice wounds modified with silverlPEMs:* During the development of embodiments of the technology, experiments were performed to demonstrate that modification of wound-beds with silver/PEMs containing 16.8 ± 0.5 µg cm⁻² silver (e.g., a loading that reduces bacterial colonization of contaminated wounds, see above) does not impair normal healing in clean surgical wounds. In particular, experiments were conducted in which wound healing was monitored until complete wound closure by re-epithelialization (observed within 14 days in all test groups). For these experiments, mice were divided into three groups: a control group with unmodified wounds (n = 4 mice), another control group with wounds modified by PEMs without silver (n = 8 mice), and a test group with wounds modified with silver/PEMs (n = 8 mice). Wounds were covered with a plastic cover slip placed over the splints and the entire construct was covered with Tegaderm®. Mice were monitored daily and wounds were photographed on day 0, 3, 7, 10 and 14 post surgery. Half of the mice in each group were euthanized on day 7 to harvest the wounds for histopathological analysis.

Images of the representative wounds treated with silver/PEMs are shown in Figure 45a, Figure 45b, and Figure 45c for post-operative day 0, 7 and 14. Similar representative images for the control groups are shown in Figure 46. These images show, qualitatively, that wounds modified with silver/PEMs exhibited similar rates of wound healing as the controls. The epithelial coverage of the wounds was assessed from digital images of wounds, and is presented in Figure 45d as the percentage of original wound size on post-operative days 3, 7, 10 and 14. The results document that the rate of wound closure in all treatment groups was not significantly different on any particular day of observation (p > 0.05, ANOVA Tukey's test).

Representative histopathological sections of the wounds modified with silver/PEMs are presented in Figure 47a and Figure 47b for day 7 and 14 post-surgery, respectively. Similar representative histopathological images for control wounds are presented in Figure 48. Images at day 7 post-surgery clearly show a migrating re-epithelialization cell layer at the wound edge. In addition, wounds modified with silver/PEMs exhibited re-epithelization similar to that in control wounds. Complete closure by re-epithelialization was observed in images on day 14. The quality of granulation tissue and degree of inflammation in all tissue sections was found not to be significantly different between the three test groups, indicating normal progress of wound healing. The epithelial gap in the wounds, measured from histopathological images on day 7 and 14 post-surgery, is presented in Figure 47c as the percentage of initial wound size. These data indicate that the rate of re-epithelialization was not significantly different among the three treatment groups (p > 0.05, Student's t-test). Overall, these results indicate that modification of the wound-bed using silver-loaded PEM/PVA microfilms (containing 16.8 ± 0.5 µg cm⁻² silver) does not cause excessive tissue inflammation and does not impair normal wound healing by re-epithelialization.

Furthermore, in additional experiments, surgical wounds (6 mm diameter) were created on the flanks of balb/c mice and inoculated with 2×10⁵ CFU S. *aureus.* Wound were covered with a Biobrane biosynthetic dressing in one group of mice (Figure 50A), and covered with a silver-microfilm wound dressing before placement of Biobrane on the wounds in another group (Figure 50B). Micrographs of haematoxylin and eosin (H&E) stained wounds on day 12 post-surgery) showed that a wound treated with Biobrane alone had an epithelialization gap of approximately 2.1 mm (see, e.g., Figure 50A, between the arrows). In contrast, H&E stain micrographs from mice treated with a silver-microfilm wound dressing showed that wounds treated with silver microfilm dressing and Biobrane had complete epithelialization (see, e.g., Figure 50B, scale=0.5 mm). Table 2 summarizes histopathology observations (by a board certified pathologist) of the two groups of wounds on day 12 post-surgery. Wounds treated with a silver microfilm dressing had significantly smaller epithelial gap, more collagen, and less inflammation (p<0.05, n=10; Mann Whitney U test).

| **Table 2. Histopathology of wounds on Day 12.** | | |
|---|---|---|
| †Significantly different values (n=10, p<0.05) | | |
| | *Biobrane* | *Biobrane*/*silver microfilm* |
| Epithelial gap (mm) | 0.55 ±0.79 | 0.03 ±0.09† |
| Re-epithelialization (mm) | 3.04 ±1.08 | 3.55 ±1.10 |
| Collagen (% area) | 11.98 ±6.55 | 19.30 ± 5.38† |
| Inflammation score (0-4) | 2.15 ± 0.99 | 1.65 ±0.75† |
| Granulation score (0-4) | 2.65 ± 0.67 | 2.70 ±0.66 |

### Reference Example 19. Use of transition metal gallium as antibiofilm agent in wound dressings

### A. Summary

Conventional antimicrobials and antibiotics are ineffective against microorganisms in biofilms. In particular, bacterial colonization of wounds can lead to chronic infections where bacteria live in biofilms, which are surface associated bacterial communities encased in an extracellular polymeric matrix (Rhoads et al. Biofilms in wounds: management strategies. Journal of Wound Care. 2008;17:502-8). Physiological changes inherent to biofilm growth make bacteria in biofilms approximately 1000 times more resistant to killing by antibiotics and/or antimicrobials than planktonic (free-living) bacteria (Stewart et al. Antibiotic resistance of bacteria in biofilms. The Lancet. 2001;358:135-8). Biofilms can cause failure of treatment, loss of limbs (e.g. diabetic foot ulcers), or death, and biofilms typically need to be surgically debrided in conventional treatment regimes to allow normal wound healing to progress (Wolcott et al. Regular debridement is the main tool for maintaining a healthy wound bed in most chronic wounds. Journal of Wound Care. 2009;18:54-6). The process leading to infection and establishment of biofilms begins at the moment of wounding and needs to be prevented at the onset of wound treatment (Kennedy et al. Burns, biofilm and a new appraisal of burn wound sepsis. Burns. 2010;36:49-56). Accordingly, there is an urgent need for improved dressings and formulations that prevent biofilm formation in acute infections and sensitize bacteria in biofilms in chronic wounds to antimicrobials.

The transition metal gallium (Ga) inhibits bacterial growth, prevents biofilm formation, and disperses established biofilms (Kaneko et al. The transition metal gallium disrupts Pseudomonas aeruginosa iron metabolism and has antimicrobial and antibiofilm activity. The Journal of Clinical Investigation. 2007;117:877-88; Zhu et al. Pre-treatment with EDTA-gallium prevents the formation of biofilms on surfaces. Experimental and therapeutic medicine. 2013;5:1001-4; Valappil et al. Effect of novel antibacterial gallium-carboxymethyl cellulose on Pseudomonas aeruginosa. Dalton Transactions. 2013;42:1778-86; Halwani et al. Co-encapsulation of gallium with gentamicin in liposomes enhances antimicrobial activity of gentamicin against Pseudomonas aeruginosa. Journal of Antimicrobial Chemotherapy. 2008;62:1291-7). Ga disrupts iron (Fe) metabolism of bacteria that is essential for the functioning of key enzymes required for bacterial growth (Kaneko et al. The transition metal gallium disrupts Pseudomonas aeruginosa iron metabolism and has antimicrobial and antibiofilm activity. The Journal of Clinical Investigation. 2007;117:877-88). Free Fe levels are already extremely low at wound sites due to multiple host defenses that sequester Fe to deter microbial infections. Ga taken up by bacteria further decreases Fe uptake of bacteria by repressing Fe-responsive transcriptor regulators (Kaneko et al. The transition metal gallium disrupts Pseudomonas aeruginosa iron metabolism and has antimicrobial and antibiofilm activity. The Journal of Clinical Investigation. 2007;117:877-88). Ga3+ has an ionic radius nearly identical to that of Fe³⁺, and many biologic systems are unable to distinguish Ga³⁺ from Fe³⁺ (Chitambar et al. Targeting iron-dependent DNA synthesis with gallium and transferrin-gallium. Pathobiology : journal of immunopathology, molecular and cellular biology. 1991;59:3-10). Substitution of Fe with Ga disrupts Fe-dependent processes because unlike Fe³⁺, Ga³⁺ cannot be reduced in physiological conditions. As such, sequential oxidation and reduction steps critical for many Fe-mediated biological functions are inhibited by Ga³⁺, resulting in bacterial cell death (Chitambar et al. Targeting iron-dependent DNA synthesis with gallium and transferrin-gallium. Pathobiology : Journal of Immunopathology, Molecular and Cellular Biology. 1991;59:3-10). Moreover, studies have shown that Ga is particularly taken up by the nutrient (and, thus, Fe) starved cells in the center of biofilms to which conventional antimicrobials and antibiotics are least effective, making Ga particularly advantageous in biofilm dispersal (Kaneko et al. The transition metal gallium disrupts Pseudomonas aeruginosa iron metabolism and has antimicrobial and antibiofilm activity. The Journal of Clinical Investigation. 2007;117:877-88). Because of the excellent antibiofilm properties of Ga, together with the fact that it has been FDA approved for intravenous injections (to treat hypercalcemia of malignancy) (Bernstein. Mechanisms of Therapeutic Activity for Gallium. Pharmacological Reviews. 1998;50:665-82), experiments were conducted to test Ga as a component of microfilm wound dressings or polymeric nanofilms to sensitize microbes in biofilms to bactericidal silver.

### B. Results

During the development of embodiments of the technology provided herein, experiments were conducted to determine the minimum concentration of Ga (e.g., as Ga(NO₃)₃) to inhibit biofilm formation. In particular, Ga was added to cultures of *Pseudomonas aeruginosa* in biofilm growth media (500 µL of 1% tryptic soy broth (TSB)) to test the inhibition of biofilm formation at the bottom of 48-well tissue culture plates.

Biofilm growth arising from suspensions of 10⁶ CFU/ml *P. aeruginosa* (ATCC 27853) was quantified after 24, 48, and 72 hours by staining biofilms with crystal violet, following methods described previously (Brandenburg et al. Tryptophan Inhibits Biofilm Formation by Pseudomonas aeruginosa. Antimicrobial Agents and Chemotherapy. 2013;57:1921-5). It was determined that 25 µM Ga³⁺ in solution was sufficient to completely inhibit biofilm formation for 72 hours (Figure 51).

There was no decrease in the free (planktonic) bacterial counts in solution, indicating that Ga³⁺ is not bactericidal at this concentration, but can be employed to sensitize bacteria in biofilms to other bactericidal agents such as silver. For example, other studies have shown that 100 µM Ga³⁺ in TSB disperses established biofilms (Kaneko et al. The transition metal gallium disrupts Pseudomonas aeruginosa iron metabolism and has antimicrobial and antibiofilm activity. The Journal of Clinical Investigation. 2007;117:877-88), and that concentrations of up to 1 mM Ga³⁺ are not cytotoxic to mammalian cells (Chandler et al. Cytotoxicity of Gallium and Indium Ions Compared with Mercuric Ion. Journal of Dental Research. 1994;73:1554-9).

### Example 20. Microfilm dressing with gallium in the sacrificial polymeric cast

During the development of embodiments of the technology provided herein, experiments were conducted to develop and evaluate microfilm dressings that provide 1) a burst release of an antibiofilm agent (e.g., transition metal gallium) in the wounds to inhibit biofilm formation, followed by 2) a sustained prolonged release of a broad spectrum antimicrobial (e.g., silver) that kill bacteria and prevent microbial colonization.

Data were collected from tests of microfilm dressings (e.g., 100-200 µm thick) comprising two distinct polymeric layers as described herein. The top layer comprises a water-soluble polymeric cast and the bottom layer comprises polymer multilayer nanofilms (e.g., 50-200 nm thick) impregnated with bioactive and/or antimicrobial agents. In this example, the microfilm dressings comprise silver nanoparticles in the polymeric nanofilms and the antibiofilm transition metal gallium in the soluble polymeric cast (see, e.g., Figure 52).

Without being bound by theory (indeed, an understanding of the theory is not required to practice the technology), it was contemplated that an initial burst release of gallium in wounds with the dissolution of the polymeric cast of the microfilm dressing would disperse biofilms and render microbes incapable of forming biofilms. Subsequently, polymeric nanofilms containing silver nanoparticles, immobilized on the wound bed, would provide sustained long-term release of antimicrobial silver ions, preventing microbial colonization and reducing need for painful dressing changes. Moreover, it was contemplated that sensitization of microbes in biofilms by the initial burst release of an antibiofilm agent (e.g., gallium) would require significantly low concentrations of bactericidal agent (e.g., silver) to prevent or clear wound infections.

Accordingly, PEMs of (PAH/PAA)₂₀ were assembled on PDMS sheets by spray coating, as described herein. Post-fabrication, PEMs were impregnated with silver nanoparticles by multiple cycles of silver ion exchange and in situ silver ion reduction, as described herein. Subsequently, a film of polyvinyl alcohol (PVA, MW 22 kDa) was applied over the PEMs by spin coating (at 1500 rpm for 10 seconds) a 5% w/v PVA (MW 22 kDa) solution containing gallium nitrate.

Based on the the data collected above in Example 19 (see Figure 51), Ga³⁺ loading in the PVA cast over the PEMs was controlled at between 1 to 10 µg/cm² by adjusting the gallium nitrate concentration in the 5% PVA casting solution in water. Ga³⁺ loading in the PVA casts was determined by dissolution in 2% nitric acid and subsequent elemental analysis by inductively coupled plasma emission spectroscopy (ICPES) (Valappil et al. Effect of novel antibacterial gallium-carboxymethyl cellulose on Pseudomonas aeruginosa. Dalton Transactions. 2013;42:1778-86). PVA casts dissolved in 0.5 mL water in the wells of a 48-well plate release approximately 28 to 280 µM Ga³⁺/cm² in solution.

### Reference Example 21. PEMs with gallium inhibits biofilm formation

During the development of embodiments of the technology provided herein, experiments were conducted to further test PEMs comprising Ga. PMMs of (PAH/PAA)₁₀ were assembled on PDMS sheets by spray coating, thetn were impregnated post-fabrication with Ga³⁺ by incubation in 10 mM Ga(NO₃)₃ solution for 1 hour. Ga³⁺ ions were exchanged with protons on the carboxylic groups of PAA in the nanofilms. PEMs were subsequently rinsed in water and baked at 215°C under N₂ for 2 hours to allow thermal crosslinking via heat induced amide bond formation between PAA and PAH (Harris et al. Synthesis of Passivating, Nylon-Like Coatings through Cross-Linking of Ultrathin Polyelectrolyte Films. Journal of the American Chemical Society. 1999;121:1978-9). Data were collected indicating that crosslinking of PEMs allowed sustained long-term release of Ga³⁺ in PBS (Figure 53).

Ga³⁺ loading in PEMs was determined by extraction in 2% nitric acid and elemental analysis by inductively coupled plasma emission spectroscopy (ICPES) (Valappil et al. Effect of novel antibacterial gallium-carboxymethyl cellulose on Pseudomonas aeruginosa. Dalton Transactions. 2013;42:1778-86). In preliminary studies, PEMs releasing 1 µg/cm² of Ga³⁺ completely inhibited formation of *P. aeruginosa* biofilms similarly to the effect of 25 µM Ga(NO₃)₃ in solution (Example 19; Figure 51). These data indicate that the technology described herein provides for Ga³⁺ incorporation and release from PEMs such that the PEMs release antibiofilm concentrations in solutions.

## Claims

1. A process for manufacturing a wound active nanoscale polymer matrix microsheet, the method comprising the following steps:
a) forming a nanoscale polymer layer about 0.5 nm to 1000 nm thick on a substrate;
b) introducing a wound active agent into said nanoscale polymer layer to provide the wound active nanoscale polymer layer; and
c) forming the sacrificial polymer layer from 0,1 µm thick to 100 µm thick on said wound active nanoscale polymer layer, wherein the sacrificial polymer layer is dissolvable.

2. The process of claim 1, further comprising step
d) removing the nanoscale polymer layer matrix microsheet comprising the bioactive nanoscale polymer layer and associated sacrificial polymer layer from the substrate.

3. The process of Claim 1, wherein said wound active nanoscale polymer layer is a polymer multilayer.

4. The process of Claim 3, wherein said polymer multilayer is formed by alternating layers of at least one positively charged polyelectrolyte and at least one negatively charged polyelectrolyte, wherein the at least one positively charged polyelectrolyte is preferably selected form the group consisting of poly(allylamine hydrochloride) (PAH), polyl-lysine (PLL), poly(ethylene imine) (PEI), poly(histidine), poly(N,N-dimethyl aminoacrylate), poly(N,N,N-trimethylaminoacrylate chloride), poly(methyacrylamidopropyltrimethyl ammonium chloride), and natural or synthetic polysaccharides such as chitosan, and wherein the at least one negatively charged polyelectrolyte is preferably selected from the group consisting of poly(acrylic acid) (PAA), poly(styrenesulfonate) (PSS), alginate, hyaluronic acid, heparin, heparan sulfate, chondroitin sulfate, dextran sulfate, poly(meth)acrylic acid, oxidized cellulose, carboxymethyl cellulose, polyaspartic acid, and polyglutamic acid.

5. The process of Claim 1, wherein said wound active agent is incorporated into said nanoscale polymer multilayer so that said wound active agent is interspersed within the layers of said nanoscale polymer multilayer.

6. The process of Claim 1, wherein said wound active agent is selected from the group consisting of an antimicrobial agent, an antibiofilm agent, a growth factor, a hemostatic agent, a bioactive peptide, a bioactive polypeptide, an analgesic, an anticoagulant, anti-inflammatory agent, and a drug molecule or a drug compound.

7. The process of Claim 6, wherein said antimicrobial agent is a metal ion antimicrobial agent, which metal ion antimicrobial agent is preferably selected from the group consisting of a silver nanoparticle, or a small molecule antimicrobial agent, which small molecule antimicrobial agent is preferably selected from the group consisting of silver, chlorhexidine, antibiotics, polyhexamethylene biguanide (PHMB), iodine, cadexomer iodine, povidone iodine (PVI), hydrogen peroxide, and vinegar (acetic acid).

8. The process of Claim 7, wherein said antibiofilm agent is selected from the group consisting of small molecule antibiofilm agents, charged small molecule antibiofilm agents, antibiofilm polypeptides, antibiofilm enzymes, metallic particles, and metal ion antibiofilm agents, which metal ion antibiofilm agent is preferably a is a gallium ion, gallium ion salt, gallium ion nanoparticle, gallium alloy, or an alloy of gallium and silver.

9. The process of Claim 1, wherein said sacrificial polymer layer comprises a wound active agent selected from the group consisting of an antimicrobial agent, an antibiofilm agent, a growth factor, a hemostatic agent, a bioactive peptide, a bioactive polypeptide, an analgesic, an anticoagulant, anti-inflammatory agent, and a drug molecule or a drug compound.

10. The process of Claim 1, wherein said wound active agent is provided in an amount so that said wound active agent in said nanoscale polymer layer is released at a rate of 0.01 to 100 µg/cm² per day.

11. The process of Claim 1, wherein said sacrificial polymer layer is formed from a polymer selected from the group consisting of polyvinyl alcohol (PVA), polyacrylic acid (PAA).

12. The process of claim 1, wherein said sacrificial polymer layer is formed from polyvinyl alcohol (PVA).

13. The process of claim 3, wherein said polymer multilayer is formed by alternating layers of at least one positively charged polyelectrolyte and at least one negatively charged polyelectrolyte, wherein the at least one positively charged polyelectrolyte is poly(allylamine hydrochloride) (PAH), and wherein the at least one negatively charged polyelectrolyte is poly(acrylic acid) (PAA).

14. The process of claim 1, wherein the substrate is selected from the group consisting of a polydimethylsiloxane (PDMS) substrate, a glass substrate, a plastic substrate, a metal substrate, and a Teflon substrate.

## Patentansprüche

1. Verfahren zur Herstellung einer wundaktiven nanoskaligen Polymermatrix-Mikrolage, wobei das Verfahren die folgenden Schritte aufweist:
a) Bilden einer nanoskaligen Polymerschicht mit einer Dicke von etwa 0,5 nm bis 1000 nm auf einem Substrat;
b) Einbringen eines wundaktiven Mittels in die nanoskalige Polymerschicht, um die wundaktive nanoskalige Polymerschicht bereitzustellen; und
c) Bilden der Opferpolymerschicht mit einer Dicke von 0,1 µm bis 100 µm auf der wundaktiven nanoskaligen Polymerschicht, wobei die Opferpolymerschicht auflösbar ist.

2. Verfahren nach Anspruch 1, ferner aufweisend den Schritt
d) Entfernen der nanoskaligen Polymermatrix-Mikrolage, die die bioaktive nanoskalige Polymerschicht und die damit assoziierte Opferpolymerschicht aufweist, von dem Substrat.

3. Verfahren nach Anspruch 1, wobei die wundaktive nanoskalige Polymerschicht eine Polymer-Mehrfachschicht ist.

4. Verfahren nach Anspruch 3, wobei die Polymer-Mehrfachschicht durch alternierende Schichten aus mindestens einem positiv geladenen Polyelektrolyten und mindestens einem negativ geladenen Polyelektrolyten gebildet wird, wobei der mindestens eine positiv geladene Polyelektrolyt vorzugsweise ausgewählt ist aus der Gruppe bestehend aus Poly(allylamin-hydrochlorid) (PAH), Polyl-Lysin (PLL), Poly(ethylenimin) (PEI), Poly(histidin), Poly(N,N-dimethylamino-acrylat), Poly(N,N,N-trimethyl-aminoacrylatchlorid), Poly(methylacryl-amidopropyl-trimethyl-ammoniumchlorid) und natürliche oder synthetische Polysaccharide wie Chitosan, wobei der mindestens eine negativ geladene Polyelektrolyt vorzugsweise ausgewählt ist aus der Gruppe bestehend aus Poly(acrylsäure) (PAA), Poly(styrolsulfonat) (PSS), Alginat, Hyaluronsäure, Heparin, Heparansulfat, Chondroitinsulfat, Dextransulfat, Poly(meth)acrylsäure, oxidierte Cellulose, Carboxymethylcellulose, Polyasparaginsäure und Polyglutaminsäure.

5. Verfahren nach Anspruch 1, wobei das wundaktive Mittel in die nanoskalige Polymer-Mehrschicht eingearbeitet wird, so dass das wundaktive Mittel in die Schichten der nanoskaligen Polymer-Mehrschicht eingestreut ist.

6. Verfahren nach Anspruch 1, wobei das wundaktive Mittel ausgewählt ist aus der Gruppe bestehend aus einem antimikrobiellen Mittel, einem Anti-Biofilm-Mittel, einem Wachstumsfaktor, einem hämostatischen Mittel, einem bioaktiven Peptid, einem bioaktiven Polypeptid, einem Analgetikum, einem Antikoagulans, einem entzündungshemmenden Mittel und einem Arzneimittelmolekül oder einer Arzneimittelverbindung.

7. Verfahren nach Anspruch 6, wobei das antimikrobielle Mittel ein antimikrobielles Metallionenmittel ist, wobei das antimikrobielle Metallionenmittel vorzugsweise aus der Gruppe ausgewählt ist, die aus einem Silber-Nanopartikel oder einem Small-Moleculeantimikrobiellen Mittel besteht, wobei das Small-Molecule-antimikrobielle Mittel vorzugsweise aus der Gruppe ausgewählt ist, die aus Silber, Chlorhexidin, Antibiotika, Polyhexamethylenbiguanid (PHMB), Iod, Cadexomer-Iod, Povidon-Iod (PVI), Wasserstoffperoxid und Essig (Essigsäure) besteht.

8. Verfahren nach Anspruch 7, wobei das Anti-Biofilm-Mittel ausgewählt ist aus der Gruppe bestehend aus Small-Molecule-Anti-Biofilm-Mitteln, geladenen Small-Molecule-Anti-Biofilm-Mitteln, Anti-Biofilm-Polypeptiden, Anti-Biofilm-Enzymen, Metall-Partikeln und Metallionen-Anti-Biofilm-Mitteln, wobei das Metallionen-Anti-Biofilm-Mittel vorzugsweise ein Gallium-Ion, ein Gallium-Ionen-Salz, ein Gallium-Ionen-Nanopartikel, eine GalliumLegierung oder eine Legierung aus Gallium und Silber ist.

9. Verfahren nach Anspruch 1, wobei die Opferpolymerschicht ein wundaktives Mittel aufweist, das aus der Gruppe ausgewählt ist, die aus einem antimikrobiellen Mittel, einem Antibiofilmmittel, einem Wachstumsfaktor, einem hämostatischen Mittel, einem bioaktiven Peptid, einem bioaktiven Polypeptid, einem Analgetikum, einem Antikoagulans, einem entzündungshemmenden Mittel und einem Arzneimittelmolekül oder einer Arzneimittelverbindung besteht.

10. Verfahren nach Anspruch 1, wobei das wundaktive Mittel in einer solchen Menge bereitgestellt wird, dass das wundaktive Mittel in der nanoskaligen Polymerschicht mit einer Rate von 0,01 bis 100 µg/cm² pro Tag freigesetzt wird.

11. Verfahren nach Anspruch 1, wobei die Opferpolymerschicht aus einem Polymer gebildet wird, das aus der Gruppe ausgewählt ist, die aus Polyvinylalkohol (PVA), Polyacrylsäure (PAA) besteht.

12. Verfahren nach Anspruch 1, wobei die Opferpolymerschicht aus Polyvinylalkohol (PVA) gebildet wird.

13. Verfahren nach Anspruch 3, wobei die Polymer-Mehrfachschicht durch alternierende Schichten aus mindestens einem positiv geladenen Polyelektrolyten und mindestens einem negativ geladenen Polyelektrolyten gebildet wird, wobei der mindestens eine positiv geladene Polyelektrolyt Poly(allylaminhydrochlorid) (PAH) ist und wobei der mindestens eine negativ geladene Polyelektrolyt Poly(acrylsäure) (PAA) ist.

14. Verfahren nach Anspruch 1, wobei das Substrat ausgewählt ist aus der Gruppe bestehend aus einem Polydimethylsiloxan (PDMS)-Substrat, einem Glassubstrat, einem Kunststoffsubstrat, einem Metallsubstrat und einem Teflonsubstrat.

## Revendications

1. Procédé pour la fabrication d'une microfeuille de matrice de polymère nanométrique active sur les plaies, le procédé comprenant les étapes suivantes :
a) formation d'une couche de polymère nanométrique d'une épaisseur d'environ 0,5 nm à 1 000 nm sur un substrat ;
b) introduction d'un agent actif sur les plaies dans ladite couche de polymère nanométrique pour fournir la couche de polymère nanométrique active sur les plaies ; et
c) formation de la couche de polymère sacrificielle d'une épaisseur de 0,1 µm à 100 µm sur ladite couche de polymère nanométrique active sur les plaies, la couche de polymère sacrificielle pouvant être dissoute.

2. Procédé selon la revendication 1, comprenant en outre l'étape
d) élimination de la microfeuille de matrice de couche de polymère nanométrique comprenant la couche de polymère nanométrique bioactive et la couche de polymère sacrificielle associée du substrat.

3. Procédé selon la revendication 1, ladite couche de polymère nanométrique active sur les plaies étant une multicouche de polymère.

4. Procédé selon la revendication 3, ladite multicouche de polymère étant formée de couches alternées d'au moins un polyélectrolyte chargé positivement et d'au moins un polyélectrolyte chargé négativement, l'au moins un polyélectrolyte chargé positivement étant préférablement choisi dans le groupe constitué par un poly(chlorhydrate d'allylamine) (PAH), une polyl-lysine (PLL), une poly(éthylène imine) (PEI), une poly(histidine), un poly(N,N-diméthylamino-acrylate), un poly(chlorure de N,N,N-triméthylaminoacrylate), un poly(chlorure de méthylacrylamidopropyl-triméthylammonium), et des polysaccharides naturels ou synthétiques comme un chitosane, et l'au moins un polyélectrolyte chargé négativement étant préférablement choisi dans le groupe constitué par un poly(acide acrylique) (PAA), un poly(sulfonate de styrène) (PSS), un alginate, un acide hyaluronique, une héparine, un sulfate d'héparane, un sulfate de chondroïtine, un sulfate de dextrane, un poly(acide (méth)acrylique), une cellulose oxydée, une carboxyméthylcellulose, un poly(acide aspartique), et un poly(acide glutamique).

5. Procédé selon la revendication 1, ledit agent actif sur les plaies étant incorporé dans ladite multicouche de polymère nanométrique de sorte que ledit agent actif sur les plaies est parsemé dans les couches de ladite multicouche de polymère nanométrique.

6. Procédé selon la revendication 1, ledit agent actif sur les plaies étant choisi dans le groupe constitué par un agent antimicrobien, un agent anti-biofilm, un facteur de croissance, un agent hémostatique, un peptide bioactif, un polypeptide bioactif, un analgésique, un anticoagulant, un agent anti-inflammatoire, et une molécule de médicament ou un composé de médicament.

7. Procédé selon la revendication 6, ledit agent antimicrobien étant un agent antimicrobien de type ion métallique, lequel agent antimicrobien de type ion métallique est préférablement choisi dans le groupe constitué par une nanoparticule d'argent, ou un agent antimicrobien de type petite molécule, lequel agent antimicrobien de type petite molécule est préférablement choisi dans le groupe constitué par l'argent, la chlorhexidine, des antibiotiques, un poly(biguanide d'hexamethylène) (PHMB), l'iode, le cadexomère d'iode, une povidone iodée (PVI), le peroxyde d'hydrogène, et le vinaigre (acide acétique).

8. Procédé selon la revendication 7, ledit agent anti-biofilm étant choisi dans le groupe constitué par des agents anti-biofilm de type petite molécule, des agents antibiofilm de type petite molécule chargée, des polypeptides anti-biofilm, des enzymes antibiofilm, des particules métalliques, et des agents anti-biofilm de type ion métallique, lequel agent anti-biofilm de type ion métallique est préférablement un ion gallium, un sel d'ion gallium, une nanoparticule d'ion gallium, un alliage de gallium, ou un alliage de gallium et d'argent.

9. Procédé selon la revendication 1, ladite couche de polymère sacrificielle comprenant un agent actif sur les plaies choisi dans le groupe constitué par un agent antimicrobien, un agent anti-biofilm, un facteur de croissance, un agent hémostatique, un peptide bioactif, un polypeptide bioactif, un analgésique, un anticoagulant, un agent anti-inflammatoire, et une molécule de médicament ou un composé de médicament.

10. Procédé selon la revendication 1, ledit agent actif sur les plaies étant fourni en une quantité telle que ledit agent actif sur les plaies dans ladite couche de polymère nanométrique soit libéré à une vitesse de 0,01 à 100 µg/cm² par jour.

11. Procédé selon la revendication 1, ladite couche de polymère sacrificielle étant formée à partir d'un polymère choisi dans le groupe constitué par un poly(alcool vinylique) (PVA), un poly(acide acrylique) (PAA).

12. Procédé selon la revendication 1, ladite couche de polymère sacrificielle étant formée à partir d'un poly(alcool vinylique) (PVA).

13. Procédé selon la revendication 3, ladite multicouche de polymère étant formée en alternant des couches d'au moins un polyélectrolyte chargé positivement et d'au moins un polyélectrolyte chargé négativement, l'au moins un polyélectrolyte chargé positivement étant un poly(chlorhydrate d'allylamine) (PAH), et l'au moins un polyélectrolyte chargé négativement étant un poly(acide acrylique) (PAA).

14. Procédé selon la revendication 1, le substrat étant choisi dans le groupe constitué par un substrat de polydiméthylsiloxane (PDMS), un substrat de verre, un substrat de plastique, un substrat de métal, et un substrat de Téflon.
